# EUROPEAN PATENT SPECIFICATION

(11) **EP 3 447 134 B1**
(45) Date of publication and mention of the grant of the patent: **11.10.2023**
(21) Application number: 17187309.4
(22) Date of filing: 22.08.2017
(51) Int. Cl.: C12N 9/12, C12N 15/82, A01H 5/10

(54) **INCREASED FUNGAL RESISTANCE IN CROP PLANTS**
ERHÖHTE PILZRESISTENZ BEI NUTZPFLANZEN
UNE RÉSISTANCE FONGIQUE ACCRUE DANS DES PLANTES CULTIVÉES

(43) Date of publication of application: 27.02.2019
(73) Proprietor: KWS SAAT SE & Co. KGaA, 37574 Einbeck (DE); University of Zurich, 8006 Zurich (CH); University of Bern, 3012 Bern (CH)
(72) Inventor: Keller, Beat, 8006 Zürich (CH); Krattinger, Simon, 8906 Bonstetten (CH); Praz, Coraline, 8008 Zurich (CH); Yang, Ping, Hai Dian District, Beijing 100081 (CN); Erb, Matthias, 3013 Bern (CH); Ouzunova, Milena, 37075 Göttingen (DE); Kessel, Bettina, 37574 Einbeck (DE); Scheuermann, Daniela, 37574 Einbeck (DE)

(56) References cited:
- WO-A1-98/40505
- WO-A1-2017/066597
- WO-A2-2015/032494
- NIEMEYER HERMANN M: "Hydroxamic Acids Derived from 2-Hydroxy-2H-1,4-Benzoxazin-3(4H)-one: Key Defense Chemicals of Cereals", JOURNAL OF AGRICULTURAL AND FOOD CHEMISTRY, vol. 57, no. 5, 11 March 2009 (2009-03-11) , pages 1677-1696, XP002774582,
- AHMAD SHAKOOR ET AL: "Benzoxazinoid Metabolites Regulate Innate Immunity against Aphids and Fungi in Maize", PLANT PHYSIOLOGY (ROCKVILLE), vol. 157, no. 1, September 2011 (2011-09), pages 317-327, XP002774583, ISSN: 0032-0889
- HURNI SEVERINE ET AL: "The maize disease resistance gene Htn1 against northern corn leaf blight encodes a wall-associated receptor-like kinase.", PROCEEDINGS OF THE NATIONAL ACADEMY OF SCIENCES OF THE UNITED STATES OF AMERICA 14 JUL 2015, vol. 112, no. 28, 14 July 2015 (2015-07-14), pages 8780-8785, XP002774584, ISSN: 1091-6490
- NIEMEYER HERMANN M: "Hydroxamic Acids Derived from 2-Hydroxy-2H-1,4-Benzoxazin-3(4H)-one: Key Defense Chemicals of Cereals", JOURNAL OF AGRICULTURAL AND FOOD CHEMISTRY, vol. 57, no. 5, 11 March 2009 (2009-03-11) , pages 1677-1696, ISSN: 0021-8561(print)
- AHMAD SHAKOOR ET AL: "Benzoxazinoid Metabolites Regulate Innate Immunity against Aphids and Fungi in Maize", PLANT PHYSIOLOGY (ROCKVILLE), vol. 157, no. 1, September 2011 (2011-09), pages 317-327, ISSN: 0032-0889
- HURNI SEVERINE ET AL: "The maize disease resistance gene Htn1 against northern corn leaf blight encodes a wall-associated receptor-like kinase.", PROCEEDINGS OF THE NATIONAL ACADEMY OF SCIENCES OF THE UNITED STATES OF AMERICA 14 JUL 2015, vol. 112, no. 28, 14 July 2015 (2015-07-14), pages 8780-8785, ISSN: 1091-6490

## Description

### Technical Field

The present invention relates to methods for producing plants with increased fungal resistance, preferably seedling resistance against Northern Corn Leaf Blight. Further provided are methods for introducing, modifying, or modulating at least one wall-associated kinase (WAK) in(to) a plant cell, tissue, organ, or whole plant and thereby causing a reduced synthesis of benzoxazinoid and in turn increased fungal resistance. There are further provided methods to identify and/or modify downstream effector molecules in a WAK signalling cascade. Finally, plant cells, tissues, organs or whole plants having increased fungal resistance and methods using substances to activate signalling pathways in a targeted way are provided. The present invention thus relates to WAKs as master regulators and crucial signaling mediators in plant defense against fungal disease and the regulation and cross-talk mechanisms in the WAK signaling cascade and further gives examples for establishing novel anti-fungal strategies relevant for a series of crop plants.

### Background of the Invention

Infections and infestations of crop plants by pathogens encompassing viruses, bacteria, fungi, nematodes and insects and the resulting damages cause significant yield losses of cultivated plants. In maize (*Zea mays*) as one of the major crop plants worldwide there are a large number of fungal pathogens which cause leaf diseases. The fungus which can cause by far the most damage under tropical and also under temperate climatic conditions, such as those in large parts of Europe and North America as well as in Africa and India, is known as *Helminthosporium turcicum* or synonymously as *Exserohilum turcicum* (teleomorph: *Setosphaeria turcica*). *H. turcicum* / *E. turcicum* is the cause of the leaf spot disease known as "Northern Corn Leaf Blight" (NCLB), which can occur in epidemic proportions during wet years, attacking vulnerable maize varieties and causing a great deal of damage and considerable losses of yield of 30% and more over wide areas (Perkins, J. M., and W. L. Pedersen. "Disease development and yield losses associated with northern leaf blight on corn." Plant disease 71.10 (1987): 940-943; Raymundo, A. D., A. L. Hooker, and J. M. Perkins. "Effect of gene HtN on the development of northern corn leaf blight epidemics." Plant disease (1981); Ullstrup, AJ, and Miles, SR 1957. The effects of some leaf blights of corn on grain yield. Phytopathology 47:331-336). Since the 1970s, then, natural resistance in genetic material has been sought.

The race for defining and establishing new resistance strategies against pathogens for major crop plants is more and more accelerated due to the increasing resistance breaking characteristics of pathogens, i.e., the evolutionary strategy of pathogens to adapt to and survive pressure of plant protective agents and/or to subvert the endogenous plant defense mechanisms.

Currently, quantitative and qualitative resistances are known. While the oligo- or polygenically inherited quantitative resistance appears incomplete and non-specific as regards race in the phenotype and is influenced by additional and partially dominant genes, qualitative resistance is typically race-specific and can be inherited through individual: For instances, mostly dominant resistance genes with regard to NCLB are Ht1, Ht2, Ht3, Htm, Htn1, or Htp (Lipps, P. E., R. C. Pratt, and J. J. Hakiza. "Interaction of Ht and partial resistance to Exserohilum turcicum in maize." Plant disease 81.3 (1997): 277-282; Wang, H., et al. "Expression of Ht2-related genes in response to the HT-Toxin of Exserohilum turcicum in Maize." Annals of applied biology 156.1 (2010): 111-120). Backcrosses in many frequently used inbred maize lines such as W22, A619, B37 or B73 have successfully brought about introgression of the HT genes, where they exhibit a partial dominance and expression as a function of the respective genetic background (Welz, HG (1998). "Genetics and epidemiology of the pathosystem Zea mays / Setosphaeria turcica." Habilitationsschrift Institut für Pflanzenzüchtung, Saatgutforschung und Populationsgenetik, Universität Hohenheim). WO 2011/163590 A1 annotated the presumed Htn1 gene in the resistance source PH26N and PH99N as a tandem protein kinase-like gene and disclosed its genetic sequence, but did not determine its functionality, for example in a transgenic maize plant. WO 2015/032494 A2 discloses the identification of another allelic variant of HTN1 gene derived from the donor Peptilla as well as a resistant maize plant into the genome of which a chromosome fragment from the donor Pepitilla has been integrated, which chromosome fragment comprises the resistance locus HTN1.

The hemibiotrophic fungal pathogen *Exserohilum turcicum* (anamorph form of the fungus) causing NCLB is found in humid climates wherever corn is grown. E. *turcicum* survives in corn debris and builds up over time in high-residue and continuous corn cropping systems. High humidity and moderate temperatures favor the persistence of the E. *turcicum* fungus causing tremendous yield losses, e.g., due to decreased photosynthesis resulting in limited ear fill, or harvest losses if secondary stalk rot infection and stalk lodging accompany loss of leaf area.

As a natural defense mechanism against various kinds of pathogens, Plants have evolved multiple layers of defense against infection by pathogenic microbes (Jones, Jonathan DG, and Jeffery L. Dangl. "The plant immune system." Nature 444.7117 (2006): 323). The primary defense is based on the extracellular perception of pathogen-associated or host damage-associated molecular patterns or signatures (PAMPs/DAMPs) by plasma membrane-anchored pattern recognition receptors (PRRs).

These receptor proteins monitor the extracellular space for the presence of microbial- or host-derived elicitors, i.e., PAMPs or DAMPs, respectively. These signatures can be highly conserved and characteristic for entire pathogen classes as in the case of the bacterial flagellin that is perceived by the leucine-rich repeat receptor kinase (LRR-RK) FLS2, which results in basal and broad-spectrum resistance against most bacteria (Macho, Alberto P., and Cyril Zipfel. "Plant PRRs and the activation of innate immune signaling." Molecular cell 54.2 (2014): 263-272). Other receptor kinases only confer resistance to certain races of a particular pathogen (Hu, Keming, et al. "Improvement of multiple agronomic traits by a disease resistance gene via cell wall reinforcement." Nature plants 3 (2017): 17009). Receptor kinases have different types of extracellular domains, including leucine-rich repeats (LRRs), lysine motifs (LysMs), lectin motifs or epidermal growth factor (EGF) like extracellular domains (Dardick, Chris, Benjamin Schwessinger, and Pamela Ronald. "Non-arginine-aspartate (non-RD) kinases are associated with innate immune receptors that recognize conserved microbial signatures." Current opinion in plant biology 15.4 (2012): 358-366).

In grasses, there is emerging evidence that WAKs might be important players in fungal and bacterial disease resistance. The WAK genes *qHSR1, Htn1* and *OsWAK* (*Xa4*) confer disease resistance (Humi, Severine, et al. "The maize disease resistance gene Htn1 against northern corn leaf blight encodes a wall-associated receptor-like kinase." Proceedings of the National Academy of Sciences 112.28 (2015): 8780-8785., Hu et al. 2017), yet little is known about the underlying mechanisms and signaling cascades responsible for the observed phenotypes.

*OsWAK* underlying resistance involves strengthening of the cell intensity by enhancing cellulose biosynthesis (Hu et al. 2017). Interestingly, there is also one case described where the wheat WAK encoded by the *Snn1* gene acts as a susceptibility factor. It has been shown that Snn1 perceives the SnTox1 toxin encoded by the fungal pathogen *Stagonospora nodorum*, which triggers cell death and allows the necrotrophic S. *nodorum pathogen* to proliferate on wheat (Shi, Gongjun, et al. "Marker development, saturation mapping, and high-resolution mapping of the Septoria nodorum blotch susceptibility gene Snn3-B1 in wheat." Molecular genetics and genomics 291.1 (2016): 107-119). In dicots, the Arabidopsis AtWAK1 was found to physically associate with and recognized cell wall-derived oligogalactouronides (OGs), which result from polysaccharide degradation (Kohom, Bruce D., et al. "Pectin activation of MAP kinase and gene expression is WAK2 dependent." The Plant Journal 60.6 (2009): 974-982). In contrast to the Arabidopsis *WAK* gene family that consists of only five members, *WAK* genes in monocots belong to a large family. For instance, in rice >100 members were found (Kanneganti, Vydehi, and Aditya K. Gupta. "Wall associated kinases from plants-an overview." Physiology and Molecular Biology of Plants 14.1-2 (2008): 109-118). The emergence of WAKs in monocots might be associated to several functional aspects, e.g. biotic diseases (Li, Hui, et al. "A novel wall-associated receptor-like protein kinase gene, OsWAK1, plays important roles in rice blast disease resistance." Plant molecular biology 69.3 (2009): 337-346; Humi et al. 2015; Zuo, Weiliang, et al. "A maize wall-associated kinase confers quantitative resistance to head smut." Nature genetics 47.2 (2015): 151-157; Shi, Gongjun, et al. "The hijacking of a receptor kinase-driven pathway by a wheat fungal pathogen leads to disease." Science advances 2.10 (2016): e1600822; Hu et al. 2017), tolerance of phosphorus deficiency (Hufnagel, Barbara, et al. "Duplicate and conquer: Multiple homologs of PHOSPHORUS-STARVATION TOLERANCE! enhance phosphorus acquisition and sorghum performance on low-phosphorus soils." Plant physiology 166.2 (2014): 659-677), root growth (Kaur, Ravneet, Kashmir Singh, and Jaswinder Singh. "A root-specific wall-associated kinase gene, HvW AK 1, regulates root growth and is highly divergent in barley and other cereals." Functional & integrative genomics 13.2 (2013): 167-177) as well as gametophyte development (Wang, Na, et al. "The rice wall-associated receptor-like kinase gene OsDEES1 plays a role in female gametophyte development." Plant physiology 160.2 (2012): 696-707; Hu et al. 2017). Therefore, there is emerging evidence that wall-associated kinases (WAKs) could play a pivotal role in plant immunity specifically in cereal crops.

Still, presently little is known about the specific molecular mechanisms underlying plant immunity based on WAKs, said immune mechanisms being highly specific for each pathogen and thus PAMP/DAMP to be recognized and furthermore depending on the downstream signaling cascade and the subsequently initiated effector mechanisms. So far, rather the phenotypic outcome of a plant comprising or not comprising a receptor-like kinase on pathogen resistance has been matched with the genotype of a plant. The molecular mechanisms which are responsible for the action of the WAKs, the precise signaling pathways and also the crosstalk between different pathways remain elusive.

Chemical fungicides have long been utilised for controlling fungal diseases. A different approach relies on the examination and elucidation of the complex biosynthetic pathways involved in pathogen resistance causing a natural pathogen defence of plants by studying the resistance ability of an existing plant cultivar to inhibit or at least limit any infestation of a pathogen to provide new strategies to combat plant pathogens and to provide new plants carrying resistance traits of interest.

Benzoxazinoids (BXDs) were identified in the 1960s as secondary plant metabolites functioning as natural pesticides. BXDs are a class of secondary metabolites found in maize and other cereal species and certain dicots and contain a 2-hydroxy-2H-1,4-benzoxazin-3(4H)-one skeleton (Niemeyer, Hermann M. "Hydroxamic acids derived from 2-hydroxy-2 H-1, 4-benzoxazin-3 (4 H)-one: key defense chemicals of cereals." Journal of Agricultural and Food Chemistry 57.5 (2009): 1677-1696). BXDs are synthesized in seedlings and stored as glucosides. The main aglucone moieties are 2,4-dihydroxy-2*H*-1,4-benzoxazin-3(*4*H)-one (DIBOA) and 2,4-dihydroxy-7-methoxy-2*H*-1,4-benzoxazin-3(4H)-one (DIMBOA). BXDs are synthesised in two subfamilies of the Poaceae and sporadically found in single species of the dicots. BXDs are predominantly stored as inactive glucosides, while upon biotic stress they are hydrolyzed to the respective toxic hydroxamic acids (e.g., DIMBOA). The first step in BXD biosynthesis converts indole-3-glycerol phosphate into indole. In maize (*Zea mays*), this reaction is catalyzed by either Benzoxazineless1 (BX1), or Indole glycerol phosphate lyase (IGL or Igl herein). The *bx1* gene is under developmental control and is mainly responsible for BX production, whereas the Igl gene is inducible by stress signals, such as wounding, herbivory, or jasmonates. The enzymatic properties of IGL are similar to BX1, but the transcriptional regulation of their corresponding genes is different. Like other Bx genes, *bx1* is constitutively expressed during the early developmental stages of the plant, which correlates with endogenous BX levels. The introduction of four oxygen atoms into the indole moiety that yields DIBOA is catalyzed by four cytochrome P450 monooxygenases termed BX2 to BX5. A further Bx enzyme, BX6, is responsible for the hydroxylation in position C-7 of the benzoxazinoids in maize (Frey, Monika, et al. "A 2-oxoglutarate-dependent dioxygenase is integrated in DIMBOA-biosynthesis." Phytochemistry 62.3 (2003): 371-376). Bx7, as further example, is an O-methyltransferase (OMT) catalyzing the formation of DIMBOA-glc from TRIBOA-glc.

Little is known about the role of these compounds in fungal disease resistance. Few field studies decades ago proposed a correlation between benzoxazinoid hydroxamic acids concentration and the disease resistance to maize stalk rot, maize northern corn leaf blight and wheat stem rust (Long, B. J., G. M. Dunn, and D. G. Routley. "Relationship of hydroxamate concentration in maize and field reaction to Helminthosporium turcicum." Crop Science 18.4 (1978): 573-575) without providing any molecular basis for this phenomenon. Other studies even found no effect of BXDs in fungal disease resistance, including maize stalk rot, Southern corn leaf blight, maize anthracnose, corn smut and head blight at all (Niemeyer, 2009).

Therefore, it was an outstanding aim of the present invention to provide new tools and methods to allow for a tight resistance management for major crop plants concerning the control of *Helminthosporium turcicum* plant pathogen.

Further, it was an object of the present invention to provide new strategies and methods to combat fungal leaf diseases based on exploiting plant-endogenous defense mechanisms as mediated by secondary metabolites and further to provide plants having a specific genotype as source of increased resistance or tolerance against fungal pathogens causing severe plant diseases. Basically, it was an object of the present invention to elucidate the molecular basis of the maize *Htnl* northern corn leaf blight (NCLB) resistance that is cause by the WAK gene *ZmWAK-RLK1* to establish molecular targets and cross-talk mechanisms between relevant biosynthesis pathways to provide new urgently needed resistance strategies for a variety of important crop plants by characterizing the molecular players involved in disease resistance in a plant.

### Summary of the Invention

The above object was achieved by identifying the molecular basis of the maize *Htnl* northern corn leaf blight (NCLB) resistance that is caused by the WAK gene *ZmWAK-RLK1.* It was demonstrated that *ZmWAK-RLK1* modulates, i.e. it functions upstream of the benzoxazinoids (BXD) biosynthesis pathway, resulting in reduced BXD concentrations. Furthermore, the interaction of WAK with downstream effectors, i.e., BX enzymes and Igl, in the regulation of BXD biosynthesis and fungal disease were demonstrated. Furthermore, the present invention builds on relevant information of the cross-talk and cross-regulation of the plant WAK signaling pathway with the jasmonic acid (JA) and further relevant plant biosynthesis pathways to elucidate the regulatory networks important to modulate and to induce plant defense against major pathogens.

Interestingly, maize plants with compromised BXD biosynthesis showed increased resistance against NCLB. Thus, these new insight into WAK-mediated quantitative disease resistance and for the first time provided a functional link between WAKs and the secondary metabolites BXDs which was used to define new fungal defense strategies going beyond the use of fungicides for disease control which are applicable for several associated fungal pathogens inducing comparable plant immune reactions by pathogen-associated PAMPs/DAMPs in several important crop plants.

The present invention thus transforms the information on molecular mechanisms, the effect of specific mutations in kinase domains on the signaling function of a WAK, and further on the cross-talk and interplay of signaling cascades to provide plants having a defined genetic background and thus to provide plants with increased resistance against fungal pathogens. Furthermore, the present invention provides methods to modulate (increase/decrease) or neutralize the action of plant secondary metabolites and the relevant genes responsible for the synthesis pathways of said secondary metabolites to enhance fungal pathogen resistance in a targeted way.

In one aspect, there is thus provided a method for producing a *Zea mays* plant having increased *Helminthosporium turcicum* resistance, wherein the *Helminthosporium turcicum* resistance is regulated by at least one wall-associated kinase, the method comprising: (i) (a) providing at least one plant cell, tissue, organ, or whole plant having a specific genotype with at least one gene encoding a wall-associated kinase in the genome of said plant cell, tissue, organ, or whole plant; or (i) (b) introducing at least one gene encoding at least one wall-associated kinase into the genome of at least one cell of at least one of a plant cell, tissue, organ, or whole plant, wherein the at least one gene encoding at least one wall-associated kinase is stably introduced into said genome, and wherein a stable introduction comprises Agrobacterium-mediated transformation, genome editing, or a combination thereof;; and (ii) (a) mutating the at least one gene encoding at least one wall-associated kinase in the at least one plant cell, tissue, organ, or whole plant via deletion, insertion or substitution of one or more nucleotides by at least one of a site-specific nuclease (SSN), oligonucleotide direct mutagenesis, chemical mutagenesis, or TILLING; and/or (ii) (b) increasing the expression level of at least one wall-associated kinase and/or reducing the expression level of at least one molecule within the signalling pathway from the at least one wall-associated kinase to the synthesis of at least one benzoxazinoid or within the synthesis pathway of at least one benzoxazinoid in the at least one plant cell, tissue, organ, or whole plant by at least one of a site-specific nuclease (SSN), oligonucleotide direct mutagenesis, chemical mutagenesis, or TILLING; and (iii) selecting/identifying a plant generated from the at least one plant cell, tissue, organ, or whole plant of (ii) (a) or (ii) (b), which has increased *Helminthosporium turcicum* resistance, based on the determination of a reduced synthesis of at least one benzoxazinoid DIM₂BOA being in the glucoside or aglucone form, or a benzoxazolinone in response to a *Helminthosporium turcicum* pathogen infection, as compared to a corresponding control plant of the same genotype lacking the mutation of (ii) (a) or the modulation of (ii) (b), wherein the synthesis of the at least one benzoxazinoid is regulated by the at least one wall-associated kinase; wherein the at least one wall-associated kinase is a WAK-RLK1 and a) is encoded by a nucleic acid molecule comprising the nucleotide sequence of SEQ ID NO: 1 or 7, b) is encoded by a nucleic acid molecule comprising the nucleotide sequence having at least 80% sequence identity to nucleotide sequence of SEQ ID NO: 1 or 7, preferably over the entire length of the sequence, c) is encoded by a nucleic acid molecule hybridizing with a complementary sequence to a) or b) under stringent conditions, wherein stringent hybridization conditions are hybridization in 4×SSC at 65°C and subsequent multiple washes in 0.1×SSC at 65°C for approximately 1 hour, d) is encoded by a nucleic acid molecule comprising the nucleotide sequence coding for an amino acid sequence of SEQ ID NO: 2 or 8, e) is encoded by a nucleic acid molecule comprising the nucleotide sequence coding for an amino acid sequence having at least 80% sequence identity to amino acid sequence of SEQ ID NO: 2 or 8, preferably over the entire length of the sequence, f) comprises the amino acid sequence of SEQ ID NO: 2 or 8, or g) comprises an amino acid sequence having at least 80% sequence identity to amino acid sequence of SEQ ID NO: 2 or 8, preferably over the entire length of the sequence, provided that any sequence of a) to g), optionally after expression, still encodes at least one functional Htn1 according to SEQ ID NO: 2, Ht2 or Ht3 according to SEQ ID NO: 8, or an allelic variant of the wall-associated kinase which is located at or mapped on a locus in bin 5 or bin 6 on the long arm of chromosome 8; wherein the at least one molecule within the signaling pathway from the at least one wall-associated kinase to the synthesis of at least one benzoxazinoid or within the synthesis pathway of at least one benzoxazinoid is selected from the group constiting of: I) the genes *bx1* (SEQ ID NO: 10), *bx2* (SEQ ID NO: 12), *igl* (SEQ ID NO: 14), *bx6* (SEQ ID NO: 16), *bx11* (SEQ ID NO: 18), *bx14* (SEQ ID NO: 20), *opr2* (SEQ ID NO: 22), *lox3* (SEQ ID NO: 24) *or aocl* (SEQ ID NO: 26), or II) a gene having identity of at least 80% to one of the genes of I), preferably over the full length of the gene, in particular the coding regions of the gene, or III) the proteins BX1 (SEQ ID NO: 11), BX2 (SEQ ID NO: 13), IGL (SEQ ID NO: 15), BX6 (SEQ ID NO: 17), BX11 (SEQ ID NO: 19), BX14 (SEQ ID NO: 21), OPR2 (SEQ ID NO: 23), LOX3 (SEQ ID NO: 25) or AOC1 gene (SEQ ID NO: 27), or IV) a protein having identity of at least 80% to one of the proteins of III), preferably over the full length of the protein, and wherein the benzoxazinoid whose synthesis is regulated by the at least one wall-associated kinase is DIM₂BOA, the aforementioned benzoxazinoid being in the glucoside or aglucone form, or a benzoxazolinone, or any combination with DIMBOA, HMBOA, HM₂BOA, HDMBOA, HDM₂BOA, HBOA, DHBOA, DIBOA or TRIBOA.

In one embodiment of the various aspects of the present invention, the at least one wall-associated kinase is a WAK-RLK1 gene, preferably selected from Htn1 according to SEQ ID NO: 2, Ht2 or Ht3 according to SEQ ID NO: 8, or an allelic variant thereof wherein the at least one wall-associated kinase a) is encoded by a nucleic acid molecule comprising the nucleotide sequence of SEQ ID NO: 1 or 7, b) is encoded by a nucleic acid molecule comprising the nucleotide sequence having at least 81%, 82%, 83%, 84%, 85%, 86%, 87%, 88%, 89%, 90%, 91%, 92%, 93%, 94%, 95%, 96%, 97%, 98% or 99% sequence identity to nucleotide sequence of SEQ ID NO: 1 or 7, preferably over the entire length of the sequence, c) is encoded a nucleic acid molecule hybridizing with a complementary sequence to a) or b) under stringent conditions, wherein stringent hybridization conditions are hybridization in 4xSSC at 65°C and subsequent multiple washes in 0.1xSSC at 65°C for approximately 1 hour, d) is encoded by a nucleic acid molecule comprising the nucleotide sequence coding for an amino acid sequence of SEQ ID NO: 2 or 8, e) is encoded by a nucleic acid molecule comprising the nucleotide sequence coding for an amino acid sequence having at least 81%, 82%, 83%, 84%, 85%, 86%, 87%, 88%, 89%, 90%, 91%, 92%, 93%, 94%, 95%, 96%, 97%, 98% or 99% sequence identity to amino acid sequence of SEQ ID NO: 2 or 8, preferably over the entire length of the sequence, f) comprising the amino acid sequence of SEQ ID NO: 2 or 8, or g) comprising an amino acid sequence having at least 81%, 82%, 83%, 84%, 85%, 86%, 87%, 88%, 89%, 90%, 91%, 92%, 93%, 94%, 95%, 96%, 97%, 98% or 99% sequence identity to amino acid sequence of SEQ ID NO: 2 or 8, preferably over the entire length of the sequence,provided that the any sequence of a) to g), optionally after expression, still encodes at least one functional Htn1, Ht2, or Ht3, or an allelic variant, a mutant, or a functional fragment thereof. Preferably, the at least one wall-associated kinase of the invention causes after expression a reduced synthesis of at least one benzoxazinoid.

In a further embodiment of the various aspects of the present invention, the benzoxazinoid whose biosynthesis is regulated by the at least one wall-associated kinase, is further selected from at least one of DIMBOA, HMBOA, HM₂BOA, HDMBOA, HDM₂BOA, HBOA, DHBOA, DIBOA or TRIBOA, the aforementioned benzoxazinoid being in the glucoside or aglucone form, or a benzoxazolinone, or any combination of the aforementioned benzoxazinoids, preferably wherein the benzoxazinoid whose biosynthesis is regulated by the at least one wall-associated kinase is further selected from at least one of DIMBOA, HMBOA or HDMBOA, the aforementioned benzoxazinoid being in the glucoside or aglucone form, or any combination of the aforementioned benzoxazinoids.

In another embodiment, there is provided a method, wherein the reduced synthesis of at least one benzoxazinoid is achieved by providing at least one wall-associated kinase, an allelic variant, a mutant or a functional fragment thereof, or a gene encoding the same, wherein the at least one wall-associated kinase comprises a sequence which can directly or indirectly influence the benzoxazinoid (synthesis) pathway and at least one further plant metabolic pathway, preferably a disease resistance associated pathway, wherein the plant metabolic pathway is selected from the group consisting of the jasmonic acid pathway, the ethylene pathway, the lignin synthesis pathway, a defense pathway, a receptor-like kinase pathway, and/or a cell wall associated pathway.

In yet a further embodiment of the various aspects of the present invention, a further fungus resistance against which resistance is increased, or the disease caused by said fungus is additionally selected from a fungus of the order of *Pleosporales* causing southern corn leaf blight *(Bipolaris maydis)*, the order of *Pucciniales* causing rust disease, comprising common rust (*Puccinia sorghi*), or Diploida leaf streak/blight *(Diploida macrospora* / *Stenocarpella macrospora*), or *Colletotrichum graminicola*, or *Fusarium spp.*, preferably *Fusarium verticilioides* causing Fusarium stalk rot, or *Gibberella spp.*, e.g., *Gibberella zeae* causing Giberella stalk rot, rust, stalk rot, maize head smut (*Sphacelotheca reiliana*), and Diploida leaf streak/blight.

In one of the embodiments of the various aspects of the present invention, the at least one gene encoding at least one wall-associated kinase is stably integrated/introduced into the genome of the at least one plant cell, tissue, organ, or whole plant, wherein the at least one gene encoding at least one wall-associated kinase is stably introduced into said genome, and wherein a stable introduction comprises Agrobacterium-mediated transformation, genome editing, or a combination thereof.

In a further embodiment of the of the various aspects of the present invention, the at least one site-specific nuclease (SSN), or the nucleic acid sequence encoding the same, is selected from at least one of a CRISPR nuclease, including Cas or Cpfl nucleases, a TALEN, a ZFN, a meganuclease, a base editor complex, a restriction endonuclease, including FokI or a variant thereof, or two site-specific nicking endonucleases, or a variant or a catalytically active fragment thereof.

In a further aspect, there is provided a plant cell, tissue, organ, whole plant or plant material, or a derivative or a progeny thereof, obtainable by a method according to any one of the embodiments of the methods of the present invention.

Further, the present application disclosesa method for identifying at least one gene involved in increased pathogen resistance, preferably increased fungal resistance, in a plant cell, tissue, organ, whole plant, or plant material as compared to a corresponding control plant cell, tissue, organ, whole plant, or plant material the method comprising: (i) determining the genotype of at least one plant cell, tissue, organ, whole plant, or plant material with respect to the presence of at least one gene encoding a wall-associated kinase in the genome of said plant cell, tissue, organ, whole plant or plant material; (ii) optionally: determining the benzoxazinoid signature of the at least one plant cell, tissue, organ, whole plant, or plant material of step (i); (iii) exposing the at least one plant cell, tissue, organ, whole plant, or plant material of step (i) or (ii) to a stimulus, optionally wherein the stimulus is correlated with the benzoxazinoid signature in the at least one plant cell, tissue, organ, whole plant, or plant material, preferably wherein the stimulus is associated with a fungal pathogen infection; (iv) performing an analysis of at least one analyte obtained from the at least one plant cell, tissue, organ, whole plant, or plant material of step (i) or (ii) after exposition to the stimulus; (v) determining at least one gene being regulated upon exposition to a stimulus according to step (iii) in at least one cell of the at least one plant cell, tissue, organ, whole plant, or plant material as derivable from the analysis of at least one analyte as defined in step (iv), (vi) subjecting the at least one gene as determined in step (v) to a functional characterization; and (vii) providing at least one gene involved in increased pathogen resistance, preferably increased fungal resistance, in a plant cell, tissue, organ, whole plant, or plant material.

Further disclosed is a plant cell, tissue, organ, whole plant or plant material, or a derivative or a progeny thereof, obtainable by introducing at least one gene as provided by the method for identifying at least one gene involved in increased pathogen resistance into at least one cell of at least one of a plant cell, tissue, organ, or whole plant.

In another embodiment of the present application discloses a plant cell, tissue, organ, whole plant or plant material, or a derivative or a progeny thereof, obtainable by the methods of the present invention, wherein the introduction of at least one gene as provided by the method for identifying at least one gene involved in increased pathogen resistance is a stable introduction, preferably a stable introduction mediated by means of molecular biology, comprising genome editing, or a combination thereof.

In yet a further aspect of the present application describes a method of increasing pathogen resistance, preferably fungal resistance, in a plant cell, tissue, organ, whole plant, or plant material as compared to a corresponding control plant cell, tissue, organ, whole plant, or plant material, the method comprising: (i) providing at least one plant cell, tissue, organ, whole plant or plant material; (ii) (a) treating the at least one plant cell, tissue, organ, whole plant or plant material according to step (i) with a substance neutralizing the effect of at least one benzoxazinoid, and/or (ii) (b) treating the at least one plant cell, tissue, organ, whole plant or plant material according to step (i) with a substance activating the signalling pathway downstream of at least one wall-associated kinase; and/or (ii) (c) modifying at least one promoter or at least one regulatory sequence of at least one gene of the at least one plant cell, tissue, organ, whole plant or plant material of step (i), wherein said at least promoter or at least one regulatory sequence is involved in the regulation of transcription of at least on gene involved in the signalling pathway of, or downstream of at least one wall-associated kinase; (iii) reducing the amount of at least one benzoxazinoid and thereby increasing pathogen resistance, preferably fungal resistance, in at least one plant cell, tissue, organ, whole plant, or plant material.

Further disclosed is a use of a substance as defined for the aspect of a method of increasing pathogen resistance for increasing pathogen resistance, preferably fungal resistance, in at least one plant cell, tissue, organ, whole plant, or plant material.

### Definitions

The term "active fragment" or "functional fragment" as used herein referring to amino acid sequences denotes the core sequence derived from a given template amino acid sequence, or a nucleic acid sequence encoding the same, comprising all or part of the active site of the template sequence with the proviso that the resulting catalytically active fragment still possesses the activity characterizing the template sequence, for which the active site of the native enzyme or a variant thereof is responsible. Said modifications are suitable to generate less bulky amino acid sequences still having the same activity as a template sequence making the catalytically active fragment a more versatile or more stable tool being sterically less demanding. For amino acid sequences not representing enzymes, the term "functional fragment" can also imply that part or domain of the amino acid sequence involved in interaction with another molecule, and/or involved in any structural function within the cell.

An "allele" or "allelic variant" as used herein refers to a variant form of a given gene. As most multicellular organisms have two sets of chromosomes; that is, they are diploid (or, if more chromosome sets are present, they are polyploidy), these chromosomes are referred to as homologous chromosomes. If both alleles at a gene (or locus) on the homologous chromosomes are the same, they and the organism are homozygous with respect to that gene (or locus). If the alleles are different, they and the organism are heterozygous with respect to that gene. Alleles can result in the same, or a different observable phenotype. The term "allele" thus refers to one or two or more nucleotide sequences at a specific locus in the genome. A first allele is on a chromosome, a second on a second chromosome at the same position. If the two alleles are different, they are heterozygous, and if they are the same, they are homozygous. Various alleles of a gene (gene alleles) differ in at least one SNP (single nucleotide polymorphism).

"Complementary" or "complementarity" as used herein describes the relationship between two DNA, two RNA, or, regarding hybrid sequences between an RNA and a DNA nucleic acid region. Defined by the nucleobases of the DNA or RNA, two nucleic acid regions can hybridize to each other in accordance with the lock-and-key model. To this end the principles of Watson-Crick base pairing have the basis adenine and thymine/uracil as well as guanine and cytosine, respectively, as complementary bases apply. Furthermore, also non-Watson-Crick pairing, like reverse-Watson-Crick, Hoogsteen, reverse-Hoogsteen and Wobble pairing are comprised by the term "complementary" as used herein as long as the respective base pairs can build hydrogen bonding to each other, i.e., two different nucleic acid strands can hybridize to each other based on said complementarity.

The term "construct", especially "genetic construct", or "recombinant construct", or "expression construct" as used herein refers to a construct comprising, *inter alia*, plasmids or plasmid vectors, cosmids, artificial yeast chromosomes or bacterial artificial chromosomes (YACs and BACs), phagemides, bacterial phage based vectors, an expression cassette, isolated single-stranded or double-stranded nucleic acid sequences, comprising DNA and RNA sequences, or amino acid sequences, viral vectors, including modified viruses, and a combination or a mixture thereof, for introduction or transformation, transfection or transduction into a target cell or plant, plant cell, tissue, organ or material according to the present disclosure.

The term "delivery construct" or "delivery vector" as used herein refers to any biological or chemical means used as a cargo for transporting a nucleic acid, including a hybrid nucleic acid comprising RNA and DNA, and/or an amino acid sequence of interest into a target cell, preferably a eukaryotic cell. The term delivery construct or vector as used herein thus refers to a means of transport to deliver a genetic or a recombinant construct according to the present disclosure into a target cell, tissue, organ or an organism. A vector can thus comprise nucleic acid sequences, optionally comprising sequences like regulatory sequences or localization sequences for delivery, either directly or indirectly, into a target cell of interest or into a plant target structure in the desired cellular compartment of a plant. A vector can also be used to introduce an amino acid sequence or a ribonucleo-molecular complex into a target cell or target structure. Usually, a vector as used herein can be a plasmid vector. Furthermore, according to certain preferred embodiments according to the present invention, a direct introduction of a construct or sequence or complex of interest is conducted. The term direct introduction implies that the desired target cell or target structure containing a DNA target sequence to be modified according to the present disclosure is directly transformed or transduced or transfected into the specific target cell of interest, where the material delivered with the delivery vector will exert its effect. The term indirect introduction implies that the introduction is achieved into a structure, for example, cells of leaves or cells of organs or tissues, which do not themselves represent the actual target cell or structure of interest to be transformed, but those structures serve as basis for the systemic spread and transfer of the vector, preferably comprising a genetic construct according to the present disclosure to the actual target structure, for example, a meristematic cell or tissue, or a stem cell or tissue. In case the term vector is used in the context of transfecting amino acid sequences and/or nucleic sequences, including hybrid nucleic acid sequences, into a target cell the term vector implies suitable agents for peptide or protein transfection, like for example ionic lipid mixtures, cell penetrating peptides (CPPs), or particle bombardment. In the context of the introduction of nucleic acid material, the term vector cannot only imply plasmid vectors but also suitable carrier materials which can serve as basis for the introduction of nucleic acid and/or amino acid sequence delivery into a target cell of interest, for example by means of particle bombardment. Said carrier material comprises, *inter alia*, gold or tungsten particles. Finally, the term vector also implies the use of viral vectors for the introduction of at least one genetic construct according to the present disclosure like, for example, modified viruses and bacterial vectors, like for example *Agrobacterium spp.*, like for example *Agrobacterium tumefaciens.* Finally, the term vector also implies suitable chemical transport agents for introducing linear nucleic acid sequences (single- or double-stranded), or amino sequences, or a combination thereof into a target cell combined with a physical introduction method, including polymeric or lipid-based delivery constructs.

Suitable "delivery constructs" or "vectors" thus comprise biological means for delivering nucleotide and/or amino acid sequences into a target cell, including viral vectors, *Agrobacterium spp.*, or chemical delivery constructs, including nanoparticles, e.g., mesoporous silica nanoparticles (MSNPs), cationic polymers, including PEI (polyethylenimine) polymer based approaches or polymers like DEAE-dextran, or non-covalent surface attachment of PEI to generate cationic surfaces, lipid or polymeric vesicles, or combinations thereof. Lipid or polymeric vesicles may be selected, for example, from lipids, liposomes, lipid encapsulation systems, nanoparticles, small nucleic acid-lipid particle formulations, polymers, and polymersomes.

The term "derivative" or "descendant" or "progeny" as used herein in the context of a prokaryotic or a eukaryotic cell, preferably a plant or plant cell or plant material according to the present disclosure relates to the descendants of such a cell or material which result from natural reproductive propagation including sexual and asexual propagation. It is well known to the person having skill in the art that said propagation can lead to the introduction of mutations into the genome of an organism resulting from natural phenomena which results in a descendant or progeny, which is genomically different to the parental organism or cell, however, still belongs to the same genus/species and possesses mostly the same characteristics as the parental recombinant host cell. Such derivatives or descendants or progeny resulting from natural phenomena during reproduction or regeneration are thus comprised by the term of the present disclosure. These terms, therefore, do not refer to any arbitrary derivative, descendant or progeny, but rather to a derivative, or descendant or progeny phylogenetically associated with, i.e., based on, a parent cell thereof, whereas this relationship between the derivative, descendant or progeny and the "parent" is clearly inferable by a person skilled in the art. "Progeny" comprises any subsequent generation of a plant, plant cell, plant tissue, or plant organ.

Furthermore, the term "derivative" can imply, in the context of a substance or molecule rather than referring to a cell or organism, directly or by means of modification indirectly obtained from another. This might imply a nucleic acid sequence derived from a cell or a plant metabolite obtained from a cell or material.

Furthermore, the terms "derived", or "derived from " as used herein in the context of a biological sequence (nucleic acid or amino acid) or a molecule or a complex imply that the respective sequence is based on a reference sequence, for example from the sequence listing, or a database accession number, or the respective scaffold structure, i.e., originating from said sequence, whereas the reference sequence can comprise more sequences, e.g., the whole genome or a full polyprotein encoding sequence, of a virus, whereas the sequence "derived from" the native sequence may only comprise one isolated fragment thereof, or a coherent fragment thereof. In this context, a cDNA molecule or a RNA can be said to be "derived from" a DNA sequence serving as molecular template. The skilled person can thus easily define a sequence "derived from" a reference sequence, which will, by sequence alignment on DNA or amino acid level, have a high identity to the respective reference sequence and which will have coherent stretches of DNA/amino acids in common with the respective reference sequence (>75% query identity for a given length of the molecule aligned provided that the derived sequence is the query and the reference sequence represents the subject during a sequence alignment). The skilled person can thus clone the respective sequences based on the disclosure provided herein by means of polymerase chain reactions and the like into a suitable vector system of interest, or use a sequence as vector scaffold. The term "derived from" is thus no arbitrary sequence, but a sequence corresponding to a reference sequence it is derived from, whereas certain differences, e.g., certain mutations naturally occurring during replication of a recombinant construct within a host cell, cannot be excluded and are thus comprised by the term "derived from". Furthermore, several sequence stretches from a parent sequence can be concentrated in a sequence derived from the parent. The different stretches will have high or even 100% homology to the parent sequence.

The term an "endogenous" in the context of nucleic acid and/or amino acid sequences refers to the nucleic acid and/or amino acid as found in a plant genome in its natural form and natural genetic context. As it is known to the skilled person, several variants, e.g., allelic variants, of a gene nucleic acid sequence may exist in a given species of plants.

A "fungus" or "fungal pathogen" as used herein means any plant pathogenic fungus including oomycetes in any developmental stage, including spores, or any part of such a fungus, which can interact with a plant or plant part or cell to induce a response in said plant or plant part or cell.

As used herein, "fusion" can refer to a protein and/or nucleic acid comprising one or more non-native sequences (e.g., moieties). A fusion can be at the N-terminal or C-terminal end of the modified protein, or both, or within the molecule as separate domain. For nucleic acid molecules, the fusion molecule can be attached at the 5' or 3' end, or at any suitable position in between. A fusion can be a transcriptional and/or translational fusion. A fusion can comprise one or more of the same non-native sequences. A fusion can comprise one 10 or more of different non-native sequences. A fusion can be a chimera. A fusion can comprise a nucleic acid affinity tag. A fusion can comprise a barcode. A fusion can comprise a peptide affinity tag. A fusion can provide for subcellular localization of the site-specific effector or base editor (e.g., a nuclear localization signal (NLS) for targeting to the nucleus, a mitochondrial localization signal for targeting to the mitochondria, a chloroplast localization signal for targeting to a chloroplast, an endoplasmic reticulum (ER) retention signal, and the like). A fusion can provide a non-native sequence (e.g., affinity tag) that can be used to track or purify. A fusion can be a small molecule such as biotin or a dye such as alexa fluor dyes, Cyanine3 dye, Cyanine5 dye. The fusion can provide for increased or decreased stability. In some embodiments, a fusion can comprise a detectable label, including a moiety that can provide a detectable signal. Suitable detectable labels and/or moieties that can provide a detectable signal can include, but are not limited to, an enzyme, a radioisotope, a member of a specific binding pair; a fluorophore; a fluorescent reporter or fluorescent protein; a quantum dot; and the like. A fusion can comprise a member of a FRET pair, or a fluorophore/quantum dot donor/acceptor pair. A fusion can comprise an enzyme. Suitable enzymes can include, but are not limited to, horse radish peroxidase, luciferase, beta-25 galactosidase, and the like. A fusion can comprise a fluorescent protein. Suitable fluorescent proteins can include, but are not limited to, a green fluorescent protein (GFP), (e.g., a GFP from *Aequoria victoria*, fluorescent proteins from *Anguilla japonica*, or a mutant or derivative thereof), a red fluorescent protein, a yellow fluorescent protein, a yellow-green fluorescent protein (e.g., mNeonGreen derived from a tetrameric fluorescent protein from the cephalochordate *Branchiostoma lanceolatum*) any of a variety of fluorescent and colored proteins. A fusion can comprise a nanoparticle. Suitable nanoparticles can include fluorescent or luminescent nanoparticles, and magnetic nanoparticles, or nanodiamonds, optionally linked to a nanoparticle. Any optical or magnetic property or characteristic of the nanoparticle(s) can be detected. A fusion can comprise a helicase, a nuclease (e.g., Fokl), an endonuclease, an exonuclease (e.g., a 5' exonuclease and/or 3' exonuclease), a ligase, a nickase, a nuclease-helicase (e.g., Cas3), a DNA methyltransferase (e.g., Dam), or DNA demethylase, a histone methyltransferase, a histone demethylase, an acetylase (including for example and not limitation, a histone acetylase), a deacetylase (including for example and not limitation, a histone deacetylase), a phosphatase, a kinase, a transcription (co-) activator, a transcription (co-) factor, an RNA polymerase subunit, a transcription repressor, a DNA binding protein, a DNA structuring protein, a long non-coding RNA, a DNA repair protein (e.g., a protein involved in repair of either single- and/or double-stranded breaks, e.g., proteins involved in base excision repair, nucleotide excision repair, mismatch repair, NHEJ, HR, microhomology-mediated end joining (MMEJ), and/or alternative non-homologous end-joining (ANHEJ), such as for example and not limitation, HR regulators and HR complex assembly signals), a marker protein, a reporter protein, a fluorescent protein, a ligand binding protein (e.g., mCherry or a heavy metal binding protein), a signal peptide (e.g., Tat-signal sequence), a targeting protein or peptide, a subcellular localization sequence (e.g., nuclear localization sequence, a chloroplast localization sequence), and/or an antibody epitope, or any combination thereof.

The term "genetically modified" or "genetic manipulation" or "genetic(ally) manipulated" is used in a broad sense herein and means any modification of a nucleic acid sequence or an amino acid sequence, a target cell, tissue, organ or organism, which is accomplished by human intervention, either directly or indirectly, to influence the endogenous genetic material or the transciptome or the proteome of a target cell, tissue, organ or organism to modify it in a purposive way so that it differs from its state as found without human intervention. The human intervention can either take place *in vitro* or *in vivo*/*in planta*, or also both. Further modifications can be included, for example, one or more point mutation(s), e.g. for targeted protein engineering or for codon optimization, deletion(s), and one or more insertion(s) or deletion(s) of at least one nucleic acid or amino acid molecule (including also homologous recombination), modification of a nucleic acid or an amino acid sequence, or a combination thereof. The terms shall also comprise a nucleic acid molecule or an amino acid molecule or a host cell or an organism, including a plant or a plant material thereof which is/are similar to a comparable sequence, organism or material as occurring in nature, but which have been constructed by at least one step of purposive manipulation. A "targeted genetic manipulation" or "targeted (base) modification" as used herein is thus the result of a "genetic manipulation", which is effected in a targeted way, i.e. at a specific position in a target cell and under the specific suitable circumstances to achieve a desired effect in at least one cell, preferably a plant cell, to be manipulated, wherein the term implies that the sequence to be targeted and the corresponding modification are based on preceding sequence considerations so that the resulting modification can be planned in advance, e.g., based on available sequence information of a target site in the genome of a cell and/or based on the information of the target specificity (recognition or binding properties of a nucleic acid or an amino acid sequence, complementary base pairing and the like) of a molecular tool of interest.

The term "genome" refers to the entire complement of genetic material (genes and non-coding sequences) that is present in each cell of an organism, or virus or organelle, and/or a complete set of chromosomes inherited as a (haploid) unit from one parent. The genome thus also defines the "genotype" being the part of the genetic makeup of a given cell, and therefore of an organism or individual, which determines a specific characteristic (phenotype) of that cell/organism/individual.

The terms "genome editing", "genome engineering", or "gene editing/engineering" are used interchangeably herein and refer to strategies and techniques for the targeted, specific modification of any genetic information or genome of a living organism. As such, the terms comprise gene editing, but also the editing of regions other than gene encoding regions of a genome. It further comprises the editing or engineering of the nuclear (if present) as well as other genetic information of a cell. Furthermore, the terms "genome editing" and "genome engineering" also comprise an epigenetic editing or engineering, i.e., the targeted modification of, e.g., methylation, histone modification or of non-coding RNAs possibly causing heritable changes in gene expression.

"Germplasm", as used herein, is a term used to describe the genetic resources, or more precisely the DNA of an organism and collections of that material. In breeding technology, the term germplasm is used to indicate the collection of genetic material from which a new plant or plant variety can be created.

The terms "guide RNA", "gRNA" or "single guide RNA" or "sgRNA" are used interchangeably herein and either refer to a synthetic fusion of a CRISPR RNA (crRNA) and a trans-activating crRNA (tracrRNA), or the term refers to a single RNA molecule consisting only of a crRNA and/or a tracrRNA, or the term refers to a gRNA individually comprising a crRNA or a tracrRNA moiety. The tracr and the crRNA moiety thus do not necessarily have to be present on one covalently attached RNA molecule, yet they can also be comprised by two individual RNA molecules, which can associate or can be associated by non-covalent or covalent interaction to provide a gRNA according to the present disclosure. The terms "gDNA" or "sgDNA" or "guide DNA" are used interchangeably herein and either refer to a nucleic acid molecule interacting with an Argonaute nuclease. Both, the gRNAs and gDNAs as disclosed herein are termed "guiding nucleic acids" or "guide nucleic acids" due to their capacity to interacting with a site-specific nuclease and to assist in targeting said site-specific nuclease to a genomic target site.

The term "hybridization" as used herein refers to the pairing of complementary nucleic acids, i.e., DNA and/or RNA, using any process by which a strand of nucleic acid joins with a complementary strand through base pairing to form a hybridized complex. Hybridization and the strength of hybridization (i.e., the strength of the association between the nucleic acids) is impacted by such factors as the degree and length of complementarity between the nucleic acids, stringency of the conditions involved, the Tm of the formed hybrid, and the G:C ratio within the nucleic acids. The term hybridized complex refers to a complex formed between two nucleic acid sequences by virtue of the formation of hydrogen bounds between complementary G and C bases and between complementary A and T/U bases. A hybridized complex or a corresponding hybrid construct can be formed between two DNA nucleic acid molecules, between two RNA nucleic acid molecules or between a DNA and an RNA nucleic acid molecule. For all constellations, the nucleic acid molecules can be naturally occurring nucleic acid molecules generated *in vitro* or *in vivo* and/or artificial or synthetic nucleic acid molecules. Hybridization as detailed above, e.g., Watson-Crick base pairs, which can form between DNA, RNA and DNA/RNA sequences, are dictated by a specific hydrogen bonding pattern, which thus represents a non-covalent attachment form according to the present invention. In the context of hybridization, the term "stringent hybridization conditions" should be understood to mean those conditions under which a hybridization takes place primarily only between homologous nucleic acid molecules. The term "hybridization conditions" in this respect refers not only to the actual conditions prevailing during actual agglomeration of the nucleic acids, but also to the conditions prevailing during the subsequent washing steps. Examples of stringent hybridization conditions are conditions under which primarily only those nucleic acid molecules that have at least at least 80%, preferably at least 85%, at least 90% or at least 95% sequence identity undergo hybridization. Stringent hybridization conditions are, for example: 4×SSC at 65°C and subsequent multiple washes in 0.1×SSC at 65°C. for approximately 1 hour. The term "stringent hybridization conditions" as used herein may also mean: hybridization at 68°C in 0.25 M sodium phosphate, pH 7.2, 7% SDS, 1 mM EDTA and 1% BSA for 16 hours and subsequently washing twice with 2×SSC and 0.1% SDS at 68°C. Preferably, hybridization takes place under stringent conditions.

The term "introgression" as used herein refers to the transfer of at least one allele of a gene of interest on a genetic locus from one genetic background to another. For example, introgression can proceed through sexual crossing of two parents of the same species. Alternatively, the transfer of a gene allele can take place by recombination between two donor genomes, e.g., in a fused protoplast, wherein at least the donor protoplast carries the gene allele of interest in its genome. In any case, any progeny or derivatives comprising the gene allele of interest can then be subjected to repeated back-crossing steps with a plant line carrying a genetic background of interest to select for the gene allele of interest in the resulting derivatives or progeny. The result may be the fixation of the gene allele of interest such introgressed in a selected genetic background. The whole process of introgression can, for example, take place by a mixture of breeding strategies and techniques of molecular biology to achieve at a genotype/phenotype of interest for a given germplasm, plant, plant cell or plant material.

The term "locus" generally refers to a genetically defined region of a chromosome carrying a gene or, possibly, two or more genes so closely linked that genetically they behave as a single locus responsible for a phenotype.

As used herein, the terms "mutation" and "modification" are used interchangeably to refer to a deletion, insertion, addition, substitution, edit, strand break, and/or introduction of an adduct in the context of nucleic acid manipulation *in vivo* or *in vitro.* A deletion is defined as a change in a nucleic acid sequence in which one or more nucleotides is absent. An insertion or addition is that change in a nucleic acid sequence which has resulted in the addition of one or more nucleotides. A "substitution" or "edit" results from the replacement of one or more nucleotides by a molecule which is a different molecule from the replaced one or more nucleotides. For example, a nucleic acid may be replaced by a different nucleic acid as exemplified by replacement of a thymine by a cytosine, adenine, guanine, or uridine. Pyrimidine to pyrimidine (e.g., C to Tor T to C nucleotide substitutions) or purine to purine (e.g., G to A or A to G nucleotide substitutions) are termed transitions, whereas pyrimidine to purine or purine to pyrimidine (e.g., G to T or G to C or A to T or A to C) are termed transversions. Alternatively, a nucleic acid may be replaced by a modified nucleic acid as exemplified by replacement of a thymine by thymine glycol. Mutations may result in a mismatch. The term mismatch refers to a non-covalent interaction between two nucleic acids, each nucleic acid residing on a different nucleotide sequence or nucleic acid molecule, which does not follow the base-pairing rules. For example, for the partially complementary sequences 5'-AGT-3' and 5'-AAT-3', a G-A mismatch (a transition) is present.

"Near isogenic lines" or "NILs" as used herein are useful for identifying genes responsible for a phenotypic trait by mapping them to genetic chromosomes by analyzing NILs. To create a near isogenic line, an organism with the phenotype of interest, often a plant, is crossed with a standard line of the same plant. The F1 generation is selfed to produce the F2 generation. F2 individuals with the target trait are selected for crossing with the standard line (the recurrent parent). This process is repeated for several generations. The genetic make-ups of sister lines can be compared. Alleles derived from the donor parent that can be found in all sister lines are said to be associated with the trait.

The terms "nucleotide" and "nucleic acid" with reference to a sequence or a molecule are used interchangeably herein and refer to a single- or double-stranded DNA or RNA of natural or synthetic origin. The term nucleotide sequence is thus used for any DNA or RNA sequence independent of its length, so that the term comprises any nucleotide sequence comprising at least one nucleotide, but also any kind of larger oligonucleotide or polynucleotide. The term(s) thus refer to natural and/or synthetic deoxyribonucleic acids (DNA) and/or ribonucleic acid (RNA) sequences, which can optionally comprise synthetic nucleic acid analoga. A nucleic acid according to the present disclosure can optionally be codon optimized. "Codon optimization" implies that the codon usage of a DNA or RNA is adapted to that of a cell or organism of interest to improve the transcription rate of said recombinant nucleic acid in the cell or organism of interest. The skilled person is well aware of the fact that a target nucleic acid can be modified at one position due to the codon degeneracy, whereas this modification will still lead to the same amino acid sequence at that position after translation, which is achieved by codon optimization to take into consideration the species-specific codon usage of a target cell or organism. Nucleic acid sequences according to the present application can carry specific codon optimization for the following non limiting list of organisms: *Hordeum vulgare, Sorghum bicolor, Secale cereale, Saccharum officinarium, Zea mays, Setaria italic, Oryza sativa, Oryza minuta, Oryza australiensis, Oryza a*/*ta, Triticum aestivum, Triticum durum,* Triticale, *Hordeum bulbosum, Brachypodium distachyon, Hordeum marinum, Aegilops tauschii, Malus domestica, Beta vulgaris, Helianthus annuus, Daucus glochidiatus, Daucus pusillus, Daucus muricatus, Daucus carota, Eucalyptus grandis, Erythranthe guttata, Genlisea aurea, Nicotiana sylvestris, Nicotiana tabacum, Nicotiana tomentosiformis, Nicotiana benthamiana, Solanum lycopersicum, Solanum tuberosum, Coffea canephora, Vitis vinifera, Cucumis sativus, Marus notabilis, Arabidopsis thaliana, Arabidopsis lyrata, Arabidopsis arenosa, Crucihimalaya himalaica, Crucihimalaya wallichii, Cardamine flexuosa, Lepidium virginicum, Capsella bursa-pastoris, Olmarabidopsis pumila, Arabis hirsuta, Brassica napus, Brassica oleracea, Brassica rapa, Brassica juncacea, Brassica nigra, Raphanus sativus, Eruca vesicaria sativa, Citrus sinensis, Jatropha curcas, Glycine max, Gossypium ssp., or Populus trichocarpa*

The term "particle bombardment" as used herein, also named "biolistic transfection" or "microparticle-mediated gene transfer", refers to a physical delivery method for transferring a coated microparticle or nanoparticle comprising a nucleic acid or a genetic construct of interest into a target cell or tissue. The micro or nanoparticle functions as projectile and is fired on the target structure of interest under high pressure using a suitable device, often called gene-gun. The transformation via particle bombardment uses a microprojectile of metal covered with the gene of interest, which is then shot onto the target cells using an equipment known as "gene gun" (Sanford, John C., et al. "Delivery of substances into cells and tissues using a particle bombardment process." Particulate Science and Technology 5.1 (1987): 27-37) at high velocity fast enough (∼1500 km/h) to penetrate the cell wall of a target tissue, but not harsh enough to cause cell death. For protoplasts, which have their cell wall entirely removed, the conditions are different logically. The precipitated nucleic acid or the genetic construct on the at least one microprojectile is released into the cell after bombardment, and integrated into the genome. The acceleration of microprojectiles is accomplished by a high voltage electrical discharge or compressed gas (helium). Concerning the metal particles used it is mandatory that they are non-toxic, non-reactive, and that they have a lower diameter than the target cell. The most commonly used are gold or tungsten. There is plenty of information publicly available from the manufacturers and providers of gene-guns and associated system concerning their general use.

A "pathogen" as used herein refers to an organism which can infect a plant, or which can cause a disease in a plant. Pathogens which can infect a plant, or which can cause a disease in a plant, include fungi, oomycetes, bacteria, viruses, viroids, virus-like organisms, phytoplasmas, protozoa, nematodes and parasitic plants. Plant parasites can cause damage by feeding on a plant and can be selected from ectoparasites like insects, comprising aphids and other sap-sucking insect, mites, and vertebrates.

The term "plant" as used herein is to be construed broadly and refers to a whole plant organism, a plant organ, differentiated and undifferentiated plant tissues, plant cells, seeds, and derivatives and progeny thereof. "Plant cells" include without limitation, for example, cells from seeds, from mature and immature embryos, meristematic tissues, seedlings, callus tissues in different differentiation states, leaves, flowers, roots, shoots, gametophytes, grains, kernels, sporophytes, pollen and microspores, protoplasts, macroalgae and microalgae. The different plant cells can either be haploid, diploid or multiploid. The term "plant organ" refers to plant tissue or a group of tissues that constitute a morphologically and functionally distinct part of a plant. Typically, the term "grain" is used to describe the mature kernel produced by a plant grower for purposes other than growing or reproducing the species, and "seed" means the mature kernel used for growing or reproducing the species. For the purposes of the present invention, "grain", "seed", and "kernel", will be used interchangeably.

A "plant material" as used herein refers to any material which can be obtained from a plant during any developmental stage. The plant material can be obtained either *in planta* or from an *in vitro* culture of the plant or a plant tissue or organ thereof. The term thus comprises plant cells, tissues and organs as well as developed plant structures as well as sub-cellular components like nucleic acids, polypeptides and all chemical plant substances or metabolites which can be found within a plant cell or compartment and/or which can be produced by the plant, or which can be obtained from an extract of any plant cell, tissue or a plant in any developmental stage. The term also comprises a derivative of the plant material, e.g., a protoplast, derived from at least one plant cell comprised by the plant material. The term therefore also comprises meristematic cells or a meristematic tissue of a plant.

A "control" or "control plant cell" or "control tissue" or "control organ" or "control plant" provides a reference point for measuring changes in phenotype of a subject plant or plant part in which (genetic) modification, modulation and/or alteration, such as indicated in the various aspects of the present invention, has been affected to a gene, a protein or a substance or molecule of interest. A control plant or control plant part (e.g. control plant cell, control tissue, or control organ) may comprise, *for example*: (a) a wild-type plant or cell, *i.e.*, of the same genotype as the starting material for the (genetic) modification, modulation and/or alteration which resulted in the subject plant or plant part; (b) a plant or plant part of the same genotype as the starting material but which has been transformed with a null construct (i.e., with a construct which has no known effect on the trait of interest, such as a construct comprising a marker gene); or (c) a plant or plant part which is a non-transformed segregant among progeny of a subject plant or plant part. In particular, a control plant or control plant cell may comprise a plant or plant part of the same genotype, but lacking the modification of the at least one gene encoding at least one wall-associated kinase or the modulation of the expression level of at least one wall-associated kinase and/or the transcription level, the expression level, or the function of at least one molecule within the signaling pathway from the at least one wall-associated kinase to the synthesis of at least one benzoxazinoid or within the synthesis pathway of at least one benzoxazinoid.

A "plasmid" refers to a circular autonomously replicating extrachromosomal element in the form of a double-stranded nucleic acid sequence. In the field of genetic engineering these plasmids are routinely subjected to targeted modifications by inserting, for example, genes encoding a resistance against an antibiotic or an herbicide, a gene encoding a target nucleic acid sequence, a localization sequence, a regulatory sequence, a tag sequence, a marker gene, including an antibiotic marker or a fluorescent marker, and the like. The structural components of the original plasmid, like the origin of replication, are maintained. According to certain embodiments of the present invention, the localization sequence can comprise a nuclear localization sequence, a plastid localization sequence, preferably a mitochondrion localization sequence or a chloroplast localization sequence, or a localization sequence for targeting a kinase of interest to the plasma membrane of a cell of interest. Said localization sequences are available to the skilled person in the field of plant biotechnology. A variety of plasmid vectors for use in different target cells of interest is commercially available and the modification thereof is known to the skilled person in the respective field.

The terms "protein", "amino acid" or "polypeptide" are used interchangeably herein and refer to an amino acid sequence having a catalytic enzymatic function or a structural or a functional effect. The term "amino acid" or "amino acid sequence" or "amino acid molecule" comprises any natural or chemically synthesized protein, peptide, polypeptide and enzyme or a modified protein, peptide, polypeptide and enzyme, wherein the term "modified" comprises any chemical or enzymatic modification of the protein, peptide, polypeptide and enzyme, including truncations of a wild-type sequence to a shorter, yet still active portion. In accordance with the present invention there may be employed conventional molecular biology, microbiology, and recombinant DNA techniques within the skill of the art. Such techniques are explained fully in the literature. See, e.g., Sambrook, Fritsch & Maniatis, Molecular Cloning: A Laboratory Manual, Second Edition (1989) Cold Spring Harbor Laboratory Press, Cold Spring Harbor, New York (herein "Sambrook et al., 1989"); DNA Cloning: A Practical Approach, Volumes I and II (D.N. Glover ed. 1985); Oligonucleotide Synthesis (M.J. Gait ed. 1984); Nucleic Acid Hybridization (B.D. Hames & SJ. Higgins eds. (1985); Transcription and Translation (B.D. Hames & S.J. Higgins, eds. (1984); among others.

The term "regulatory sequence" or "regulatory region" as used herein refers to a nucleic acid or an amino acid sequence, which can direct and/or influence the transcription and/or translation and/or modification of a nucleic acid sequence of interest. A regulatory sequence may be a promoter sequence, an enhancer, a silencer, a transcription factor and the like.

The terms "resistance" or "tolerance" or "resistant" or "tolerant" as used herein refers to the capacity of a plant to resist to the phenotype as caused by infestation with a pathogen, in particular a fungal pathogen, disclosed herein to a certain degree, i.e., the prevention, reduction or delay of an infection caused by a (fungal) pathogen. "Resistance"/"Tolerance", therefore, does not exclusively refer to a "black or white" phenotype implying a phenotype where no symptoms occur at all after infestation for a resistant plant. The terms "resistance" or "tolerance" or "resistant" or "tolerant" rather imply a gradual improvement of infestation symptoms as observed for a plant having no resistance to a given pathogen. A classification score scheme for phenotyping experiments in field trials at various locations with natural and artificial *H. turcicum* inoculation (from the Deutsche Maiskomitee (DMK, German maize committee); AG variety 27.02.02; (DMK J. Rath; RP Freiburg H. J. Imgraben) shows a resistance level from 9 (low) to 1 (high) for maize as exemplary plant, wherein each score represents the following phenotype: 1: Plants exhibit no symptoms of disease, 0%; 2: Beginning of infestation, first small spots (less than 2 cm) visible. Less than 5% of leaf surface affected. 3: Some spots have developed on a leaf stage. Between 5-10% of leaf surface affected. 4: 10-20% of leaf surface affected. Clearly visible spots on several leaf stages. 5: 20-40% of leaf surface affected. Spots start to coalesce. 6: 40-60% of leaf surface affected. Systematic infestation visible on leaves. 7: 60-80% of leaf surface affected. Approximately half of leaves destroyed or dried out because of fungal infestation. 8: 80-90% of leaf surface affected. More than half of leaves destroyed or dried out because of fungal infestation. 9: 90-100% of leaf surface affected. The plants are almost completely dried out.

The term "TILLING" as used herein is an abbreviation for "Targeting Induced Local Lesions in Genomes" and describes a well-known reverse genetics technique designed to detect unknown SNPs (single nucleotide polymorphisms) in genes of interest using an enzymatic digestion and is widely employed in plant genomics. The technique allows for the high-throughput identification of an allelic series of mutants with a range of modified functions for a particular gene. TILLING combines mutagenesis (e.g., chemical or via UV-light) with a sensitive DNA screening-technique that identifies single base mutations.

The terms "transgene" or "transgenic" as used herein refer to at least one nucleic acid sequence that is taken from the genome of one organism, or produced synthetically, and which is then introduced into a host cell or organism or tissue of interest and which is subsequently integrated into the host' s genome by means of "stable" transformation or transfection approaches. In contrast, the term "transient" transformation or transfection or introduction refers to a way of introducing molecular tools including at least one nucleic acid (comprising at least one of DNA, RNA, single-stranded or double-stranded or a mixture thereof) and/or at least one amino acid sequence, optionally comprising suitable chemical or biological agents, to achieve a transfer into at least one compartment of interest of a cell, including, but not restricted to, the cytoplasm, an organelle, including the nucleus, a mitochondrion, a vacuole, a chloroplast, or into a membrane, resulting in transcription and/or translation and/or association and/or activity of the at least one molecule introduced without achieving a stable integration or incorporation and thus inheritance of the respective at least one molecule introduced into the genome of a cell.

The term "transient introduction" as used herein thus refers to the transient introduction of at least one nucleic acid and/or amino acid sequence according to the present disclosure, preferably incorporated into a delivery vector or into a recombinant construct, with or without the help of a delivery vector, into a target structure, for example, a plant cell, wherein the at least one nucleic acid sequence is introduced under suitable reaction conditions so that no integration of the at least one nucleic acid sequence into the endogenous nucleic acid material of a target structure, the genome as a whole, occurs, so that the at least one nucleic acid sequence will not be integrated into the endogenous DNA of the target cell. As a consequence, in the case of transient introduction, the introduced genetic construct will not be inherited to a progeny of the target structure, for example a prokaryotic or a plant cell. The at least one nucleic acid and/or amino acid sequence or the products resulting from transcription, translation, processing, post-translational modifications or complex building thereof are only present temporarily, i.e., in a transient way, in constitutive or inducible form, and thus can only be active in the target cell for exerting their effect for a limited time. Therefore, the at least one sequence or effector introduced via transient introduction will not be heritable to the progeny of a cell. The effect mediated by at least one sequence or effector introduced in a transient way can, however, potentially be inherited to the progeny of the target cell.

A "variant" in the context of a nucleic acid or amino acid sequence protein means a nucleic acid or amino acid sequence derived from the native nucleic acid or amino acid sequence, or another starting sequence, by deletion (so-called truncation) or addition of one or more sequences to the 5'/N-terminal and/or 37C-terminal end of the native nucleic acid or amino acid sequence; deletion or addition of one or more nucleic acid or amino acid sequence at one or more sites in the native nucleic acid or amino acid sequence; or substitution of one or more nucleic acid or amino acid sequence at one or more sites in the native protein. Variant proteins encompassed by the present invention are biologically active, that is they continue to possess all or some of the activity of the native proteins of the invention as described herein. Such variants may result from, for example, genetic polymorphism or from human manipulation.

As used herein, a "homolog" means a protein in a group of proteins that perform the same biological function, e.g. proteins that belong to the same Pfam protein family. Homologs are expressed by homologous genes. With reference to homologous genes, homologs include orthologs, e.g., genes expressed in different species that evolved from a common ancestral genes by speciation and encode proteins retain the same function, but do not include paralogs, e.g., genes that are related by duplication but have evolved to encode proteins with different functions. Homologous genes include naturally occurring alleles and artificially- created variants. Degeneracy of the genetic code provides the possibility to substitute at least one base of the protein encoding sequence of a gene with a different base without causing the amino acid sequence of the polypeptide produced from the gene to be changed. When optimally aligned, homolog proteins have typically at least about 60% identity, in some instances at least about 70%, for example about 80% or 85% and even at least about 90%, 92%, 94%, 96%, 97%, 98%, 99% or 99.5% identity, preferably over the full length of the protein; homologous genes have typically at least about 60% identity, in some instances at least about 70%, for example about 80% or 85% and even at least about 90%, 92%, 94%, 96%, 97%, 98%, 99% or 99.5% identity, preferably over the full length of the gene, in particular the coding regions of the gene.

Homologs are identified by comparison of amino acid sequence, e.g. manually or by use of a computer-based tool using known homology-based search algorithms such as those commonly known and referred to as BLAST, FASTA, and Smith-Waterman. A local sequence alignment program, e.g. BLAST, can be used to search a database of sequences to find similar sequences, and the summary Expectation value (E-value) used to measure the sequence base similarity. Because a protein hit with the best E-value for a particular organism may not necessarily be an ortholog, e.g., have the same function, or be the only ortholog, a reciprocal query is used to filter hit sequences with significant E-values for ortholog identification. The reciprocal query entails search of the significant hits against a database of amino acid sequences from the base organism that are similar to the sequence of the query protein. A hit can be identified as an ortholog, when the reciprocal query's best hit is the query protein itself or a protein encoded by a duplicated gene after speciation. A further aspect of the homologs encoded by DNA useful in the transgenic plants of the invention are those proteins that differ from a disclosed protein as the result of deletion or insertion of one or more amino acids in a native sequence.

Other functional homolog proteins differ in one or more amino acids from those disclosed herein as the result of one or more of the well-known conservative amino acid substitutions, e.g., valine is a conservative substitute for alanine and threonine is a conservative substitute for serine. Conservative substitutions for an amino acid within the native sequence can be selected from other members of a class to which the naturally occurring amino acid belongs. Representative amino acids within these various classes include, but are not limited to: (1) acidic (negatively charged) amino acids such as aspartic acid and glutamic acid; (2) basic (positively charged) amino acids such as arginine, histidine, and lysine; (3) neutral polar amino acids such as glycine, serine, threonine, cysteine, tyrosine, asparagine, and glutamine; and (4) neutral nonpolar (hydrophobic) amino acids such as alanine, leucine, isoleucine, valine, proline, phenylalanine, tryptophan, and methionine. Conserved substitutes for an amino acid within a native amino acid sequence can be selected from other members of the group to which the naturally occurring amino acid belongs. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Naturally conservative amino acids substitution groups are: valine-leucine, valine-isoleucine, phenylalanine-tyrosine, lysine-arginine, alanine- valine, aspartic acid-glutamic acid, and asparagine-glutamine. A further aspect of the invention includes proteins that differ in one or more amino acids from those of a described protein sequence as the result of deletion or insertion of one or more amino acids in a native sequence.

Whenever the present disclosure relates to the percentage of the homology or identity of nucleic acid or amino acid sequences these values define those as obtained by using the EMBOSS Water Pairwise Sequence Alignments (nucleotide) programme (www.ebi.ac.uk/Tools/psa/emboss_water/nucleotide. html) nucleic acids or the EMBOSS Water Pairwise Sequence Alignments (protein) programme (www.ebi.ac.uk/Tools/psa/emboss_water/) for amino acid sequences, preferably over the entire length of the sequence, i.e., any percentage value provided means the %homology or %identity as measured over the whole length of a subject or starting sequence in comparison to an identical or variant further sequence. Those tools provided by the European Molecular Biology Laboratory (EMBL) European Bioinformatics Institute (EBI) for local sequence alignments use a modified Smith-Waterman algorithm (see www.ebi.ac.uk/Tools/psa/ and Smith, T.F. & Waterman, M.S. "Identification of common molecular subsequences" Journal of Molecular Biology, 1981 147 (1):195-197). When conducting an alignment, the default parameters defined by the EMBL-EBI are used. Those parameters are (i) for amino acid sequences: Matrix = BLOSUM62, gap open penalty = 10 and gap extend penalty = 0.5 or (ii) for nucleic acid sequences: Matrix = DNAfull, gap open penalty = 10 and gap extend penalty = 0.5.

### Brief description of the Drawings

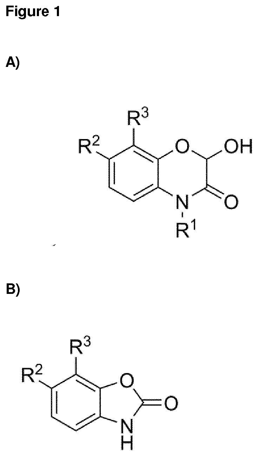

Figure 1 (Fig. 1 A and B) shows (Fig. 1 A) the structure of benzoxazinone (BXD) in the aglucone form. Derivatives from the BXD basic structure of Fig. 1 A are: HBOA (R¹=H, R²=H, R³=H), DHBOA (R¹=H, R²=OH, R³=H), HMBOA (R¹=H, R²=OMe, R³=H), HM₂BOA (R¹=H, R²=OMe, R³=OMe), DIBOA (R¹=OH, R²=H, R³=H), TRIBOA (R¹=OH, R²=OH, R³=H), DIMBOA (R¹=OH, R²=OMe, R³=H), DIM₂BOA (R¹=OH, R²=OMe, R³=OMe), HDMBOA (R¹=OMe, R²=OMe, R³=H), or HDM₂BOA (R¹=OMe, R²=OMe, R³=OMe). Fig. 1 B shows the basic structure of a benzoxazolinone, a natural degradation product of BXDs and also comprised by the term BXD as used herein. Specific benzoxazolinones are BOA (R²=H, R³=H), MBOA (R²=OMe, R³=H), or M₂BOA (R²=OMe, R³=OMe).

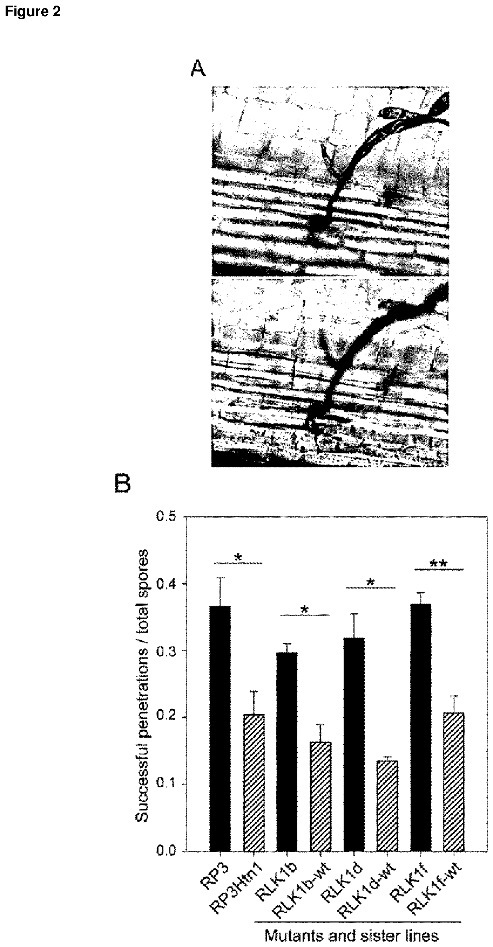

Figure 2 (Fig. 2 A and B) shows the rate of successful penetration events for fungal penetrations as described in Example 9. (Fig. 2 A) Hyphae detected inside of host tissues. In the upper and lower panels the focus is on the epidermis and hyphae, respectively. The arrows in the lower image of Fig. A indicate the hyphae inside the host tissues. (Fig. 2 B) RLK1b, RLK1d and RLK1f are *ZmWAK-RLK1* mutants with compromised NCLB resistance that were produced in RP3Htn1, while RLK1b-wt, RLK1d-wt and RLK1f-wt are the corresponding sister lines, respectively. Statistics was conducted using Student's t test, based on three independent experiments. The asterisks represent a significant difference of ^{∗∗}*p*<0.01 or ^{∗}*p*<0.05. Error bars indicate ± standard error.

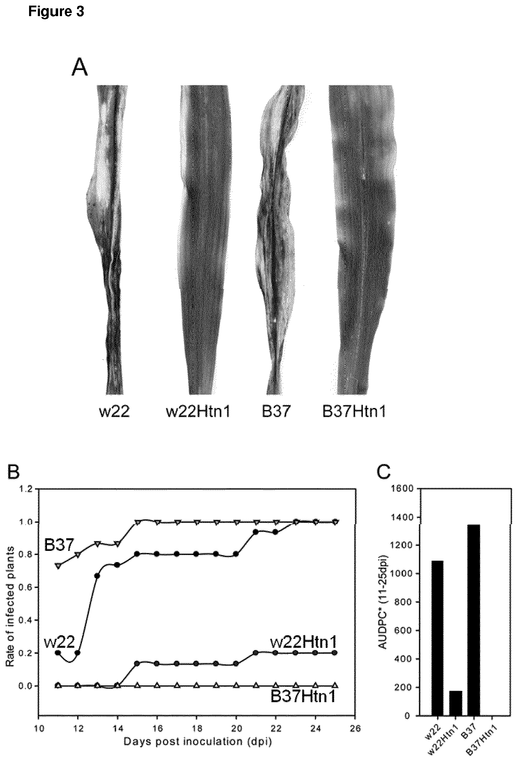

Figure 3 (Fig. 3 A to C) shows the disease phenotype of *Htn1*-NILs and the corresponding parents as detailed in Example 10 below. (Fig 3 A) The disease symptom of the second leaves at 16 dpi (from left to right for the lines w22, w22Htn1, B37 and B37Htn1). (Fig 3 B) Rate of infected of tested plants B37, w22, w22Htn1, and B37Htn1. (Fig 3 C) Area under the disease progress curve (AUDPC) for the isogenic lines detailed below the x-axis. *AUDPC in this panel was calculated as described in Humi et al. 2015, based on calculating sum of rate of infected plants (%).

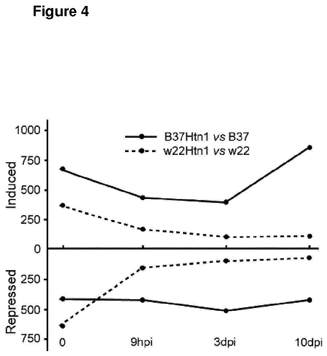

Figure 4 shows the result of a transcriptome analysis in *Htn1*-NILs, which revealed a set of DEGs. Fig. 4 shows the number of DEGs in the two *Htnl* NILs compared to the corresponding susceptible lines as further described in Example 10 below.

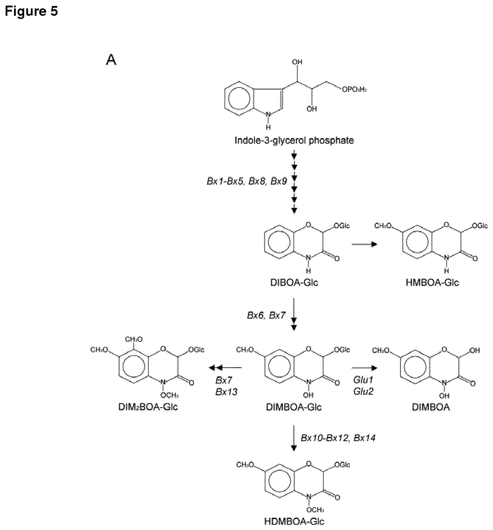

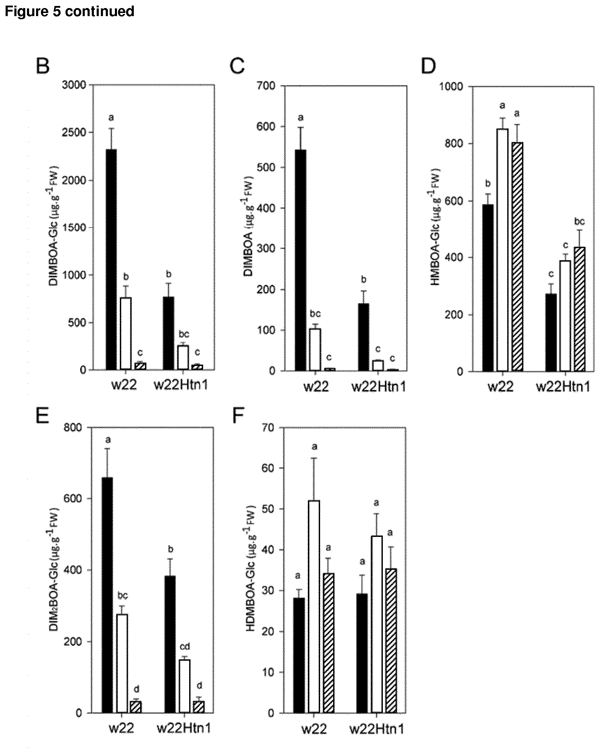

Figure 5 (Fig. 5 A to F) shows the content of BXDs in w22 and w22Htn1. (Fig. 5 A) shows the proposed bio-synthesis pathway of secondary metabolites BXDs. The genes encoding the proteins catalyzing each step of enzymatic reactions are presented besides and below the arrows. The contents of BXDs compounds DIMBOA-Glc (Fig. 5 B), DIMBOA (Fig. 5 C), HMBOA-Glc (Fig. 5 D), DIM₂BOA-Glc (Fig. 5 E) and HDMBOA-Glc (Fig. 5 F) were determined at before inoculation, at 3 dpi and 10 dpi. The statistics were conducted using Tukey's HSD (*P* = 0.05) in eight biological replicates. The ns stands for no significance. Error bars are ± SE. See also Example 10 below.

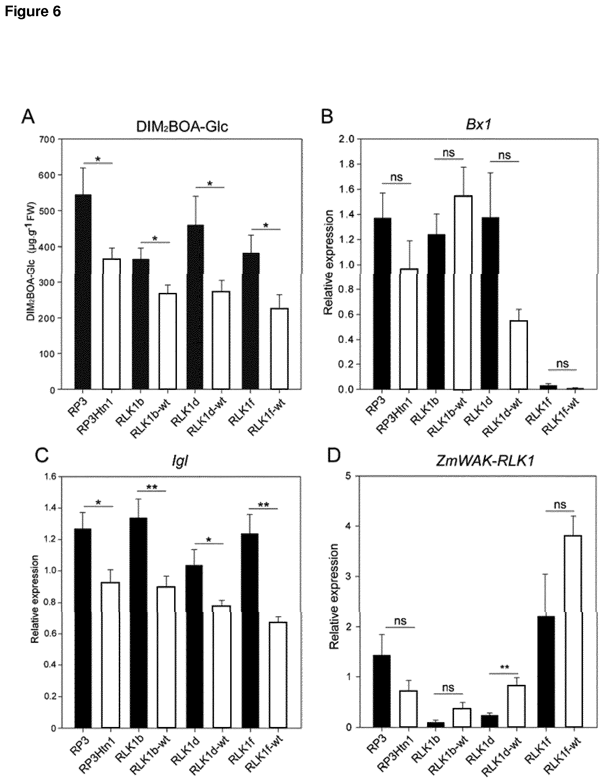

Figure 6 (Fig. 6 A to D) shows that the presence of ZmWAK-RLK1 results in decreased DIM₂BOA-Glc content possibly by down-regulating *Igl* expression in the maize RP3 genetic background (see also Example 11). (A) Content of DIM₂BOA-Glc (n=8). Expression analysis of *Bx1* (B), *Igl* (C) and *ZmWAK-RLK1* (D) in *Htnl* mutants and sister lines before infection at 21 days after sowing (n=5). Statistics was conducted using Student's t test. The asterisks represent a significant difference of ^{∗∗}*p*<0.01 or *^{∗}p*<0.05. Error bars are ± SE.

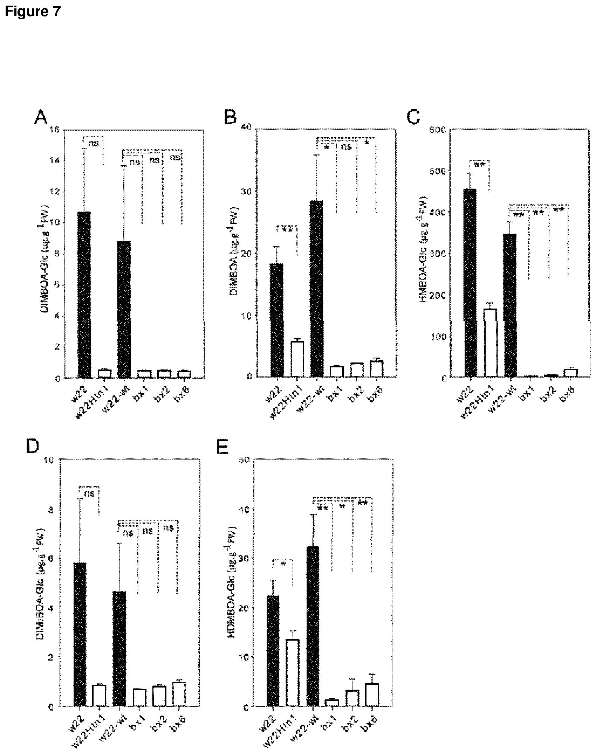

Figure 7 (Fig. 7 A to E) shows the content of BXDs compounds DIMBOA-Glc (A), DIMBOA (B), HMBOA-Glc (C), and DIM₂BOA-Glc (D), HDMBOA-Glc (E) in second leaves at 10 dpi. Further information is provided in Example 12 below. The statistics were conducted using Student's t test (P=0.05) in eight biological replicates. The asterisks represent a significant difference of ^{∗∗}*p*<0.01 or ^{∗}*p*<0.05. The ns stands for no significance. Error bars are ± SE.

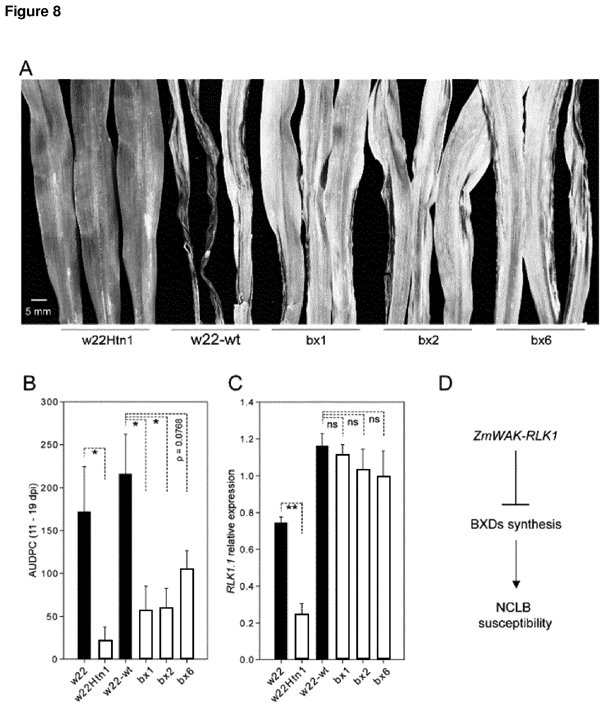

Figure 8 (Fig. 8 A to D) (see also Example 12) shows that compromising the biosynthesis of BXDs decreases the susceptibility of NCLB disease at the seedling stage. (Fig. 8 A and B): Visual symptoms and quantified NCLB disease severity in *bx* mutants. (Fig. 8 C): The transcriptional level of *ZmWAK-RLK1* at 10 dpi. (Fig. 8 D) shows the proposed model of *ZmWAK-RLK1* underlying NCLB disease resistance. The resistance allele suppressed the biosynthesis of major BXDs compounds, which likely served as the susceptibility component for promoting NCLB disease. Statistic test was conducted using Student's t test. The asterisks represent a significant difference of ^{∗∗}*p*<0.01 or p<0.05. The ns stands for no significance. Error bars are ± SE.

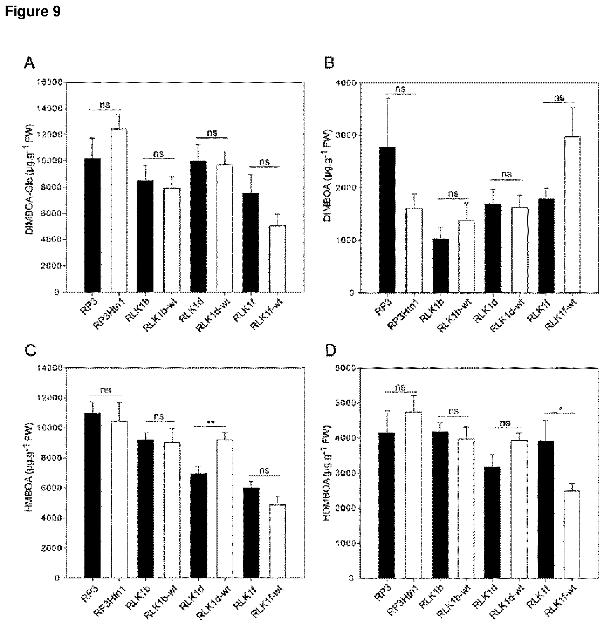

Figure 9 (Fig. 9 A to D) shows the content of BXDs compounds DIMBOA-Glc (A), DIMBOA (B), HMBOA-Glc (C), and HDMBOA-Glc (D) in second leaves of *Htnl* NILs and mutants at 21 days after sowing. Further information is provided in Example 12 below. The statistics were conducted using Student's t test (P=0.05) in eight biological replicates. The asterisks represent a significant difference of ^{∗∗}*p*<0.01 or ^{∗}*p*<0.05. The ns stands for no significance. Error bars are ± SE.

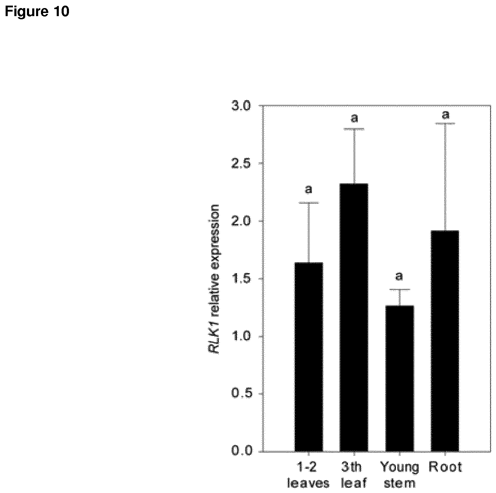

Figure 10 shows relative *RLK1* expression in tissues. These samples were harvested from 21 days old seedlings without pathogen inoculation. Different lower case letters in the graphs indicate a difference which is statistically different. See also Example 13 below.

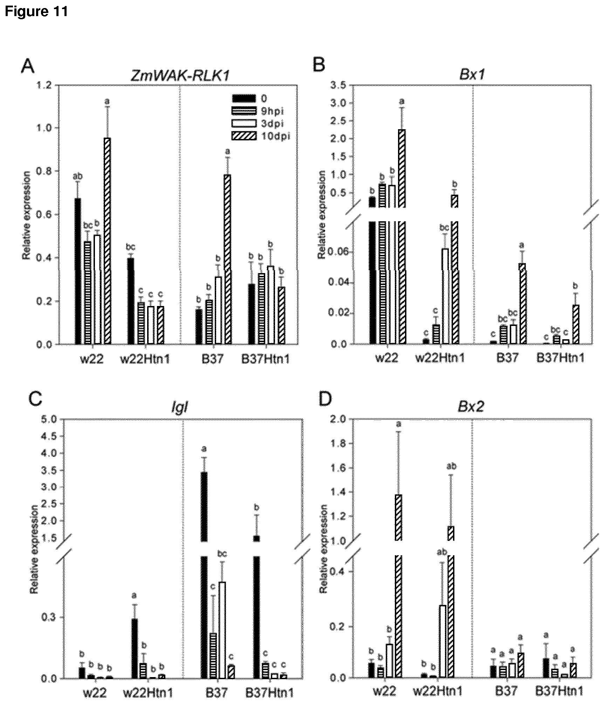

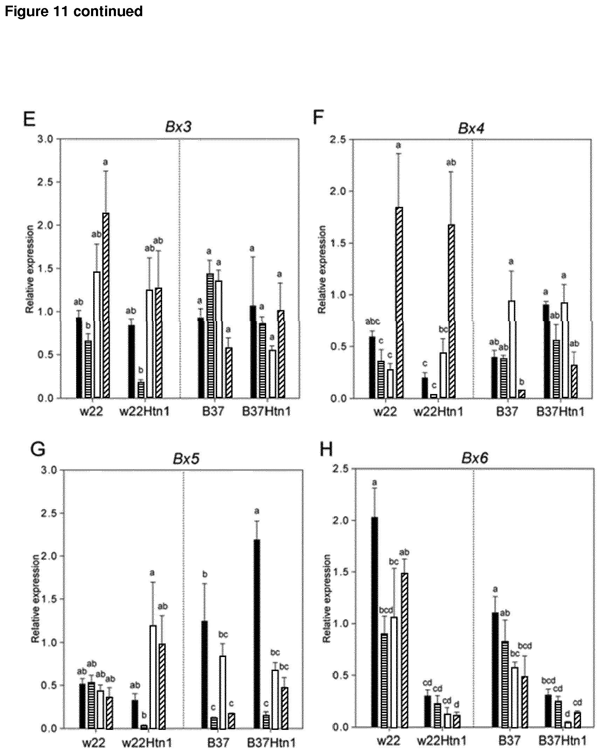

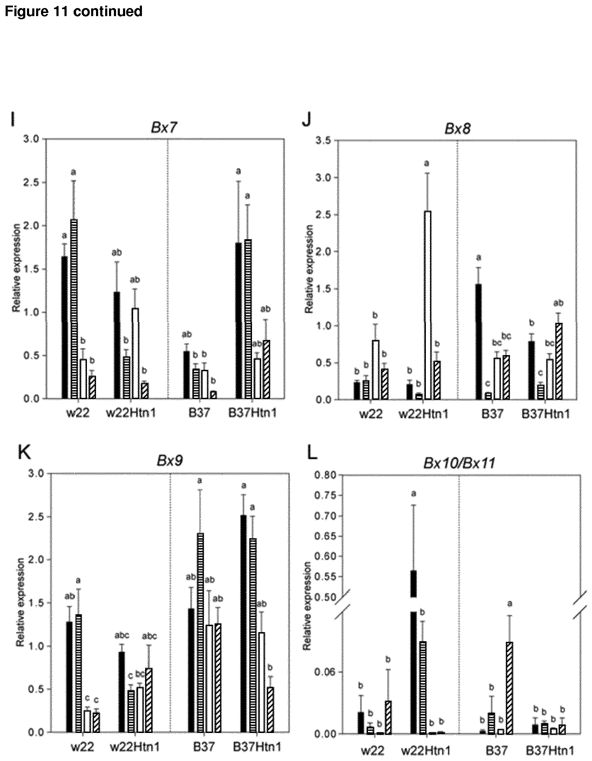

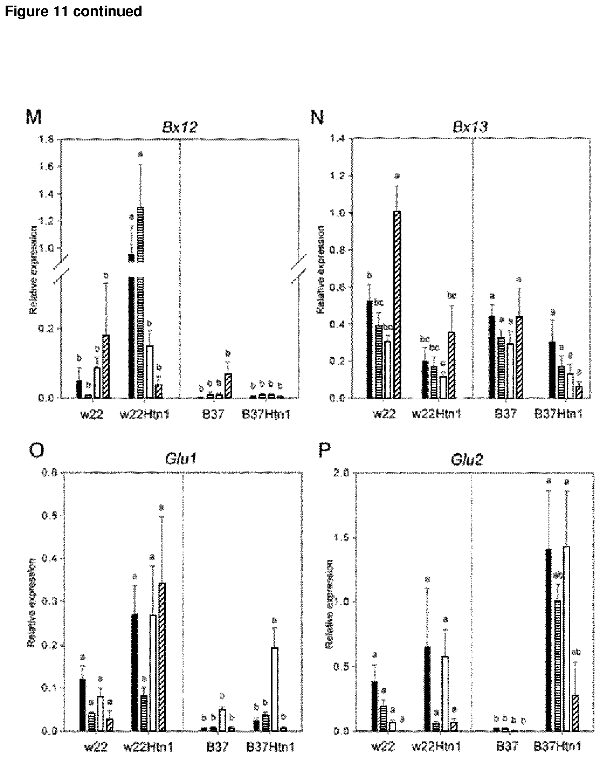

Figure 11 (Fig. 11 A and P) shows the transcription levels of genes in *Htn1*-NILs and the corresponding parental lines. The expression of genes (Fig. 11 A) *ZmWAK-RLK1*, (Fig. 11 B) *Bx1*, (Fig. 11 C) *Igl*, (Fig. 11 D) *Bx2*, (Fig. 11 E) *Bx3*, (Fig. 11 F) *Bx4*, (Fig. 11 G) *Bx5*, (Fig. 11 H) *Bx6*, (Fig. 11 I) *Bx7*, (Fig. 11 J) *Bx8*, (Fig. 11 K) *Bx9*, (Fig. 11 L) *Bx10*/*11*, (Fig. 11 M) *Bx12*, (Fig. 11 N) *Bx13,* (Fig. 11 O) *Glu1* and (Fig. 11 P) *Glu2* were quantified. The different colors and patterns of the bars indicate timepoints before and after infection as shown in the legend for Fig. 11 A which also applies for Fig. 11 Bto P. The statistics were conducted separately in w22 and B37 genetic background using Tukey's HSD (*P* = 0.05) in four biological replicates. Error bars are ± SE. Different lower case letters in the graphs indicate a difference which is statistically different. See also Example 10 below.

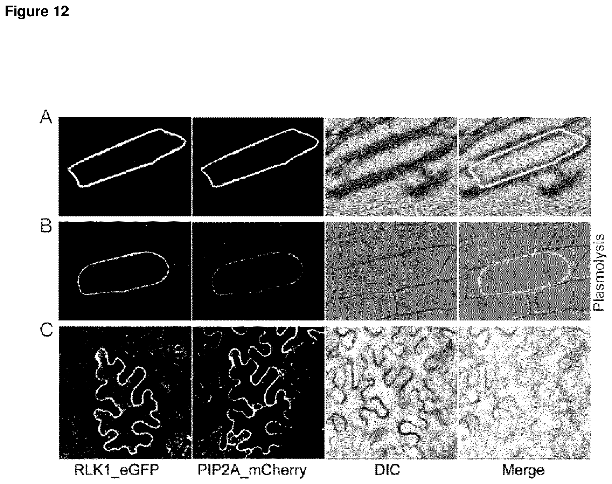

Figure 12 (Fig. 12 A to C) shows *ZmWAK-RLK1* localization to the plasma membrane. (Fig. 12 A-B) Fluorescent signals in onion epidermal cells after transient expression of ZmWAK-RKL1-eGFP (SEQ ID NO: 9) and the positive control PIP2A-mCherry that is known to localize to the plasma membrane (Kammerloher, Werner, et al. "Water channels in the plant plasma membrane cloned by immunoselection from a mammalian expression system." The Plant Journal 6.2 (1994): 187-199). Signals are shown before (Fig. 12 A) and after (Fig. 12 B) plasmolysis with 0.8 M mannitol. (Fig. 12 C) Fluorescent signals in N. benthamiana leaves two days after infiltration. Notably, the signals shown in white in the respective graphs correspond to the originally green (column 1 RLK_eGFP), red (column 2 PIP2A_mCherry), and yellow (merge, column 4) fluorescent signal. Column 3 (DIC) represents the differential interference contrast to visualize the cellular structures as control.

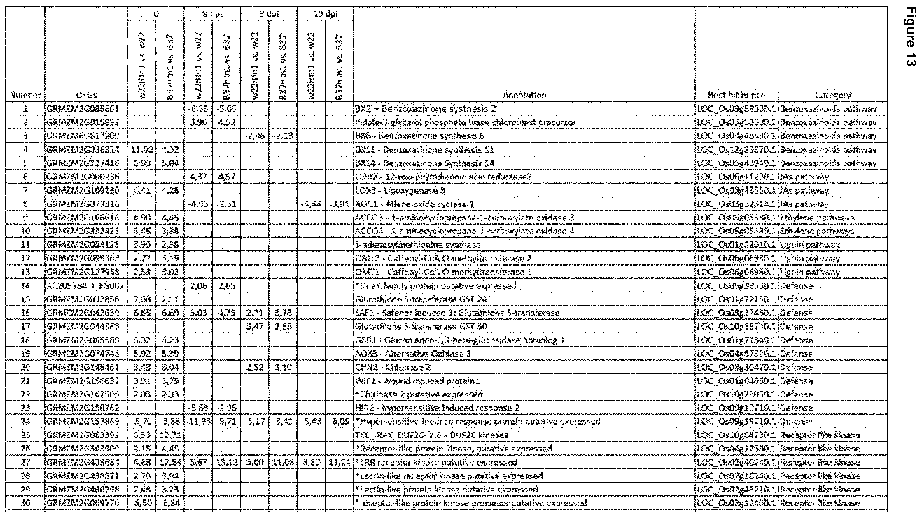

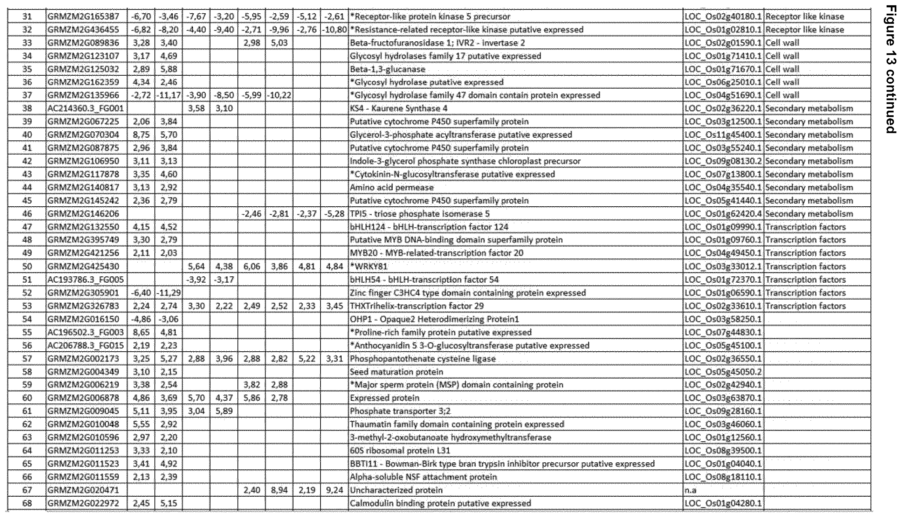

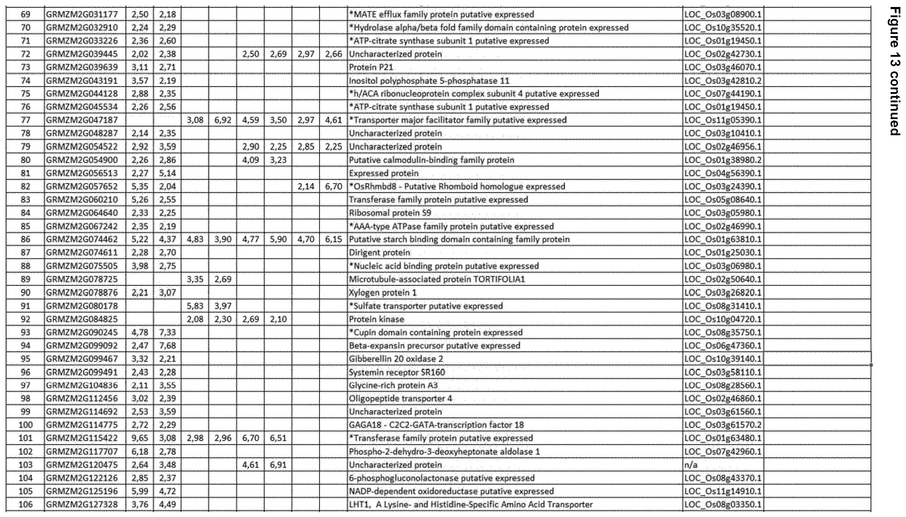

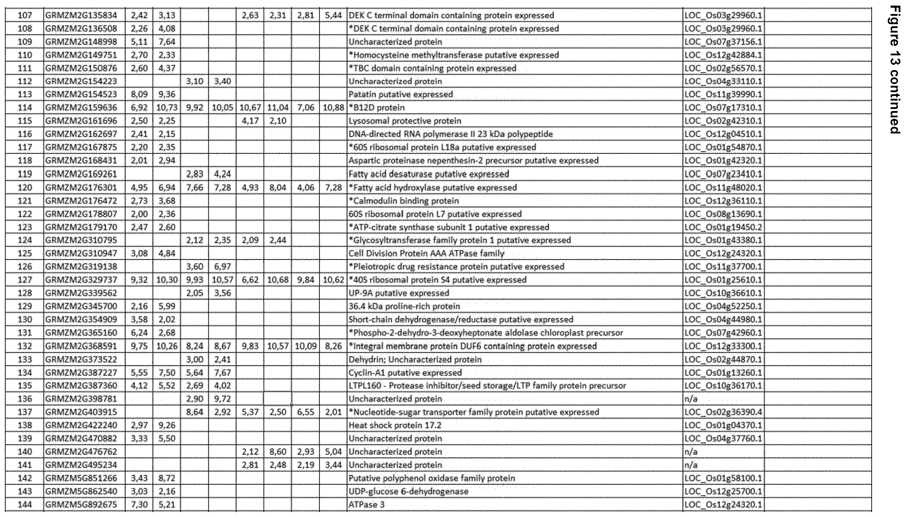

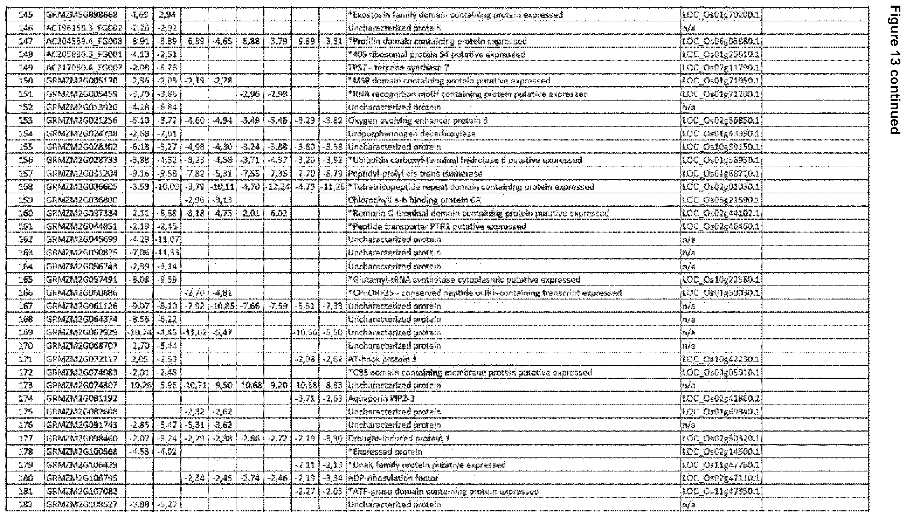

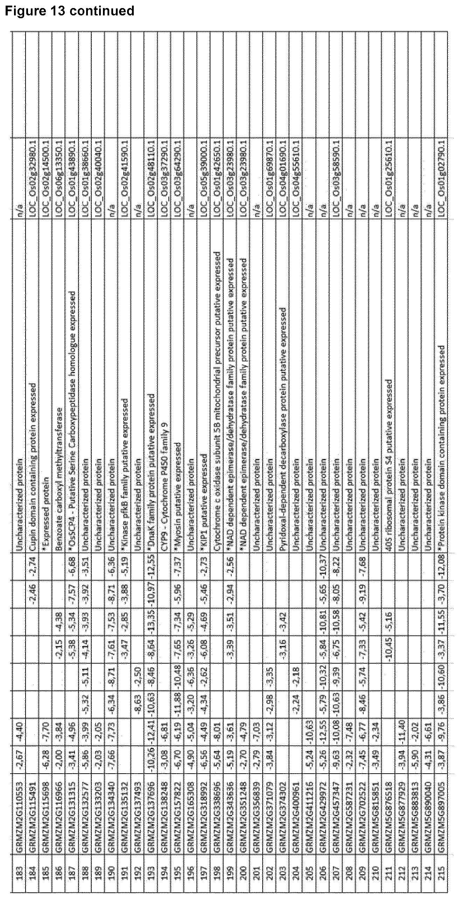

Figure 13 is a table showing the logFC and annotation of 215 the differentially expressed genes (DEGs) detected in B37Htn1/B37 and w22Htn1/w22 in at least one of time points.

### Detailed Description

Based on the experiments and data underlying the present invention, it was found that the genes involved in the biosynthesis of benzoxazinoids (BXDs) and derivatives thereof, mainly hydrolysis based derivatives like hydroxamic acids, are not only involved in the defense mechanism against E. *turcicum* but rather can also effect and mediate plant-defense, i.e., resistance mechanisms against various other fungal pathogens in a series of crop plants. The present invention thus implements both the link between a wall associated kinases (WAK), downstream signaling molecules, such as Bx1, Bx2, Bx6, Bx14 and Igl, or any other enzyme involved in the benzoxazinoid synthesis pathway, and the decrease of BXD secondary metabolites and further technically implements the finding that this decrease of BXD secondary metabolites is associated with increased fungal resistance.

The present invention thus provides in a first aspect a method for producing a *Zea mays* plant having increased *Helminthosporium turcucum* resistance, wherein the *Helminthosporium turcucum* resistance is regulated by at least one wall-associated kinase, the method comprising: (i) (a) providing at least one plant cell, tissue, organ, or whole plant having a specific genotype with at least one gene encoding a wall-associated kinase in the genome of said plant cell, tissue, organ, or whole plant; or (i) (b) introducing at least one gene encoding at least one wall-associated kinase into the genome of at least one cell of at least one of a plant cell, tissue, organ, or whole plant; and (ii) (a) mutating at least one gene encoding at least one wall-associated kinase in the at least one plant cell, tissue, organ, or whole plant via deletion, insertion or substitution of one or more nucleotides by at least one of a site-specific nuclease (SSN), oligonucleotide directed mutagenesis, chemical mutagenesis, or TILLING; and/or (ii) (b) increasing the expression level of at least one wall-associated kinase and/or reducing the expression level of at least one molecule within the signaling pathway from the at least one wall-associated kinase to the synthesis of at least one benzoxazinoid or within the synthesis pathway of at least one benzoxazinoid in the at least one plant cell, tissue, organ, or whole plant by at least one of a site-specific nuclease (SSN), oligonucleotide directed mutagenesis, chemical mutagenesis, or TILLING; and (iii) selecting a plant generated from the at least one plant cell, tissue, organ, or whole plant of (ii) (a) or (ii) (b) which has an increased *Helminthosporium turcucum* resistance, based on the determination of a reduced synthesis of a benzoxazinoid DIM₂BOA being in the glucoside or aglucone form, or a benzoxazolinone, in response to a *Helminthosporium turcucum* infection as compared to a corresponding control plant of the sanme genotype lacking the mutation of (ii) (a) or the modulation of (ii) (b), wherein the synthesis of the benzoxazinoid is regulated by the at least one wall-associated kinase; wherein the at least one wall-associated kinase is a WAK-RLK1 and a) is encoded by a nucleic acid molecule comprising the nucleotide sequence of SEQ ID NO: 1 or 7, b) is encoded by a nucleic acid molecule comprising the nucleotide sequence having at least 80% sequence identity to nucleotide sequence of SEQ ID NO: 1 or 7, preferably over the entire length of the sequence, c) is encoded by a nucleic acid molecule hybridizing with a complementary sequence to a) or b) under stringent conditions, wherein stringent hybridization conditions are hybridization in 4×SSC at 65°C and subsequent multiple washes in 0.1×SSC at 65°C for approximately 1 hour, d) is encoded by a nucleic acid molecule comprising the nucleotide sequence coding for an amino acid sequence of SEQ ID NO: 2 or 8, e) is encoded by a nucleic acid molecule comprising the nucleotide sequence coding for an amino acid sequence having at least 80% sequence identity to amino acid sequence of SEQ ID NO: 2 or 8, preferably over the entire length of the sequence, f) comprises the amino acid sequence of SEQ ID NO: 2 or 8, or g) comprises an amino acid sequence having at least 80% sequence identity to amino acid sequence of SEQ ID NO: 2 or 8, preferably over the entire length of the sequence, provided that any sequence of a) to g), optionally after expression, still encodes at least one functional Htn1 according to SEQ ID NO: 2, Ht2 or Ht3 according to SEQ ID NO: 8, or an allelic variant of the wall-associated kinase which is located at or mapped on a locus in bin 5 or bin 6 on the long arm of chromosome 8; wherein the at least one molecule within the signaling pathway from the at least one wall-associated kinase to the synthesis of at least one benzoxazinoid or within the synthesis pathway of at least one benzoxazinoid is selected from the group constiting of: I) the genes *bx1* (SEQ ID NO: 10), *bx2* (SEQ ID NO: 12), *igl* (SEQ ID NO: 14), *bx6* (SEQ ID NO: 16), *bx11* (SEQ ID NO: 18), *bx14* (SEQ ID NO: 20), *opr2* (SEQ ID NO: 22), *lox3* (SEQ ID NO: 24) *or aocl* (SEQ ID NO: 26), or II) a gene having identity of at least 80% to one of the genes of I), preferably over the full length of the gene, in particular the coding regions of the gene, or III) the proteins BX1 (SEQ ID NO: 11), BX2 (SEQ ID NO: 13), IGL (SEQ ID NO: 15), BX6 (SEQ ID NO: 17), BX11 (SEQ ID NO: 19), BX14 (SEQ ID NO: 21), OPR2 (SEQ ID NO: 23), LOX3 (SEQ ID NO: 25) or AOC1 gene (SEQ ID NO: 27), or IV) a protein having identity of at least 80% to one of the proteins of III), preferably over the full length of the protein, and wherein the benzoxazinoid whose synthesis is regulated by the at least one wall-associated kinase is DIM₂BOA, the aforementioned benzoxazinoid being in the glucoside or aglucone form, or a benzoxazolinone, or any combination with DIMBOA, HMBOA, HM₂BOA, HDMBOA, HDM₂BOA, HBOA, DHBOA, DIBOA or TRIBOA.

Wall associated kinases (WAKs) have recently been identified as major components of fungal and bacterial disease resistance in several cereal crop species. However, the molecular mechanisms of WAK-mediated resistance are presently largely unknown. According to this invention, the function of the maize gene *ZmWAK-RLK1* (*Htn1*) that confers quantitative resistance to northern corn leaf blight (NCLB) caused by the hemibiotrophic fungal pathogen *Exserohilum turcicum* was investigated. ZmWAK-RLK1 (Htn1) was found to localize to the plasma membrane and its presence resulted in a modification of the infection process by specifically reducing pathogen penetration into host tissues. Furthermore, the ubiquitous expression of ZmWAK-RLK1 and the findings on the signaling pathway downstream of ZmWAK-RLK1 demonstrate the function of this and associated wall-associated kinases as master regulators and crucial signaling mediators in plant defense against fungal disease.

A transcriptome analysis of near-isogenic lines (NILs) differing for *ZmWAK-RLK1* revealed that several genes involved in the biosynthesis of the secondary metabolites benzoxazinoids (BXDs) were differentially expressed in the presence of *ZmWAK-RLK1.* Particularly the content of BXD compounds DIMBOA-Glc, DIMBOA, HMBOA-Glc and DIM₂BOA-Glc were significantly lower in the NILs with *ZmWAK-RLK1.* Furthermore, DIM₂BOA-Glc, which is an inactive glucoside of BXDs, was significantly elevated in *ZmWAK-RLK1* mutants with compromised NCLB resistance. In addition, maize mutants that were affected in BXDs biosynthesis showed reduced susceptibility to E. *turcicum* infection at the seedling stage. We thus conclude that BXD biosynthesis increases susceptibility to E. *turcicum* infection and that the *ZmWAK-RLK1*-mediated NCLB resistance results from a reduction of these compounds. These findings indicate a novel link between WAKs underlying quantitative disease resistance and the defense mechanism mediated by the secondary metabolites BXDs that have been known for their involvement in cereal insect resistance. The term "WAK" as used herein may comprise a plant receptor-like kinase associated with the signal transduction directly or indirectly effecting the biosynthesis of genes involved in the BXD synthesis, or interacting with signaling mechanism and/or protein-protein interactions being involved in the BXD synthesis.

The plant immune response "caused" by a WAK can thus be of direct or indirect nature. As it is known to the skilled person, receptor-like kinases usually comprise at least one extracellular signaling domain, e.g., for sensing PAMPs and/or DAMPs, a transmembrane domain, and an intracellular kinase domain. The kinase domain allows the WAK to transform the extracellular signal into an intracellular response transferred via a cascade of proteins involved in the downstream signal transduction. Usually, receptor kinases thus indirectly initiate the activation and transfer into the nucleus/organelle of a transcription factor which regulates the transcription of a target gene. Furthermore, plant WAKs can trigger defense responses such as reactive oxygen species (ROS) accumulation through the activation of a NADPH oxidase, nitric oxide production, callose deposition, besides a MAP kinase-mediated activation of defense gene expression. The terms "causing" or "caused" as used herein in the context of a WAK or another plant receptor kinase is thus to be construed broadly to comprise any direct or indirect effect the activity of the WAK can have on downstream signaling molecules, wherein the molecules can be selected from at least one amino acid sequence, preferably an enzyme in the signal transduction cascade downstream of the WAK or a peptide being able to stimulate or inhibit complex formation downstream of the WAK or signal transduction downstream of a WAK, a metabolite, such as any secondary metabolite produced by a plant, a ROS, or an indirect effect on the regulation of the transcription and/or translation of another downstream gene/protein. A physical interaction of the WAK in the form of a signaling complex may occur to cause an action. In another embodiment, the action caused by the WAK is mediated by a downstream molecule, e.g., a downstream kinase phosphorylating another molecule, in an indirect way. In the terminal part of the WAK signaling cascade, a transcription activator or repressor can be induced to regulate the transcription of a target gene of a WAK, preferably a target gene in the jasmonic acid and/or BXD biosynthesis pathway. Besides a protein-DNA interaction, WAK signaling can also imply protein-protein interactions influencing the BXD biosynthesis pathway.

In another aspect, the methods of the present invention further may comprise the step of introducing, modifying and/or modulating at least one further or other gene into at least one plant cell, tissue, organ, or whole plant to provide a synergistic effect in increasing fungal disease by decreasing the synthesis of at least one BXD compound associated with fungal resistance. As disclosed herein, certain genes involved in the jasmonic acid pathway, the ethylene pathway, the lignin synthesis pathway, a plant defense pathway, a further receptor-like kinase pathway, or a cell wall pathway, and preferably certain genes involved in the jasmonic acid pathway, contribute to the signaling pathway of at least one functional WAK, wherein there may be a synergistic effect provided by the presence of a specific functional WAK and a specific non-functional or less functional gene of the jasmonic acid pathway, as the presence of both will contribute to an even significantly reduced amount of a BXD compound of interest and thus a more than additive increase in fungal resistance.

The present invention thus provides specific target genes which can be modulated in addition or alternatively to the at least one WAK of interest to provide a significantly improved fungal defense strategy for a plant of interest. These results are based on different functional studies including comparative transcriptome analysis in defined specific WAK genotypes, namely in two pairs of near isogenic lines, w22 and W22Htn1 as well as B37 and B37Htn1 (see Example 10 below), after fungal specific stimuli by analyzing the RNA sequencing datasets. Furthermore, additional RT-qPCR experiments and systematic RNA sequencing were conducted to decipher the plant immune network as triggered by a WAK, e.g., Htn1, (Examples 6 and 10 and Tables 1 and 2). These data demonstrated a cross-talk between the WAK and the benzoxazinoid synthesis and jasmonic acid pathway and thus provided new candidates to provide new elite plant lines comprising both a specific WAK as well as a specific genotype with respect to enzymes involved in the benzoxazinoid synthesis and jasmonic acid pathway.

In one embodiment according to the present invention, the method for producing a plant having increased *Helminthosporium turcucum* resistance may comprise the modification of at least one gene encoding at least one wall-associated kinase, and optionally at least one further or other gene, for example a *bx1*, *bx2*, *igl*, *bx6*, *bx11*, *bx14*, *opr2*, *lox3 or aoc1* gene, in the at least one plant cell, tissue, organ, or whole plant. The modification can be conducted by any means of techniques of molecular biology.

The present application further discloses the modulation or modulating of at least one wall-associated kinase, and/or of at least one further or other gene, for example a *bx1*, *bx2*, *igl*, *bx6*, *bx11*, *bx14*, *opr2*, *lox3 or aocl* gene, can thus comprise at least one of modulating the expression level of at least one wall-associated kinase, preferably increasing the expression at least one wall-associated kinase, and/or modulating the function or activity of and/or activity of at least one wall-associated kinase, for example, by providing at least one molecule interacting with the extracellular signalling domain of at least one WAK, e.g., an activator, or by providing at least one molecule interacting with the intracellular signalling domain of at least one WAK, such as a molecule inducing or inhibiting kinase activity. Additionally, the modulation or modulating of at least one wall-associated kinase, and/or of at least one further or other gene, for example a *bx1, bx2, igl, bx6, bx11, bx14, opr2, lox3 or aocl* gene, can comprise the targeted introduction of at least one mutation into a WAK and/or a further or other gene of interest to modulate the activity of the WAK and the further or other protein encoded by the at least one further or other gene in a targeted way. The modulation of at least one wall-associated kinase thus aim at influencing the activity of the at least one WAK within without modifying the nucleic acid sequence and thus possibly the amino acid sequence of a WAK of interest. Further it is described that wherein at least one further or other gene, for example a *bx1*, *bx2*, *igl*, *bx6*, *bx11*, *bx14*, *opr2*, *lox3 or aocl* gene, is modulated, the modulation may aim at reducing the activity of a at least one allele of a *bx1*, *bx2*, *igl*, *bx6*, *bx11*, *bx14*, *opr2*, *lox3 or aocl* gene to decrease the amount of BXD compound synthesized. For example, BX1 and IGL enzymes are accountable for the bulk of BX biosynthesis. Therefore, inhibiting the presence of a functional BX enzyme, preferably a BX1, BX2 or BX6 enzyme, or an Igl enzyme can contribute to the provision of a reduced BXD synthesis and thus an increased fungal resistance in a plant of interest.

The modulation according to the present application can comprise any direct or indirect interaction between two molecules, i.e., a receptor-ligand interaction, a transcription factor- transcription factor binding site interaction, an interaction of an enzyme, e.g., a kinase with its target site, an interaction of a peptide or nucleic acid modulator with a target site, an antibody-antigen interaction, an interaction with a DNA or histone binding protein and its cognate ligand (DNA or histone), a hybridization between two nucleic acid sequences/molecules and the like.

In one embodiment, the transcription level of at least one WAK within at least one cell of at least one of a plant cell, tissue, organ, or whole plant can be modified or modulated by specifically influencing a regulatory sequence of a WAK gene. In another embodiment, the modulation affects at least one gene, for example a *bx1*, *bx2*, *igl*, *bx6*, *bx11*, *bx14*, *opr2*, *lox3 or aocl* gene. This modulation or modification can comprise the introduction of at least one specific mutation, for example to activate a promoter of interest, or the modulation or modification can be in trans by providing a transcription factor modulating the transcription of at least one WAK gene, wherein the at least one WAK gene according to all embodiments of the present invention may comprise an endogenously occurring WAK gene, or a WAK gene introduced into at least one cell of at least one of a plant cell, tissue, organ, or whole plant.

According to the various aspects and embodiments of the present invention, a signalling pathway from the at least one wall-associated kinase to the synthesis of at least one benzoxazinoid in at least one plant cell, tissue, organ, or whole plant thus implies the whole chain of molecular actions downstream of a WAK as sensing molecule triggering a signalling cascade involving various different effectors until the synthesis of a BXD compound.

In one embodiment according to the various aspects of the present invention, the at least one wall-associated kinase is WAK-RLK1, preferably selected from Htn1, Ht2, or Ht3, or an allelic variant thereof, or a gene encoding the same, preferably wherein the at least one wall-associated kinase a) is encoded by a nucleic acid molecule comprising the nucleotide sequence of SEQ ID NO: 1 or 7, b) is encoded by a nucleic acid molecule comprising the nucleotide sequence having at least 81%, 82%, 83%, 84%, 85%, 86%, 87%, 88%, 89%, 90%, 91%, 92%, 93%, 94%, 95%, 96%, 97%, 98% or 99% sequence identity to nucleotide sequence of SEQ ID NO: 1 or 7, preferably over the entire length of the sequence, c) is encoded a nucleic acid molecule hybridizing with a complementary sequence to a) or b) under stringent conditions, wherein stringent hybridization conditions are hybridization in 4xSSC at 65°C and subsequent multiple washes in 0.1×SSC at 65°C for approximately 1 hour, d) is encoded by a nucleic acid molecule comprising the nucleotide sequence coding for an amino acid sequence of SEQ ID NO: 2 or 8, e) is encoded by a nucleic acid molecule comprising the nucleotide sequence coding for an amino acid sequence having at least 81%, 82%, 83%, 84%, 85%, 86%, 87%, 88%, 89%, 90%, 91%, 92%, 93%, 94%, 95%, 96%, 97%, 98% or 99% sequence identity to amino acid sequence of SEQ ID NO: 2 or 8, preferably over the entire length of the sequence, f) comprising the amino acid sequence of SEQ ID NO: 2 or 8, or g) comprising an amino acid sequence having at least 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98% or 99% sequence identity to amino acid sequence of SEQ ID NO: 2 or 8, preferably over the entire length of the sequence" provided that any sequence of a) to g), optionally after expression, still encodes at least one functional Htn1, Ht2, or Ht3, or an allelic variant, a mutant, or a functional fragment thereof. In a preferred embodiment, the at least one wall-associated kinase is selected from Htn1 (RLK1) or an allelic variant, a mutant or a functional fragment thereof, or a gene encoding the same. Variants may further comprise any functional splice variant of a WAK gene. As it is known to the skilled person, eukaryotic mRNA comprising introns is spliced during processing from a precursor mRNA into a mature mRNA giving rise to a protein after translation (protein biosynthesis).

"Functional" or "functional fragment" or "variant" as used in connection with a WAK or any other receptor-like kinase or any at least one further gene/protein, for example a *bx1*, *bx2*, *igl*, *bx6*, *bx11*, *bx14*, *opr2*, *lox3 or aocl* gene, or the corresponding proteins Bx1, Bx2, Igl, Bx6, Bx11, Bx14, Opr2, Lox3 or Aoc 1, according to the present disclosure means a fragment of an amino acid or nucleic acid sequence with reference to the respective (longer) sequence occurring in the natural environment of a plant genome of interest, whereas the functional fragment still comprises - optionally after transcription, processing and translation - at least one function of the respective parent sequence. The functional fragment may be less sterically demanding and thus more convenient for certain approaches. Furthermore, the functional fragment may be fused to another domain to create a fusion molecule for functional assays, e.g., a fusion with a gene encoding a protein having fluorescence activity. In another embodiment, the functional fragment may be fused to a tag and the like. Therefore, a functional fragment may also comprise a sequence comprising codon optimizations on the nucleic acid level, or comprising certain mutations, said mutations not influencing the activity or function of a WAK, or another receptor-like kinase of interest.

Preferably, any function variant at least comprises a truncated form of the extracellular signalling domain of a WAK and an active intracellular kinase domain, wherein the intracellular kinase domain is able to initiate downstream signalling. Notably, the extracellular domain, the transmembrane domain and/or the intracellular kinase domain of a WAK according to the present invention can comprise at least one mutation. Said mutation may lead to an increased signalling activity to represent a functional variation or functional mutation in the sense of the present invention.

In certain aspects according to the present invention, at least one further or other gene, for example a *bx1*, *bx2*, *igl*, *bx6*, *bx11*, *bx14*, *opr2*, *lox3 or aocl* gene, is introduced, and/or modified and/or modulated according to the methods of the present invention, variants or mutants representing "loss-of-function", or having reduced activity might be specifically preferred for the purpose of the present invention in case that the at least one variant or mutant results in a decreased BXD synthesis. Particularly, it was found according to the present invention that there is a cross-talk between the WAK signaling pathway and the BXD synthesis pathway, mainly the BXD synthesis pathway as mediated by Bx1, Bx2, Bx6, Bx11 and BX14 and/or Igl, wherein the targeted insertion, modulation or modification of at least one WAK, or the gene encoding the same, and a further effector, or the gene encoding the same, for example a *bx1*, *bx2*, *igl*, *bx6*, *bx11*, *bx14*, *opr2*, *lox3 or aocl* gene, contribute to a enhanced fungal resistance, in particular NCLB resistance, in a plant as the regulation of both pathways leads to a reduced BXD signature. Wherein the WAK pathway plays the role of the general "pacemaker" in this regulatory network which senses and forwards signals due to its recognition and kinase function, there is also a feedback regulation between the further effectors involved in the jasmonic acid and BXD synthesis pathway. The master regulator function of WAK is demonstrated by the fact that the combined expression of *Bx1* and *Igl* was consistently lower in genotypes with *ZmWAK-RLK1* (Fig. 11 B and C and Example 10) demonstrating that *ZmWAK-RLK1* and other WAKs have the capacity to induce a concerted action also regulating the benzoxazinoid pathway and the jasmonic acid pathway.

In one embodiment according to the various aspects of the present invention, the method for producing a plant having increased *Helminthosporium turcucum* resistance thus comprises the introduction and/or modification and/or modulation of at least one WAK, or a gene encoding the same, wherein the WAK at least comprises a functional intracellular kinase domain, for example a sequence selected from SEQ ID NOs: 1, 2, 7, or 8, or an allelic variant or mutant thereof, and wherein the method further comprises the introduction and/or modification and/or modulation of at least one BX or Igl protein, or a gene encoding the same, wherein the corresponding *bx* gene, or the *Igl* gene comprises at least one mutation, or wherein the *bx* gene, or the *Igl* gene is of a specific genotype is knocked-out, so that the WAK activity and the decreased or deleted BX protein or Igl protein activity results in a decreased BXD biosynthesis.

Receptor-like kinases can be further divided into RD and non-RD kinases, depending on the presence or absence of an arginine residue at the catalytic site of the kinase domain. ZmWAK-RLK2 contains an RD kinase and ZmWAK-RLK1 has a non-RD kinase domain (cf. for instance positions 505 and 506 of SEQ ID NO: 2, amino acids F (phenylalanine) and D (aspartic acid), respectively). Most receptor-like kinases involved in plant immunity identified so far belong to the non-RD kinases, whereas RD kinases are thought to play a role in other processes such as development. Variants of SEQ ID NO: 2 have been constructed (cf. SEQ ID NOs: 3 to 6 and Hurni et al., 2015). It was found that mutations at positions M455, G497 and G548 (with reference to SEQ ID NO: 2) may result in a higher susceptibility to NCLB. All said positions reside in the serine threonine kinase domain of ZmWAK-RLK1. A functional variant according to the present invention will thus avoid any mutation or combination of mutations in the kinase domain of a WAK which results in decreased fungal resistance. Exemplary mutants of SEQ ID NO: 2 are presented with SEQ ID NOs: 3 and 4 (RLK1b, M455I) and SEQ ID NOs: 5 and 6 (RLK1d, G497E). A further mutant analyzed herein, RLK1f, comprises a mutation G548R in comparison to the wild-type sequence according to SEQ ID NO: 2.

All mutants were tested in comparison to the respective sister lines as described herein. Based on these structural data the importance of a functional intracellular kinase domain of a WAK could be deduced. Therefore, a functional variant or a functional mutant of a WAK may comprise at least one mutation in comparison to the cognate wild-type sequence which at least one mutation does not disturb the downstream signaling of the WAK in that sense that a functional mutant or variant will decrease the level of a specific BXD compound to in turn increase fungal resistance of a plant, plant cell, tissue, or organ comprising such a functional variant of a WAK, or the sequence encoding the same.

According to certain embodiments of the present invention, more than one gene encoding a WAK, or a functional fragment thereof, or the sequence encoding the same, can be introduced into, or modulated or modified in at least one plant cell, tissue, organ, or whole plant. The introduction of several WAKs can have a synergistic effect in providing enhanced fungal resistance, particularly in case an elite line can be established based on the staggering of more than one WAK into the genome of a plant of interest according to the disclosure of the present invention. As described herein, WAKs represent the key signalling molecules initiating an immune cascade downstream of and mediated by the intracellular kinase domain of the WAKs. Therefore, more than one WAK may thus have a dosage effect positively downregulating BXD synthesis and thus increasing fungal resistance in a plant, in particular a crop plant, of interest. Furthermore, at least one further gene or protein, preferably being selected from any one of SEQ ID NOs: 10 to 27 or homologous genes or homologs thereof, can be additionally or alternatively modified as detailed above to provide a plant cell, tissue, organ or whole plant as material for producing a plant with improved fungal resistance properties, preferably resistance against NCLB. Further target sequences to be modified having an implication in the cross-talk between WAK signalling and BXD biosynthesis are disclosed in Tables 1 and 3 herein.

In a further embodiment according to the present invention, there is provided a method, wherein the reduced synthesis of at least one benzoxazinoid is achieved by providing at least one wall-associated kinase, an allelic variant, a mutant or a functional fragment thereof, or a gene encoding the same, wherein the at least one wall-associated kinase comprises a sequence which can directly or indirectly influence the benzoxazinoid pathway and at least one further plant metabolic pathway, preferably a disease resistance associated pathway, wherein the plant metabolic pathway is selected from the group consisting of the jasmonic acid pathway, the ethylene pathway, the lignin synthesis pathway, a defense pathway, a receptor-like kinase pathway, a cell wall associated pathway, preferably, wherein the at least one further plant metabolic pathway is the jasmonic acid pathway and wherein the the reduced synthesis of at least one benzoxazinoid is achieved by an decreased or down-regulated Igl and/or Bx1 expression as induced by at least one WAK of interest.

Several differentially expressed genes (DEGs) identified by the inventors of the present invention belonged to several different immune networks and to different disease resistance associated pathways including benzoxazinoids (BXDs) biosynthesis, (phytohormone) jasmonic acids (JAs), ethylene, lignin, defense and receptor-like kinases as well as cell wall were found in *Htnl* NILs (Example 10, Fig. 13). Surprisingly, six genes of the BXDs biosynthesis pathway showed differential expression in at least one timepoint, including *Bx1* (SEQ ID NOs: 10 and 11), *Bx2* (SEQ ID NOs: 12 and 13), *Igl-like* (SEQ ID NOs: 14 and 15), *Bx6* (SEQ ID NOs: 16 and 17), *Bx11* (SEQ ID NOs: 18 and 19) and *Bxl4* (SEQ ID NOs: 20 and 21) (see also Fig. 13, column 2 of table for further reference to publicly available data base entries for gene IDs and names) demonstrating a cross-regulatory network between the WAK and further pathways in fungal defense. This was particularly surprising because BXDs as secondary metabolites have so far not been associated with defense against fungi mediated by WAK kinases. More surprisingly, the transcriptome data and functional assays also, for the first time, revealed DEGs that are part of immune networks including the phytohormone jasmonic acids that plays a central role in regulating resistance against hemibiotrophic and necrotrophic diseases. JAs treatment can induce the accumulation of BXD compounds (Oikawa, Akira, Atsushi Ishihara, and Hajime Iwamura. "Induction of HDMBOA-Glc accumulation and DIMBOA-Glc 4-O-methyltransferase by jasmonic acid in poaceous plants." Phytochemistry 61.3 (2002): 331-337; Oikawa, Akira, et al. "Accumulation of HDMBOA-Glc is induced by biotic stresses prior to the release of MBOA in maize leaves." Phytochemistry 65.22 (2004): 2995-3001). The present invention thus provides evidence that there is an additional link between the WAK kinase signaling pathway and the jasmonic acid pathway which paves the way for a variety of new disease and particularly fungal resistance strategies as disclosed herein. The enzymatic properties of IGL are similar to BX1, but the transcriptional regulation of their corresponding genes is different. Like other *Bx* genes, *Bx1* is constitutively expressed during the early developmental stages of the plant, which correlates with endogenous BX levels. Plants carrying the mutant alleles of the *Bx1* gene produce only a fraction of the BXs that are found in *Bx1* wild-type plants. Therefore, a WAK according to the present invention may act as a master regulator bridging anti-fungal signalling with the effectors of the jasmonic acid pathway and other pathways, preferably an effector selected from the group consisting of SEQ ID NOs: 10 to 27 or homologous genes or homologs thereof.

In one embodiment, the introduction at least one additional gene encoding at least one wall-associated kinase into at least one cell of at least one of a plant cell, tissue, organ, or whole plant may comprise the introduction of a nucleic acid sequence, comprising DNA and/or RNA in a single stranded and/or double stranded form, or an amino acid sequence, by means of molecular biology to transfer a functional WAK of interest, or an additional functional WAK of interest, or the sequence encoding the same, into at least one cell of interest. Said at least one additional gene can be also any gene, wherein the resulting protein/enzyme is involved in the BXD biosynthesis pathway or in a jasmonic acid pathway, such as Bx1, Bx2, Bx3, Bx4, Bx5, Bx7, Bx8, Bx9, Bx10, Bx11, Bx12, Bx13, Bx14, Igl, Glu1, Glu2, OPR2, LOX3 or AOC1 (see Fig. 13 and SEQ ID NOs: 10 to 27), or any variant thereof, or a combination of the aforementioned genes/proteins. Preferably, the at least one additional gene comprises at least one mutation which changes the function of the naturally occurring respective additional gene, wherein the mutation, in the coding or within a regulatory region, causes decreased synthesis of the respective BXD compound, or wherein the mutation, in a regulatory region, such as a promoter region, or in a coding region, causes a reduced signal transduction from a WAK kinase located upstream in the signalling cascade so the said mutation results in a decreased synthesis of a BXD compound. In another embodiment, the gene encoding at least one of Bx1, Bx2, Bx3, Bx4, Bx5, Bx7, Bx8, Bx9, Bx10, Bx11, Bx12, Bx13, Bx14, Igl, Glul, Glu2, OPR2, LOX3 or AOC1 may be deleted or partially deleted within the genome of a plant cell of interest, or the gene may be modified in a targeted way.

Further enzymes involved in the regulation of the BXD synthesis which can be modulated, introduced or modified according to the methods of the present invention to achieve an increased fungal resistance in a plant cell, plant or plant material pathway are selected from the group of jasmonic synthesis pathway enzymes, including 12-oxo-phytodienoic acid reductase 2 (OPR2), Lipoxygenase 3 (LOX3) or Allene oxide cyclase 1 (AOC1), ethylene pathway enzymes, such as S-adenosylmethionine synthase, lignin pathway enzymes, such as, for example, Caffeoyl-CoA O-methyltransferase 1 (OMT1) or OMT2, enzymes and proteins involved in plant defense mechanisms, such as, for example SAF1 - Safener induced 1; Glutathione S-transferase, and any combination thereof.

Presently, WAKs are the only known proteins that can physically link the cell wall to the plasma membrane (Brutus, Alexandre, et al. "A domain swap approach reveals a role of the plant wall-associated kinase 1 (WAK1) as a receptor of oligogalacturonides." Proceedings of the National Academy of Sciences 107.20 (2010): 9452-9457). Therefore, further structurally and functionally related cell wall spanning or associated kinases are suitable as WAKs according to the present invention, e.g., maize *qHSR1* (Zuo, Weiliang, et al. "A maize wall-associated kinase confers quantitative resistance to head smut." Nature genetics 47.2 (2015): 151-157), or rice *OsWAK*/*Xa4* gene conferring quantitative rice blight resistance by strengthening the cell wall (Hu et al. 2017).

In another embodiment according to the present invention, the step of introducing at least one gene into at least one cell of at least one of a plant cell, tissue, organ, or whole plant may comprise the introduction of a gene, wherein the amino acid sequence or enzyme encoded by said gene is involved in the catalytic pathway downstream of a WAK kinase, wherein the additional gene is introduced alone, or together with at least one gene encoding a WAK kinase or a variant thereof.

"Benzoxazinoids" or "BXDs" are a class of indole-derived plant chemical defenses comprising compounds with a 2-hydroxy-2*H*-1,4-benzoxazin-3(4*H*)-one skeleton and their derivatives. BXDs have been described as phytochemicals in monocots, including grasses, including important cereal crops such as maize, wheat and rye, as well as a certain dicot species. The term "BXDs" as used herein refers to both benzoxazinones (glucosides and corresponding aglucones containing a 2-hydroxy-2H-1,4-benzoxazin-3(4*H*)-one skeleton) and their downstream derivative products during metabolic pathways, benzoxazolinones, as well as any intermediates. The term BXD may thus also comprise a derivative being the result of the activity of hydrolyzing glucosidases found in plastids, cytoplasm, and cell walls, or derivatives and intermediated being the result of degradation to benzoxazolinones via oxo-cyclo/ring-chain tautomerism. Further comprised are downstream metabolites directly being derivable from any benzoxazolinone. The term "BXDs" shall further comprise any open form, nitrenium form or complex, e.g., a metal complex from a BXD. BXD basic structures are represented in Fig. 1.

By the term "reduced/decreased synthesis of a benzoxazinoid" or "reduced synthesis of at least one benzoxazinoid" or "reducing the amount of at least one benzoxazinoid" or "reduction on BXDs content" or "reduced amount of a BXD compound" or the like, is meant that the plant cell, tissue, organ, or whole plant according to the present invention exhibit an amount of a benzoxazinoid, at least one benzoxazinoid or the benzoxazinoid of interest which is reduced by at least 10%, 15%, 20% or 25%, preferably by at least 30%, 35%, 40% or 45%, more preferably by at least 50%, 60% or 70% as compared to a corresponding control plant cell, control tissue, control organ, or control whole plant of the same genotype, but lacking the modification of the at least one gene encoding at least one wall-associated kinase or the modulation of the expression level of at least one wall-associated kinase and/or the transcription level, the expression level, or the function of at least one molecule within the signaling pathway from the at least one wall-associated kinase to the synthesis of at least one benzoxazinoid or within the synthesis pathway of at least one benzoxazinoid. In one embodiment according to the various aspects of the present invention, the benzoxazinoid whose synthesis is regulated by the at least one wall-associated kinase and optionally regulated by the at least one further enzyme of the jasmonic acid and/or benzoxazionoid pathway is selected from at least one of DIM₂BOA, DIMBOA, HMBOA, HM₂BOA, HDMBOA, HDM₂BOA, HBOA, DHBOA, DIBOA or TRIBOA, the aforementioned benzoxazinoid being in the glucoside or aglucone form, or a benzoxazolinone, or any combination of the aforementioned benzoxazinoids, preferably wherein the benzoxazinoid whose synthesis is regulated by the at least one wall-associated kinase is selected from at least one of DIM₂BOA, DIMBOA, HMBOA or HDMBOA, the aforementioned benzoxazinoid being in the glucoside or aglucone form, or any combination of the aforementioned benzoxazinoids.

In one embodiment according to the various aspects of the present invention, a reduced content of BXDs can be achieved by introducing at least one gene encoding at least one wall-associated kinase into at least one cell of at least one of a plant cell, tissue, organ, or whole plant, wherein the at least one wall-associated kinase causes a reduced synthesis of at least one BXD. More than one WAK encoding gene and different allelic variants of a WAK gene may be introduced into a cell of interest in addition to a WAK gene potentially already being present in the genome of a plant cell of interest. The presence of several WAKs or receptor-like kinases involved in the BXD synthesis may thus be favourable in order to increase the copy number and thus the dosage effect of a gene of interest.

In one embodiment, quantitative NCLB disease resistance is based on a decrease of the biosynthesis of at least one secondary metabolite BXDs, preferably DIM₂BOA-Glc, DIMBOA, HMBOA, DIMBOA-Glc or HMBOA-Glc, and the methods according to the various aspects of the present invention comprise the addition of a scavenger molecules interacting with and this neutralizing the activity of at least one secondary metabolite BXD to reduce the amount of the of at least one secondary metabolite BXD susceptibility component to decrease fungal infection at least one plant cell, tissue, organ, or whole plant.

According to the present invention, there are thus provided methods for producing a plant having increased *Helminthosporium turcucumresistance,* wherein the *Helminthosporium turcucumresistance* is regulated by at least one wall-associated kinase. "Regulated" in this context thus implies a direct or indirect regulation mediated by at least one wall-associated kinase. This regulation may imply a signalling cascade initiated by the at least one wall-associated kinase and proceeding through further molecules involved in the signalling cascade. The regulation can be on a protein, RNA or nucleic acid level. Furthermore, the regulation may imply a cross-talk or feedback regulation, for example implying a Bx1, Bx2, Bx3, Bx4, Bx5, Bx7, Bx8, Bx9, Bx10, Bx11, Bx12, Bx13, Bx14, Igl, Glul, Glu2, OPR2, LOX3 or AOC1 enzyme, or the gene encoding the same, or the transcriptional regulation of such a further gene encoding Bx1, Bx2, Bx3, Bx4, Bx5, Bx7, Bx8, Bx9, Bx10, Bx11, Bx12, Bx13, Bx14, Igl, Glu1, Glu2, OPR2, LOX3 or AOC1, or a modulation or modification of a gene encoding Bx1, Bx2, Bx3, Bx4, Bx5, Bx7, Bx8, Bx9, Bx10, Bx11, Bx12, Bx13, Bx14, Igl, Glul, Glu2, OPR2, LOX3 or AOC1.

In one embodiment, the pathogen according to the present disclosure is a fungal pathogen infesting a plant. The disease caused by a fungal pathogen and the respective fungus may be selected from Plume blotch *Septoria* (Stagonospora) *nodorum*, Leaf blotch (*Septoria tritici*), Ear fusarioses (Fusarium spp.), Late blight (*Phytophthora infestans*), Anthrocnose leaf blight or Anthracnose stalk rot (*Colletotrichum graminicola* (teleomorph: *Glomerella graminicola* Politis) *Glomerella tucumanensis*), Curvularia leaf spot (*Curvularia clavata*, *C. eragrostidis*, = *C. maculans* (teleomorph: *Cochliobolus eragrostidis*), *Curvularia inaequalis*, *C. intermedia* (teleomorph: *Cochliobolus intermedius*), *Curvularia lunata* (teleomorph: *Cochliobolus lunatus*), *Curvularia pallescens* (teleomorph: *Cochliobolus pallescens*), *Curvularia senegalensis*, *C. tuberculata* (teleomorph: *Cochliobolus tuberculatus*), Didymella leaf spot (*Didymella exitalis*), Diplodia leaf spot or streak (*Stenocarpella macrospora* = *Diplodialeaf macrospora*), Brown stripe downy mildew (*Sclerophthora rayssiae var. zeae*), Crazy top downy mildew (*Sclerophthora macrospora* = *Sclerospora macrospora*), Green ear downy mildew (*Sclerospora graminicola*), Leaf spots (various minor leaf spots) (*Alternaria alternata*, *Ascochyta maydis*, *A. tritici*, *A. zeicola*, *Bipolaris victoriae* = *Helminthosporium victoriae* (teleomorph: *Cochliobolus victoriae*), *C. sativus* (anamorph: *Bipolaris sorokiniana = H. sorokinianum = H. sativum), Epicoccum nigrum, Exserohilum prolatum = Drechslera prolata* (teleomorph: *Setosphaeria prolata*) *Graphium penicillioides*, *Leptosphaeria maydis*, *Leptothyrium zeae*, *Ophiosphaerella herpotricha*, (anamorph: *Scolecosporiella* sp.), *Paraphaeosphaeria michotii*, *Phoma* sp., *Septoria zeae*, *S. zeicola*, *S. zeina*, Northern corn leaf blight (*Setosphaeria turcica* (anamorph: *Exserohilum turcicum* = *Helminthosporium turcicum*), Northern corn leaf spot (*Cochliobolus carbonum* (anamorph: *Bipolaris zeicola* = *Helminthosporium carbonum*)), Phaeosphaeria leaf spot (*Phaeosphaeria maydis* = *Sphaerulina maydis*), Rostratum leaf spot (*Setosphaeria rostrata*, (anamorph: *Helminthosporium rostratum*)), Java downy mildew (*Peronosclerospora maydis* = *Sclerospora maydis*), Philippine downy mildew (*Peronosclerospora philippinensis* = *Sclerospora philippinensis*), Sorghum downy mildew (*Peronosclerospora sorghi* = *Sclerospora sorghi*), Spontaneum downy mildew (*Peronosclerospora spontanea* = *Sclerospora spontanea*), Sugarcane downy mildew (*Peronosclerospora sacchari* = *Sclerospora sacchari*), Sclerotium ear rot (southern blight) (*Sclerotium rolfsii* Sacc. (teleomorph: *Athelia rolfsii*)), Seed rot-seedling blight (*Bipolaris sorokiniana*, *B. zeicola* = *Helminthosporium carbonum*, *Diplodia maydis*, *Exserohilum pedicillatum, Exserohilum turcicum = Helminthosporium turcicum, Fusarium avenaceum*, *F. culmorum*, *F. moniliforme*, *Gibberella zeae* (anamorph: *F. graminearum*), *Macrophomina phaseolina*, *Penicillium* spp., *Phomopsis* sp., *Pythium* spp., *Rhizoctonia solani*, *R. zeae*, *Sclerotium rolfsii*, *Spicaria* sp.), Selenophoma leaf spot (*Selenophoma* sp.), Yellow leaf blight (*Ascochyta ischaemi*, *Phyllosticta maydis* (teleomorph: *Mycosphaerella zeae-maydis*), Zonate leaf spot (*Gloeocercospora sorghi*).

Further plant pathogenic fungi include Plasmodiophoromycota, such as *Plasmodiophora brassicae* (clubroot of crucifers), *Spongospora subterranea*, *Polymyxa graminis*, Oomycota, such as *Bremia lactucae* (downy mildew of lettuce), *Peronospora* (downy mildew) in snapdragon (*P. antirrhini*), onion (*P. destructor*), spinach (*P. effusa*), soybean (*P. manchurica*), tobacco ("blue mold"; P. *tabacina*) alfalfa and clover (*P. trifolium*), *Pseudoperonospora humuli* (downy mildew of hops), Plasmopara (downy mildew in grapevines) (*P. viticola*) and sunflower (*P. halstedii*), *Sclerophthora macrospora* (downy mildew in cereals and grasses), Pythium (for example damping-off of Beta beet caused by *P. debaryanum*), Phytophthora infestans (late blight in potato and in tomato and the like), Albugo spec., Ascomycota, such as *Microdochium nivale* (snow mold of rye and wheat), Fusarium, *Fusarium graminearum, Fusarium culmorum* (partial ear sterility mainly in wheat), *Fusarium oxysporum* (Fusarium wilt of tomato), *Blumeria graminis* (powdery mildew of barley (sp. hordei) and wheat (f.sp. tritici)), *Erysiphe pisi* (powdery mildew of pea), *Nectria galligena* (Nectria canker of fruit trees), *Uncinula necator* (powdery mildew of grapevine), *Pseudopeziza tracheiphila* (red fire disease of grapevine), *Claviceps purpurea* (for example, rye and grasses), *Gaeumannomyces graminis* (take-all on wheat, rye and other grasses), *Magnaporthe grisea, Pyrenophora graminea* (leaf stripe of barley), *Pyrenophora teres* (net blotch of barley), *Pyrenophora tritici-repentis* (leaf blight of wheat), *Venturia inaequalis* (apple scab), *Sclerotinia sclerotium* (stalk break, stem rot), *Pseudopeziza medicaginis* (leaf spot of alfalfa, white and red clover), Basidiomycetes, such as *Typhula incarnata* (typhula blight on barley, rye, wheat), *Ustilago maydis* (blister smut on maize), *Ustilago nuda* (loose smut on barley), *Ustilago tritici* (loose smut on wheat, spelt), *Ustilago avenae* (loose smut on oats), *Rhizoctonia solani* (rhizoctonia root rot of potato), *Sphacelotheca* spp. (head smut of sorghum), *Melampsora lini* (rust of flax), *Puccinia graminis* (stem rust of wheat, barley, rye, oats), *Puccinia recondita* (leaf rust on wheat), *Puccinia dispersa* (brown rust on rye), *Puccinia hordei* (leaf rust of barley), *Puccinia coronata* (crown rust of oats), *Puccinia striiformis* (yellow rust of wheat, barley, rye and a large number of grasses), *Uromyces appendiculatus* (brown rust of bean), *Sclerotium rolfsii* (root and stem rots of many plants), Deuteromycetes (Fungi imperfecti), such as *Septoria* (*Stagonospora*) *nodorum* (glume blotch) of wheat (*Septoria tritici*), *Pseudocercosporella herpotrichoides* (eyespot of wheat, barley, rye), *Rynchosporium secalis* (leaf spot on rye and barley), *Alternaria solani* (early blight of potato, tomato), *Phoma betae* (blackleg on Beta beet), *Cercospora beticola* (leaf spot on Beta beet), *Alternaria brassicae* (black spot on oilseed rape, cabbage and other crucifers), *Verticillium dahliae* (verticillium wilt), Colletotrichum, such as *Colletotrichum lindemuthianum* (bean anthracnose), *Phoma lingam* (blackleg of cabbage and oilseed rape), *Botrytis cinerea* (grey mold of grapevine, strawberry, tomato, hops and the like).

Preferred fungal diseases to be prevented and the corresponding causative pathogens which can be combated based on the disclosure of the present invention in a crop plant of interest are selected from a fungus from the order of Pleosporales, comprising *E. turcicum* / *H. turcicum* causing northern corn leaf blight (NCLB), particularly affecting maize and wheat plants, or comprising *Bipolaris maydis* causing southern corn leaf blight, the order of *Pucciniales* causing rust disease, comprising *Puccinia sorghi* causing common rust, or *Diploida macrospora* causing Diploida leaf streak/blight, or *Colletotrichum graminicola* causing Anthracnose, or *Fusarium* spp., preferably *Fusarium verticilioides* causing Fusarium stalk rot, or *Gibberella* spp., e.g., *Gibberella zeae* causing Giberella stalk rot, or *Sphacelotheca reiliana* causing maize head smut are thus plant diseases caused by pathogenic fungi which can be prevented in the plants and by the methods of the present invention.

In one embodiment according to the various aspects of the present invention the at least one gene encoding at least one wall-associated kinase may be stably integrated into the genome of the at least one plant cell, tissue, organ, or whole plant.

In another embodiment according to the various aspects of the present invention at least one further gene encoding at least one enzyme within the signalling cascade downstream of a wall-associated kinase may be stably integrated into the genome of the at least one plant cell, tissue, organ, or whole plant.

Methods for introducing a gene of interest into a plant cell of interest by means of molecular biology and associated tools and methodologies are disclosed herein and are known to the skilled person.

In one embodiment according to the various aspects of the present invention the at least one gene encoding at least one wall-associated kinase may be stably integrated into the genome of the at least one plant cell, tissue, organ, or whole plant.

Furthermore, the methods of the present invention can result in the creation or provision of a plant material, comprising grains or seeds, relating to any means known in the art to produce further plants, plant parts or seeds and includes *inter alia* vegetative reproduction methods, such as, for example, air or ground layering, division, (bud) grafting, micropropagation, stolons or runners, storage organs such as bulbs, corms, tubers and rhizomes, striking or cutting, or twin-scaling, and asexual reproduction, such as e.g. apomixis, somatic hybridization and the like.

In one embodiment according to the various aspects of the present invention, the modification of the at least one gene encoding at least one wall-associated kinase within step (ii) (a) or (ii) (b) of the method for producing a plant having increased fungal resistance, or a modification of a gene encoding Bx1, Bx2, Bx3, Bx4, Bx5, Bx7, Bx8, Bx9, Bx10, Bx11, Bx12, Bx13, Bx14, Igl, Glul, Glu2, OPR2, LOX3 or AOC1, may be performed by at least one of a site-specific nuclease (SSN) or a catalytically active fragment thereof, or a nucleic acid sequence encoding the same, oligonucleotide directed (ODM) mutagenesis (ODM), chemical mutagenesis, or TILLING.

TILLING, initially a functional genomics tool in model plants, has been extended to many plant species and become of paramount importance to reverse genetics in crops species. A major recent change to TILLING has been the application of next-generation sequencing (NGS) to the process, which permits multiplexing of gene targets and genomes. NGS will ultimately lead to TILLING becoming an *in silico* procedure. Because it is readily applicable to most plants, it remains a dominant non-transgenic method for obtaining mutations in known genes and thus represents a readily available method for non-transgenic approaches according to the methods of the present invention. As it is known to the skilled person, TILLING usually comprises the chemical mutagenesis, e.g., using ethyl methanesulfonate (EMS), or UV light induced modification of a genome of interest, together with a sensitive DNA screening-technique that identifies single base mutations in a target gene, wherein the target gene may encode a protein being selected from the group of a receptor-like kinase, such as a WAK, an enzyme involved in benzoxazinoid synthesis or metabolism, defense, the lignin pathway, the jasmonic acid synthesis pathway, or a transcription factor involved in one of the aforementioned metabolic and/or signalling pathways.

SSNs and ODM mutagenesis both are suitable techniques for precision genome engineering in plant cells. As it is known to the skilled person, ODM offers a rapid, precise and non-transgenic breeding alternative for trait improvement in agriculture to address this urgent need. ODM is a precision genome editing technology, wich uses oligonucleotides to make targeted edits in plasmid, episomal and chromosomal DNA of plant systems.

In one embodiment according to the various aspects and embodiments of the present invention, the at least one site-specific nuclease (SSN), or the nucleic acid sequence encoding the same, may be selected from at least one of a CRISPR nuclease, including Cas or Cpfl nucleases, a TALEN, a ZFN, a meganuclease, a base editor complex, a restriction endonuclease, including FokI or a variant thereof, or two site-specific nicking endonucleases, or a variant or a catalytically active fragment thereof. Said targeted genome engineering SSNs can be suitable for both, the introduction of a gene of interest not yet present in a specific genotype, as well as the targeted mutagenesis of a gene of a given specific genotype to modulate (up- or downregulate) the activity of an enzyme encoded by a gene of interest to be modified in a highly precise way.

SSNs meanwhile emerged as indispensable prerequisite for site-directed genome engineering. SSNs are (programmable) nucleases, which can be used to break a nucleic acid of interest at a defined position to induce either a double-strand break (DSB) or one or more single-strand breaks. Alternatively, said nucleases can be chimeric or mutated variants, no longer comprising a nuclease function, but rather operating as recognition molecules in combination with another enzyme. Those nucleases or variants thereof are thus key to any gene editing or genome engineering approach. In recent years, many suitable nucleases, especially tailored endonucleases have been developed comprising meganucleases, a base editor complex, zinc finger nucleases, TALE nucleases, , and CRISPR nucleases, comprising, for example, Cas, Cpfl, CasX or CasY nucleases as part of the Clustered Regularly Interspaced Short Palindromic Repeats (CRISPR) system. The use of those SSNs and the necessary accessory molecules, for example crRNAs, tracrRNAs, or gRNAs, and delivery systems are thus envisaged for performing the methods according to the present invention.

A "base editor" as used herein refers to a protein or a fragment thereof having the same catalytical activity as the protein it is derived from, which protein or fragment thereof, alone or when provided as molecular complex, referred to as base editing or base editor complex herein, has the capacity to mediate a targeted base modification, i.e., the conversion of a nucleotide base of interest resulting in a point mutation of interest which in turn can result in a targeted mutation, if the base conversion does not cause a silent mutation, but rather a conversion of an amino acid encoded by the codon comprising the position to be converted with the base editor. Preferably, the base editor is temporarily or permanently linked to at least one site-specific effector, or optionally to a component of at least one site-specific effector complex. The linkage can be covalent and/or non-covalent. Multiple publications have shown targeted base conversion, primarily cytidine (C) to thymine (T), using a CRISPR/Cas9 nickase or non-functional nuclease linked to a cytidine deaminase domain, Apolipoprotein B mRNA-editing catalytic polypeptide (APOBEC1), e.g., APOBEC derived from rat. The deamination of cytosine (C) is catalyzed by cytidine deaminases and results in uracil (U), which has the base-pairing properties of thymine (T). Most known cytidine deaminases operate on RNA, and the few examples that are known to accept DNA require single-stranded (ss) DNA.

A "CRISPR nuclease" according to the present invention can be a CRISPR-based nuclease, or the nucleic acid sequence encoding the same, which is selected from the group consisting of (a) Cas9, including SpCas9, SaCas9, SaKKH-Cas9, VQR-Cas9, St1Cas9, or (b) Cpfl, including AsCpfl, LbCpf1, FnCpf1, (c) CasX, or (d) CasY, or any variant or derivative of the aforementioned CRISPR-based nucleases, or a CRISPR-based nuclease comprising a mutation in comparison to the respective wild-type sequence so that the resulting CRISPR-based nuclease is converted to a single-strand specific DNA nickase, or to a DNA binding effector lacking all DNA cleavage ability. A "CRISPR(-based) nuclease", as used herein, is thus any nuclease which has been identified in a naturally occurring CRISPR system, which has subsequently been isolated from its natural context, and which preferably has been modified or combined into a recombinant construct of interest to be suitable as tool for targeted genome engineering. Any CRISPR-based nuclease can be used and optionally reprogrammed or additionally mutated to be suitable for the various embodiments according to the present invention as long as the original wild-type CRISPR-based nuclease provides for DNA recognition, i.e., binding properties. Said DNA recognition can be PAM dependent. CRISPR nucleases having optimized and engineered PAM recognition patterns can be used and created for a specific application. The expansion of the PAM recognition code can be suitable to target the site-specific effector complexes to a target site of interest, independent of the original PAM specificity of the wild-type CRISPR-based nuclease. Cpfl variants can comprise at least one of a S542R, K548V, N552R, or K607R mutation, preferably mutation S542R/K607R or S542R/K548V/N552R in AsCpfl from *Acidaminococcus* (cf. SEQ ID NO: 24). Furthermore, modified Cas variant, e.g., Cas9 variants, can be used according to the methods of the present invention as part of a base editing complex, e.g. BE3, VQR-BE3, EQR-BE3, VRER-BE3, SaBE3, SaKKH-BE3 (see Kim et al., Nat. Biotech., 2017, doi: 10.1038/nbt.3803). Therefore, according to the present invention, artificially modified CRISPR nucleases are envisaged, which might indeed not be any "nucleases" in the sense of double-strand cleaving enzymes, but which are nickases or nuclease-dead variants, which still have inherent DNA recognition and thus binding ability. Other suitable Cpfl-based effectors for use in the methods of the present invention are derived from *Lachnospiraceae bacterium* (LbCpf1, e.g., NCBI Reference Sequence: WP_051666128.1), or from *Francisella tularensis* (FnCpf1, e.g., UniProtKB/Swiss-Prot: A0Q7Q2.1). Variants ofCpf1 are known (cf. Gao et al., BioRxiv, http://dx.doi.org/10.1101/091611). Variants of AsCpfl with the mutations S542R/K607R and S542R/K548V/N552R that can cleave target sites with TYCV/CCCC and TATV PAMs, respectively, with enhanced activities *in vitro* and *in vivo* are thus envisaged as site-specific effectors according to the present invention. Genome-wide assessment of off-target activity indicated that these variants retain a high level of DNA targeting specificity, which can be further improved by introducing mutations in non-PAM-interacting domains. Together, these variants increase the targeting range of AsCpfl and thus provide a useful addition to the CRISPR/Cas genome engineering toolbox.

Due to the fact that receptor-like kinases and BX enzymes (cf. SEQ ID NOs: 10 to 13 and 16 to 21), Igl (SEQ ID NOs: 14 and 15), OPR2 (SEQ ID NOs:22 and 23), LOX3 (SEQ ID NOs:24 and 25), and AOC1 (SEQ ID NOs:26 and 27) are ubiquitously found in a variety of plants, particularly monocotyledonous plants (monocots) and dicotyledonous plants (dicots) of agronomic interest, the methods according to the present invention can be used for the targeted optimization of several important monoct and dicot crop plants.

In one aspect of the present application, there is thus disclosed a plant cell, tissue, organ, whole plant or plant material, or a derivative or a progeny thereof, obtainable by any one of the methods according to the various aspects disclosed herein. The plant cell, tissue, organ, whole plant or plant material, or a derivative or a progeny thereof obtained according to the present invention will have at least one optimized agronomic trait, wherein this trait is disease resistance or tolerance, preferably fungus resistance or tolerance, more preferably resistance or tolerance against NCLB caused by E. *turcicum* or a related fungal diseases caused by any one of the related fungal pathogens disclosed herein. Based on the disclosure provided herein demonstrating the functional mechanism of a WAK induced quantitative NCLB resistance, said resistance being associated with a reduction of the BXD biosynthesis, which in turn inhibits the hemibiotrophic fungus E. *turcicum* and related fungi, the teachings provided herein can be used to provide a plant cell, tissue, organ, whole plant or plant material, or a derivative or a progeny thereof having a favourable BXD content, preferably a reduced BXD content, so that the plant cell, tissue, organ, whole plant or plant material, or a derivative or a progeny thereof has an increased resistance against fungal infection, i.e., fungal infestation and persistence.

In yet a further embodiment according to the present invention, more than one agronomic property of a plant cell or plant of interest can be modified in addition to the introduction, modulation and/or modification of a WAK or WAK gene of interest. Said agronomic properties are selected from seed emergence, vegetative vitality, stress tolerance, disease resistance or tolerance against a further fungus, or against another pathogen, comprising a virus, bacterium, a nematode, an insect etc., herbicide resistance, branching tendency, flowering time, seed clusters, seed density, stability and storability, threshing capability (uniform ripening), lodging resistance, increased yield (seed size, yield etc.), or a modified composition of a molecule of agronomic importance (e.g. starch, carbohydrate, protein etc.) of interest, and the like.

In addition, the present application discloses a method for identifying at least one gene involved in increased pathogen resistance, preferably increased fungal resistance, in a plant cell, tissue, organ, whole plant, or plant material the method comprising: (i) determining the genotype of at least one plant cell, tissue, organ, whole plant, or plant material with respect to the presence of at least one gene encoding a wall-associated kinase in the genome of said plant cell, tissue, organ, whole plant or plant material; (ii) optionally: determining the benzoxazinoid signature of the at least one plant cell, tissue, organ, whole plant, or plant material of step (i); (iii) exposing the at least one plant cell, tissue, organ, whole plant, or plant material of step (i) or (ii) to a stimulus, optionally wherein the stimulus is correlated with the benzoxazinoid signature in the at least one plant cell, tissue, organ, whole plant, or plant material, preferably wherein the stimulus is associated with a fungal pathogen infection; (iv) performing an analysis of at least one analyte obtained from the at least one plant cell, tissue, organ, whole plant, or plant material of step (i) or (ii) after exposition to the stimulus; (v) determining at least one gene being regulated upon exposition to a stimulus according to step (iii) in at least one cell of the at least one plant cell, tissue, organ, whole plant, or plant material as derivable from the analysis of at least one analyte as defined in step (iv), (vi) subjecting the at least one gene as determined in step (v) to a functional characterization; and (vii) providing at least one gene involved in increased pathogen resistance, preferably increased fungal resistance, in a plant cell, tissue, organ, whole plant, or plant material.

In one embodiment, it is described that the determination of the genotype of at least one plant cell, tissue, organ, whole plant, or plant material with respect to the presence of at least one gene encoding a wall-associated kinase may be performed by determining in the genome of a plant cell, tissue, organ, whole plant, or plant material of interest the presence and/or transcript level of a WAK gene of interest, preferably a WAK gene comprising a nucleotide sequence according to SEQ ID NO: 1 or 7, or comprising a nucleotide sequence having at least 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98% or 99% sequence identity to one of the nucleotide sequence according to SEQ ID NO: 1 or 7, preferably over the entire length of the sequence, or comprising a nucleotide sequence hybridizing with a nucleotide sequence complementary to the nucleotide sequence according to SEQ ID NO: 1 or 7 preferably under stringent conditions, or comprising a nucleotide sequence encoding for an amino acid sequence of SED ID NO: 2 or 8 or for an amino acid sequence having at least 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98% or 99% sequence identity to one of SEQ ID NO: 2 or 8.

In another embodiment, determining the benzoxazinoid signature comprises a step of determination of the presence and/or the transcript level of at least one gene from the BXD biosynthesis pathway and/or the jasmonic acid pathway, The gene may be selected from any one of SEQ ID NOs: 10, 12, 14, 16, 18, 20, 22, 24 or 26, or a variant, homologous gene, allel or mutant or a fragment thereof. Bioinformatic tools for the determination and/or alignment of sequences of interest are disclosed herein, or are readily available to the skilled person.

In one embodiment, the determination of the genotype of at least one plant cell, tissue, organ, whole plant, or plant material with respect to the presence of at least one gene encoding a wall-associated kinase may also comprise the sequencing of a gene having a certain sequence identity, e.g., 80%, 81%, 82%, 83%, 84%, 85%, 86%, 87%, 88%, 89%, 90%, 91%, 92%, 93%, 94%, 95%, 96%, 97%, 98% or 99% sequence identity to one of the gene sequences disclosed herein, which gene has not yet been annotated in a publicly available genome database to determine the precise sequence of said gene by means of molecular biology, e.g., PCR techniques.

In one embodiment, it is disclosed that the method for identifying at least one gene involved in increased pathogen resistance, preferably increased fungal resistance, in a plant cell, tissue, organ, whole plant, or plant material may comprise the determination of the benzoxazinoid (BXD) signature of the at least one plant cell, tissue, organ, whole plant, or plant material of step (i). The benzoxazinoid signature means the qualitative and/or quantitative determination of at least one BXD secondary metabolite of interest as disclosed herein. This determination can provide a reference value for any subsequent analysis. The benzoxazinoid signature determination, which can be performed at different timepoints and with or without the addition of a stimulus, thus can provide information on the background level of a specific BXD present before and after addition of a stimulus. Furthermore, the BXD signature may provide data on the total amount of mixed BXD compounds synthesized in a plant, plant cell, tissue, organ or whole plant under suitable and defined conditions. The BXD signature may thus serve as reference value to have a benchmark for any subsequent modifications and/or modulations performed in accordance with the methods of the present invention. Due to the fact that BXD synthesis depends on the action of different enzymes in the terminal branch of the synthesis pathway, more than one different BXD compound may be analyzed to provide a BXD signature of a plant cell, tissue, organ, or whole plant of interest representing a full picture of the different BXD compounds synthesized by the plant under defined conditions (timepoint, stimulus, stimulus amount and environmental factors, for example, biotic or abiotic stress).

In one embodiment, the method for identifying at least one gene involved in increased pathogen resistance, preferably increased fungal resistance, in a plant cell, tissue, organ, whole plant, or plant material may comprise the exposition of the at least one plant cell, tissue, organ, whole plant, or plant material of step (i) or (ii) to a stimulus, optionally wherein the stimulus is correlated with the benzoxazinoid signature in the at least one plant cell, tissue, organ, whole plant, or plant material, preferably wherein the stimulus is associated with a fungal pathogen infection.

A "stimulus" in this context refers to any naturally occurring, endogenous or exogenous, or non-naturally occurring substance chemical substance stimulating a plant cell, tissue, organ, whole plant. Preferably, the stimulus is a stimulus derived from or associated with a pathogen, preferably a fungal pathogen. The stimulus may be a known PAMP or DAMP triggering an immune response mediated by a receptor-like kinase in a plant cell, tissue, organ, whole plant. The correlation may be of direct or indirect nature. The "stimulus" may also be an endogenous substance, e.g., a BXD or jasmonic acid, or a synthetic variant thereof, as BXD compounds and jasmonic acids may induce feedback regulation mechanisms in a plant cell. The "stimulus" may the pathogen by itself causing the desired response in a plant.

The stimulus may thus be any environmental stimulus which will cause a response in a plant, wherein the response is effected by a signal cascade, or reaction within a plant cell, tissue, organ, whole plant, e.g., resulting in a different transcriptome profile in comparison to the transcriptome profile of a non-stimulated plant. Preferably, the stimulus is correlated with a benzoxazinoid signature in at least one plant cell, tissue, organ, whole plant, or plant material. In one embodiment, where the correlation between a stimulus and the BXD signature is not known, a correlation between a stimulus of interest and the BXD signature can be easily determined by measuring the up- or down-regulation of genes within the BXD signalling pathway upon addition of a stimulus of interest to determine a direct or indirect correlation.

In a preferred embodiment, the stimulus is associated with a fungal pathogen, but is not restricted thereto. As it is known in the field of plant pathophysiology, plants evolved sophisticated strategies to respond to a stimulus as provided by a variety of different plant pathogens to initiate defense responses. Certain response may be highly specific for a pathogen, or one specific molecule associated or produced by said pathogen, whereas other defense strategies are part of a global regulatory network as associated by a stimulus of interest. According to the methods of the present invention it is thus possible to analyze the effect of a stimulus of interest a pathway of interest to identify any implication in the BXD or jasmonic acid biosynthesis pathway having a favourable effect on plant fungal response as disclosed herein in a highly targeted way to identify new target genes contributing to a favourable fungal defense response in a plant cell, tissue, organ, or whole plant of interest.

In one embodiment, the method for identifying at least one gene involved in increased pathogen resistance, preferably increased fungal resistance, in a plant cell, tissue, organ, whole plant, or plant material according to the present invention can comprise an additional step of electronically transmitting and/or electronically storing data on a computer readable medium.

An "analyte" obtained from the at least one plant cell, tissue, organ, or whole plant may comprises a nucleic acid, including DNA and RNA, an amino acid sequence, or a plant metabolite.

In one embodiment, a transcriptome analysis, i.e., an analysis of the sum total of all the messenger RNA molecules expressed from the genes of an organism, using RNA obtained from the at least one plant cell, tissue, organ, or whole plant of step (ii) after exposition to the stimulus is performed to obtain data on any changes in the transcription profile of certain genes in a plant cell, tissue, organ, whole plant treated with a stimulus of interest in comparison to plant cell, tissue, organ, or whole plant not treated with the respective stimulus. A variety of different tools to perform a transcriptome analysis of genome-wide differentially expressed RNA and to analyze altered gene expression/transcription is available to the skilled person. In one embodiment, the determination of at least one gene being regulated upon exposition to a stimulus according to step (iii) of the above method for identifying at least one gene involved in increased pathogen resistance in at least one cell of the at least one plant cell, tissue, organ, whole plant thus comprises the determination of the transcription level of a gene. Preferably, differentially regulated, or highly regulated genes, e.g., genes being significantly up- or down-regulated in comparison to a non-treated plant or plant cell, may be further analyzed.

In another embodiment, a proteome analysis, i.e., an analysis of the entire complement of proteins that is or can be expressed by a plant cell, tissue, or organism, using amino acids obtained from the at least one plant cell, tissue, organ, or whole plant of step (ii) after exposition to the stimulus is performed to obtain data on any changes in the transcription profile in a plant cell, tissue, organ, whole plant treated with a stimulus of interest in comparison to plant cell, tissue, organ, or whole plant not treated with the respective stimulus. Several methods for quantitative and qualitative proteome analysis, of the whole proteome or parts thereof, are available to the skilled person.

In yet another embodiment, an analysis of a metabolite, e.g., a substance produced by the at least one plant cell, tissue, organ or whole plant and representing an intermediate or product of its metabolism, is performed to identify the effect of a stimulus has on the overall constitution and production level with respect to said metabolite of interest.

In one embodiment of the method for identifying at least one gene involved in increased pathogen resistance, preferably increased fungal resistance, in a plant cell, tissue, organ, whole plant, or plant material, a gene of interest determined, said gene being regulated upon exposition to a stimulus, preferably a stimulus influencing a BXD signature, may be subjected to a functional characterization. The functional characterization may comprise an *in silico* analysis, an *in vitro* analysis, an *in vivo* analysis, or a combination of the aforementioned analyses. The *in silico* analysis may comprise the determination of any known function of said gene in different plant, or information on available allelic variants of said gene in different plants or different germplasm. Furthermore, the *in silico* analysis may comprise the determination of the locus of a gene such determined in the genome of a plant of interest, or the determination of regulatory sequences associated with the gene of interest. An *in vitro* analysis or manipulation may comprise the cloning, sequencing and characterization of the gene of interest and/or the creation of an expression construct, or vector, or a fusion construct, or the creation of mutants of a gene of interest. An *in vitro* analysis or manipulation may further comprise the introduction of a gene of interest, comprised by a suitable construct, into a target plant, tissue, organ or whole plant of interest by a suitable delivery vector. The *in vivo* analysis may comprise the analysis of different plants or plant cells, tissues or organs from different species, cultivars or varieties comprising or not comprising the gene of interest in their genome to provide a functional characterization of the phenotype the gene of interest may participate in, optionally by subjecting the different plants or plant cells, tissues or organs from different species, cultivars or varieties to different stimuli und controlled conditions to be able to compare the respective results.

In one embodiment, at least one gene involved in increased pathogen resistance as identified according to the methods as disclosed in the present application can be further subjected to directed mutagenesis studies and subsequent functional analyses to identify mutations positively or negatively effecting a phenotype of interest, wherein the phenotype is a change in the BXD signature, or a change of fungal resistance in comparison to the respective wild-type. Methods to introduce (multiple) site-directed mutations into a given gene of interest are available to the skilled person.

Further the present application discloses a plant cell, tissue, organ, whole plant or plant material, or a derivative or a progeny thereof, obtainable by introducing at least one gene as provided by the method for identifying at least one gene involved in increased pathogen resistance, preferably increased fungal resistance into the genome of at least one cell of at least one of a plant cell, tissue, organ, or whole plant.

In one embodiment of the disclosure, the plant cell, tissue, organ, whole plant or plant material, or a derivative or a progeny thereof, comprises at least one wall-associated kinase (WAK) selected from Htn1, Ht2, or Ht3, or an allelic variant, a mutant or a functional fragment thereof, or a gene encoding the same, preferably wherein the at least one wall-associated kinase a) is encoded by a nucleic acid molecule comprising the nucleotide sequence of SEQ ID NO: 1 or 7, or a functional fragment thereof, b) is encoded by a nucleic acid molecule comprising the nucleotide sequence having at least 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98% or 99% sequence identity to nucleotide sequence of SEQ ID NO: 1 or 7, preferably over the entire length of the sequence, c) is encoded a nucleic acid molecule hybridizing with a complementary sequence to a) or b) under stringent conditions, d) is encoded by a nucleic acid molecule comprising the nucleotide sequence coding for an amino acid sequence of SEQ ID NO: 2 or 8, or a functional fragment thereof, e) is encoded by a nucleic acid molecule comprising the nucleotide sequence coding for an amino acid sequence having at least 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98% or 99% sequence identity to amino acid sequence of SEQ ID NO: 2 or 8, preferably over the entire length of the sequence, f) comprising the amino acid sequence of SEQ ID NO: 2 or 8, or g) comprising an amino acid sequence having at least 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98% or 99% sequence identity to amino acid sequence of SEQ ID NO: 2 or 8, preferably over the entire length of the sequence, provided that the sequence, optionally after expression, still encodes at least one functional Htn1, Ht2, or Ht3, or an allelic variant, a mutant, or a functional fragment thereof. In a preferred embodiment, the plant cell, tissue, organ, whole plant or plant material, or a derivative or a progeny thereof, comprises at least one wall-associated kinase or an allelic variant, a mutant or a functional fragment thereof, or a gene encoding the same, which has been introduced, or which at least one wall-associated kinase or an allelic variant, a mutant or a functional fragment thereof, or a gene encoding the same, comprises at least one mutation enhancing the kinase activity of the at least one WAK.

In another preferred embodiment, the plant cell, tissue, organ, whole plant or plant material, or a derivative or a progeny thereof, comprises at least one further introduced enzyme, or the gene encoding the same, wherein the at least one further gene or enzyme is selected from a *bx1, bx2, igl, bx6, bx11, bx14, opr2, lox3 or aocl* gene (SEQ ID NOs: 10, 12, 14, 16, 18, 20, 22, 24 or 26, respectively), or a homologous gene thereof, or the respective proteins encoded by said genes (SEQ ID NOs: 11, 13, 15, 17, 19, 21, 23, 25 or 27, respectively), or a homolog thereof or an allelic variant or mutant thereof, preferably a mutant resulting in decreased transcription and/or translation of the *bx1, bx2, igl, bx6, bx11, bx14, opr2, lox3 or aocl* gene or protein, respectively. The at least one mutation may thus reside in a regulatory region of such a gene leading to a reduced transcription, or the mutation may result in at least one point mutation affecting the catalytic activity of the translated protein so that said protein or enzyme has a decreased capability to synthesize a BXD compound.

In one embodiment, the introduction of at least one gene into plant cell, tissue, organ, whole plant or plant material, obtainable by introducing at least one gene as provided by the method for identifying at least one gene involved in increased pathogen resistance, preferably increased fungal resistance, is a stable introduction, preferably a stable introduction mediated by means of molecular biology, comprising *Agrobacterium*-mediated transformation, genome editing, or a combination thereof.

In one embodiment, the introduction may be effected by any means of molecular biology. In one embodiment the introduction of a gene or allele determined can take place by recombination between two donor genomes, e.g., in a fused protoplast, wherein at least the donor protoplast carries the gene allele of interest in its genome. In any case, any progeny or derivatives comprising the gene allele of interest can then be subjected to repeated back-crossing steps with a plant line carrying a genetic background of interest to select for the gene allele of interest in the resulting derivatives or progeny.

In one embodiment of the disclosure, there is an improved donor source of germplasm having, e.g. by introgression, enhanced resistance to a fungus of interest, preferably wherein the fungus resistance against which resistance is increased, or the disease caused by said fungus is selected from a fungus of the order of *Pleosporales,* comprising *E. turcicum* / *H. turcicum* causing northern corn leaf blight (NCLB), particularly affecting maize and wheat plants, southern corn leaf blight *(Bipolaris maydis),* the order of *Pucciniales* causing rust disease, comprising common rust (*Puccinia sorghi*), or Diploida leaf streak/blight *(Diploida macrospora* / *Stenocarpella macrospora*), or *Colletotrichum graminicola*, or *Fusarium* spp., preferably *Fusarium verticilioides* causing Fusarium stalk rot, or *Gibberella* spp., e.g., *Gibberella zeae* causing Giberella stalk rot, rust, stalk rot, maize head smut (*Sphacelotheca reiliana*), and Diploida leaf streak/blight. This germplasm can then serve as basis for further breeding steps.

In another embodiment, the introduction of at least one gene as identified and provided by the method for identifying at least one gene involved in increased pathogen resistance into at least one plant cell, tissue, organ, whole plant may be effected by at least one means of molecular biology, comprising the use of a delivery vehicle or vector. Optionally, the method can further comprise the modification or modulation of a gene of interest using at least one of a site-specific nuclease (SSN) or a catalytically active fragment thereof, or a nucleic acid sequence encoding the same, oligonucleotide directed mutagenesis, chemical mutagenesis, or TILLING, wherein the at least one site-specific nuclease (SSN), or the nucleic acid sequence encoding the same, is selected from at least one of a CRISPR nuclease, including Cas or Cpf1 nucleases, a TALEN, a ZFN, a meganuclease, a base editor complex, a restriction endonuclease, including FokI or a variant thereof, or two site-specific nicking endonucleases, or a variant or a catalytically active fragment thereof.

Additionally, the present applications describes a method of increasing pathogen resistance, preferably fungal resistance, in a plant cell, tissue, organ, whole plant, or plant material, the method comprising: (i) providing at least one plant cell, tissue, organ, whole plant or plant material; (ii) (a) treating the at least one plant cell, tissue, organ, whole plant or plant material according to step (i) with a substance neutralizing the effect of at least one benzoxazinoid, and/or (ii) (b) treating the at least one plant cell, tissue, organ, whole plant or plant material according to step (i) with a substance activating the signalling pathway downstream of at least one wall-associated kinase; and/or (ii) (c) treating the at least one plant cell, tissue, organ, whole plant or plant material according to step (i) with a substance modulating or modifying the activity of at least promoter or at least one regulatory sequence of at least one gene of the at least one plant cell, tissue, organ, whole plant or plant material of step (i), wherein said at least promoter or at least one regulatory sequence is involved in the regulation of transcription of at least on gene involved in the signalling pathway of, or downstream of at least one wall-associated kinase or involved in the synthesis pathway of at least one benzoxazinoid; (ii) (d) treating the at least one plant cell, tissue, organ, whole plant or plant material according to step (i) with a substance inhibiting the synthesis of at least one benzoxazinoid; (iii) reducing the amount of at least one benzoxazinoid and thereby increasing pathogen resistance, preferably fungal resistance, in at least one plant cell, tissue, organ, whole plant, or plant material.

A "a substance neutralizing the effect of at least one benzoxazinoid " as used herein is to be construed broadly and comprises any naturally occurring or synthetic molecule, which can interact with a BXD compound to decrease the natural effect of the BXD compound said BXD compound would exert (endogenously and/or exogenously) on a plant or the plant environment. Preferably, the substance neutralizing the effect of at least one benzoxazinoid can be added to a plant cell, tissue, organ, or whole plant, optionally coated or together with a suitable delivery vehicle, so that the substance can be transferred into a plant cell of interest. Alternatively, the substance can be added to a plant cell, tissue, organ or whole plant to neutralize the effect of a volatile compound released by a plant cell, tissue, organ or whole plant. Preferably, the substance neutralizing the effect of at least one benzoxazinoid is not toxic to the plant cell, tissue, organ, or whole plant, or to the environment. As it is known that jasmonic acid (JA) treatment can induce the accumulation of BXD compounds (Oikawa et al, 2002 and 2004), such substance may also be a substance scavenging or reducing the amount of jasmonic acid to decrease the accumulation of a BXD compound, which in turn leads to the increased fungal resistance of a plant cell, tissue, organ or whole plant of interest. The substance may also interfere with the transcription of at least one *Bx, Igl* or a further gene involved in the BXD or jasmonic acid biosynthesis pathway.

In a further embodiment, alone or in combination with the use of a neutralizing substance, at least one plant cell, tissue, organ, whole plant or plant material can be treated with a substance activating the signalling pathway downstream of at least one wall-associated kinase. As used in the context of molecular biology, the terms "upstream" and "downstream" can refer to the temporal and mechanistic order of cellular and molecular events. For example, in signal transduction cascade, the second messenger or an intracellular kinase acts downstream to - that is to say, temporally *after* - activation of cell membrane receptors, for example a WAK. The other way around, activation of cell membrane receptors occurs upstream of - that is to say, prior to - the production of second messengers or the activation or inhibition of further enzymes acting later and intracellularly in the signalling cascade. Such an activating substance can be selected from a substance acting from the exterior of a plant or plant cell, for example, a substance being a PAMP or DAMP for a receptor-like kinase, e.g., a WAK, so that a stronger signal is received and the receptor mediated response is enhanced. Furthermore, the substance may activate the kinase function of a WAK, or any kinase downstream of the WAK. Finally, the substance may act at the interface between the WAK and a further BXD or jasmonic acid biosynthesis pathway.

For the wheat WAK gene *TaWAK*/*Snn1* it was shown that it is hijacked by the necrotrophic effector SnTox1 that triggers programmed cell death allowing a pathogen to feed and grown on the dead tissue (Shi et al. 2016). Furthermore, these data show that elicitors recognized by WAKs can both be cell wall derived degraded polysaccharides (e.g. OGs) or pathogenic short peptides (SnTox1) (Brutus et al. 2010; Shi et al. 2016). Thus, there is increasing evidence for a complex nature and functional divergence of WAKs in perception of types of ligands and in their role of interacting with biotic diseases in a direct as well as an indirect way. Preferably, an activating substance as described above is a substance directly activating a WAK of interest which in turn, directly or indirectly, leads to a decreased synthesis of at least one BXD compound, which in turn increases the fungal resistance of a plant cell, tissue, organ or whole plant.

The inventors of the present invention demonstrated that the WAK *ZmWAK-RLK1* functions upstream of the BXDs biosynthesis pathway and decreases the content of secondary metabolites BXDs compounds, e.g., DIM₂BOA-Glc. As the BXD class of secondary metabolites has been found in many of cereal species such maize, wheat and rice, which are the most important food crops worldwide, the methods according to the various aspects of the present invention can thus be used to effect the WAK signaling cascade intrinsically linked to the BXD synthesis, which in turn was found to be key to provide new strategies in fungal defense in plants, preferably for reducing susceptibility to northern corn leaf blight already at the seeding stage. For example, the storage glucoside DIM₂BOA-Glc was found to be constantly lower in susceptible *ZmWAK-RLK1* mutants, which suggested DIM₂BOA-Glc severed as a candidate susceptibility compound for promoting *E. turcicum* infection. Knock-out of this compound has been shown to slightly increase the performance of corn leaf aphids *Rhopalosiphum maidis* (Handrick, Vinzenz, et al. "Biosynthesis of 8-O-methylated benzoxazinoid defense compounds in maize." The Plant Cell 28.7 (2016): 1682-1700), a completely different class of plant pathogens, not infecting, yet feeding on a plant, whereas the functional mechanism of DIM₂BOA-Glc in interaction with phloem-feeding insects as presently known is possibly different and antagonistic. The methods and findings according to the present invention and mainly the new insights in gap bridge of WAKs and the secondary defense metabolite BXDs can also be used to provide new defense mechanisms against aphids and other phloem feeding insects to a plant, preferably a crop plant.

In one preferred embodiment, the methods comprise the modulation or modification of at least one further gene from a BXD and/or jasmonic acid biosynthesis pathway as disclosed herein to further decrease the content of secondary metabolites BXDs compounds, e.g., DIM₂BOA-Glc and thus to enhance fungal resistance in a plant.

In yet a further embodiment of the method of increasing pathogen resistance, preferably fungal resistance, in a plant cell, tissue, organ, whole plant, or plant material according to the present invention, the method comprises treating the at least one plant cell, tissue, organ, whole plant or plant material according to step (i) with a substance modulating the activity of at least promoter or at least one regulatory sequence of at least one gene of the at least one plant cell, tissue, organ, whole plant or plant material of step (i), wherein said at least promoter or at least one regulatory sequence is involved in the regulation of transcription of at least on gene involved in the signalling pathway of, or downstream of at least one wall-associated kinase, or involved in the synthesis pathway of at least one benzoxazinoid. By modulating or modifying the activity of a promoter or regulatory sequence, the transcription level of a gene of interest and in turn the expression level of a protein of interest can be influenced in a targeted way on a molecular level, or by introducing a transcription factor, preferably a synthetic transcription factor like TAL efector activator/repressor or CRISPR-dCas9 activator/repressor, for a given promoter/gene into a cell. In embodiments, where a promoter is modified in a targeted way, the modification is performed by at least one of a site-specific nuclease (SSN) or a catalytically active fragment thereof, or a nucleic acid sequence encoding the same, oligonucleotide directed mutagenesis, chemical mutagenesis, or TILLING.

In one embodiment, the at least one site-specific nuclease (SSN), or the nucleic acid sequence encoding the same, is selected from at least one of a CRISPR nuclease, including Cas or Cpf1 nucleases, a TALEN, a ZFN, a meganuclease, a base editor complex, a restriction endonuclease, including FokI or a variant thereof, a recombinase, or two site-specific nicking endonucleases, or a variant or a catalytically active fragment thereof. Preferably, a targeted point mutation is introduced modifying the promoter region.

In yet another or further embodiment of the method of increasing pathogen resistance, preferably fungal resistance, in a plant cell, tissue, organ, whole plant, or plant material according to the present invention, the method comprises treating the at least one plant cell, tissue, organ, whole plant or plant material according to step (i) with a substance inhibiting the synthesis of at least one benzoxazinoid. A substance inhibiting the synthesis of at least one benzoxazinoid can be a double stranded RNA (dsRNA) which is suitable to reduce the expression level of at least on gene involved in the signalling pathway of, or downstream of at least one wall-associated kinase, or involved in the synthesis pathway of at least one benzoxazinoid, wherein by said reduction of the expression level the synthesis or the amount of at least one benzoxazinoid and thereby increasing pathogen resistance, preferably fungal resistance, in at least one plant cell, tissue, organ, whole plant, or plant material. This down regulating of gene expression is well-known to a person skilled in art as RNAi approach or miRNA interference approach (Fire, A, Xu, S, Montgomery, M, Kostas, S, Driver, S, Mello, C. (1998). Potent and specific genetic interference by double-stranded RNA in Caenorhabditis elegans, Nature 391 (6669): 806-811). Preferably the substance inhibiting the synthesis of at least one benzoxazinoid is at least one siRNA or an siRNA library directed to at least on gene involved in the signalling pathway of, or downstream of at least one wall-associated kinase, or involved in the synthesis pathway of at least one benzoxazinoid. The siRNA or siRNA library can be part of one or more expression cassettes. The siRNA may comprise a first strand of RNA of 15 to 30 nucleotides in length having a 5' end and a 3' end, wherein the first strand is complementary to at least 15 nucleotides of the at least on gene involved in the signalling pathway of, or downstream of at least one wall-associated kinase, or involved in the synthesis pathway of at least one benzoxazinoid, and an second strand of RNA of 15 to 30 nucleotides in length having a 5' end and a 3' end, and wherein at least 12 nucleotides of the first and second strands are complementary to each other and form a small interfering RNA (siRNA) duplex under physiological conditions, and wherein the siRNA silences the at least on gene involved in the signalling pathway of, or downstream of at least one wall-associated kinase, or involved in the synthesis pathway of at least one benzoxazinoid.

The various embodiments of the present application relate to a method of increasing pathogen resistance, preferably fungal resistance, in a plant cell, tissue, organ, whole plant, or plant material, alone or in combination, may result in the targeted reduction of the amount of at least one benzoxazinoid and thereby may lead to an increased pathogen resistance, preferably fungal resistance, in at least one plant cell, tissue, organ, whole plant, or plant material.In yet a further aspect according to the present disclosure there is thus provided use of a substance a for increasing pathogen resistance, preferably fungal resistance, in at least one plant cell, tissue, organ, whole plant, or plant material. The substance may act as a plant protective agent and may be applied to a plant exogenously, or the substance may be a scavenger of any plant molecule or material, or the substance may act as a modulator of WAK, of the downstream signalling cascade, or of a cellular pathway disclosed herein being related to the WAK signalling pathway, preferably a BXD and/or jasmonic acid biosynthesis pathway, or the substance may act on the transcription of any gene involved in the WAK or an associated pathway as disclosed herein, wherein the substance can thus directly or indirectly influence, preferably decrease, the amount of a BXD compound produced and stored in a plant cell. Thereby the use of the substance according to the present disclosure will lead to a decreased BXD level and thus an increased fungal resistance in a plant cell, tissue, organ, or whole plant of interest.

### Delivery methods:

A variety of suitable delivery techniques suitable according to the methods of the present invention for introducing genetic material into a plant cell are known to the skilled person., e.g. by choosing direct delivery techniques ranging from polyethylene glycol (PEG) treatment of protoplasts (Potrykus, Ingo, et al. "Direct gene transfer to cells of a graminaceous monocot." Molecular and General Genetics MGG 199.2 (1985): 183-188), procedures like electroporation (D'Halluin, Kathleen, et al. "Transgenic maize plants by tissue electroporation." The plant cell 4.12 (1992): 1495-1505), microinjection (Neuhaus, G., et al. "Transgenic rapeseed plants obtained by the microinjection of DNA into microspore-derived embryoids." Theoretical and Applied Genetics 75.1 (1987): 30-36), silicon carbide fiber whisker technology (Kaeppler, H. F., et al. "Silicon carbide fiber-mediated stable transformation of plant cells." Tag Theoretical and Applied Genetics 84.5 (1992): 560-566), viral vector mediated approaches (Gelvin, Nature Biotechnology 23, "Viral-mediated plant transformation gets a boost", 684-685 (2005)) and particle bombardment (see e.g. Sood et al., 2011, Biologia Plantarum, 55, 1-15).

Despite transformation methods based on biological approaches, like *Agrobacterium* transformation or viral vector mediated plant transformation, and methods based on physical delivery methods, like particle bombardment or microinjection, have evolved as prominent techniques for introducing genetic material into a plant cell or tissue of interest. Helenius et al. ("Gene delivery into intact plants using the HeliosTM Gene Gun", Plant Molecular Biology Reporter, 2000, 18 (3):287-288) discloses a particle bombardment as physical method for introducing material into a plant cell. Currently, there thus exists a variety of plant transformation methods to introduce genetic material in the form of a genetic construct into a plant cell of interest, comprising biological and physical means known to the skilled person on the field of plant biotechnology and which can be applied to introduce at least one gene encoding at least one wall-associated kinase into at least one cell of at least one of a plant cell, tissue, organ, or whole plant. Notably, said delivery methods for transformation and transfection can be applied to introduce the tools of the present invention simultaneously. A common biological means is transformation with *Agrobacterium spp.* which has been used for decades for a variety of different plant materials. Viral vector mediated plant transformation represents a further strategy for introducing genetic material into a cell of interest. Physical means finding application in plant biology are particle bombardment, also named biolistic transfection or microparticle-mediated gene transfer, which refers to a physical delivery method for transferring a coated microparticle or nanoparticle comprising a nucleic acid or a genetic construct of interest into a target cell or tissue. Physical introduction means are suitable to introduce nucleic acids, i.e., RNA and/or DNA, and proteins. Likewise, specific transformation or transfection methods exist for specifically introducing a nucleic acid or an amino acid construct of interest into a plant cell, including electroporation, microinjection, nanoparticles, and cell-penetrating peptides (CPPs). Furthermore, chemical-based transfection methods exist to introduce genetic constructs and/or nucleic acids and/or proteins, comprising *inter alia* transfection with calcium phosphate, transfection using liposomes, e.g., cationic liposomes, or transfection with cationic polymers, including DEAD-dextran or polyethylenimine, or combinations thereof. Said delivery methods and delivery vehicles or cargos thus inherently differ from delivery tools as used for other eukaryotic cells, including animal and mammalian cells and every delivery method has to be specifically fine-tuned and optimized so that a construct of interest for introducing and/or modifying at least one gene encoding at least one wall-associated kinase in the at least one plant cell, tissue, organ, or whole plant; and/or can be introduced into a specific compartment of a target cell of interest in a fully functional and active way. The above delivery techniques, alone or in combination, can be used for *in vivo* (*in planta*) or *in vitro* approaches.

In one embodiment, a regulatory sequence as described above may be a promoter sequence, wherein the editing or mutation or modulation of the promoter comprises replacing the promoter, or promoter fragment with a different promoter (also referred to as replacement promoter) or promoter fragment (also referred to as replacement promoter fragment), wherein the promoter replacement results in any one of the following or any one combination of the following: an increased promoter activity, an increased promoter tissue specificity, a decreased promoter activity, a decreased promoter tissue specificity, a new promoter activity, an inducible promoter activity, an extended window of gene expression, a modification of the timing or developmental progress of gene expression in the same cell layer or other cell layer, for example, extending the timing of gene expression in the tapetum of anthers, a mutation of DNA binding elements and/or a deletion or addition of DNA binding elements. The promoter (or promoter fragment) to be modified can be a promoter (or promoter fragment) that is endogenous, artificial, pre-existing, or transgenic to the cell that is being edited. The replacement promoter or fragment thereof can be a promoter or fragment thereof that is endogenous, artificial, pre-existing, or transgenic to the cell that is being edited.

The present invention will now be illustrated by the following Examples, which are not construed to limit the scope of the present invention.

### Examples

### Example 1: Plant material and growth conditions

Seventeen maize inbred lines were used, including: (1) historical cultivars B37 and w22, and the NILs B37Htn1 and w22Htn1 that contain the NCLB resistance gene *Htn1*; (2) Breeding line RP3 and its NIL line RP3Htn1 carrying *Htn1* from KWS (see US 2016/0201080 A1); (3) three pairs of mutants RLK1b (S, compromising *Htn1* resistance), RLK1d and RLK1f, and corresponding sister lines RLK1b-wt (R, carrying functional *Htn1*), RLK1d-wt, and RLK1f-wt, which were produced by EMS-mutagenesis in RP3Htn1 (Hurni et al. 2015); (4) three maize mutants (*bx1*, *bx2* and *bx6*) and parental line w22, which were provided by Prof. Georg Jander (Cornell University, Ithaca, US). Two or three maize seeds were sown in each Jiffy pot (ø 8 cm), and fifteen pots were placed in one tray. Seedling plants were grown in a greenhouse condition of 16 h at 20°C in the day, 8 h at 18°C in the night and approximately 60% relative humidity.

### Example 2: NCLB infection tests in the greenhouse

Testing for NCLB resistance using *E*. *turcicum* isolate Passau-1 was performed as previously described with minor modification (Hurni et al. 2015). After the second leaves had fully emerged, the newly emerged leaves were cut and removed until the end of each test experiment. Single spore inoculation and culture on PDA medium plate, harvest and quantification of progeny spores were described (Hurni et al. 2015). Instead of infection by dropping 80 µl spore suspension into the leaf sheath of the second leaf twice, here maize seedlings were infected once by spray (sprayer: ø 28 mm, Semadeni, Ostermundigen, Switzerland). Each 4 trays (ca. 60-80 seedlings) were sprayed with 4 ml of spore suspension (4.5 × 10⁴ spores/ml). A very high humidity mic-condition was produced by placing plastic hoods on top of each tray after infection. Each plant was scored for disease symptom between 11 and 25 days and the severity was evaluated by calculating the area under the disease progress curve (AUDPC) or by quantifying the diseased leaf area of the inoculated second leaves (PrimDLA). About 15 seedling plants were scored in each genotype in each experiment.

### Example 3: Vector construction and subcellular localization

The coding sequence of *ZmWAK-RLK1* was amplified using a cDNA clone as template, which was initially amplified in NCLB resistance genotype RP1Htn1 (Hurni et al. 2015). The PCR fragment was introduced into the Gateway donor vector pDONR207 using the Gateway^{®} BP Clonase^{®} II Enzyme mix (Thermo Fisher Scientific, Wilmington, USA). The generated entry vector carrying the target *ZmWAK-RLK1* sequence was inserted by recombination with the destination vector pUBC-GFP-DEST, to produce an in-frame *ZmWAK-RLK1* + c'-eGFP fusion protein construct driven by Arabidopsis ubiqutin-10 (UBQ10) gene promotor (Grefen, Christopher, et al. "A ubiquitin-10 promoter-based vector set for fluorescent protein tagging facilitates temporal stability and native protein distribution in transient and stable expression studies." The Plant Journal 64.2 (2010): 355-365). The *UBQ10::ZmWAK-RLK1-c'-eGFP* construct (SEQ ID NO:9) together with a reference plasmid PIP2A-mCherry (Cutler et al., Random GFP::cDNA fusions enable visualization of subcellular structures in cells of Arabidopsis at a high frequency. Proc. Natl Acad. Sci. USA 97, 3718-3723, (2000) contains *35S::PIP2A_c'_RFP* construct, which is localized to the plasma membrane) were mixed with nanograde gold particles and co-bombarded into onion epidermal cells, which were subsequently incubated at 20°C in the dark for 2-3 days until being ready for observation using a confocal microscope. Plasmolysis was induced by adding a 0.8 M mannitol solution. Furthermore, both plasmids were transformed into Agrobacterium GV3101 and co-infiltrated into 4-week-old *N. bentaniana* leaves, which were ready for observation 2 days post infiltration. The primers used for vector construct are provided in the below Table 1.

**Table 1: Forward (F) and reverse (R) primers used for vector constructs**

| Order | Target genes | Primers (5' to 3') | PCR efficiency (E) *R²* of Calibration curve slope | Description |
|---|---|---|---|---|
| 1 | *Actin* | F - SEQ ID NO:28 | E=109.8%, R²=0.995, Slope=-3.107 | Reference gene |
| | | R - SEQ ID NO:29 | | |
| 2 | *FPGS* | F - SEQ ID NO:30 | E=104.9%, R²=0.990, Slope=-3.209 | Reference gene |
| | | R - SEQ ID NO: 31 | | |
| 3 | *ZmWAK-RLK1* | F - SEQ ID NO:32 | E=104.0%, R²=0.996, Slope=-3.229 | *WAK-RLK1* = *ZmWAK-RLK1* herein. |
| | | R - SEQ ID NO:33 | | |
| 4 | *BX1* (BX: benzoxazinless) | F - SEQ ID NO:34 | E=100.1%, R²=0.994, Slope=-3.319 | Benzoxazinoid pathway |
| | | R - SEQ ID NO:35 | | |
| 5 | *BX2* | F - SEQ ID NO:36 | E=126.1%, R²=0.989, Slope=-2.821 | Benzoxazinoid pathway |
| | | R - SEQ ID NO:37 | | |
| 6 | *BX3* | F - SEQ ID NO:38 | E=107.4%, R²=0.994, Slope=-3.156 | Benzoxazinoid pathway |
| | | R - SEQ ID NO:39 | | |
| 7 | *EX4* | F - SEQ ID NO:40 | E=100.9%, R²=0.994, Slope=-3.300 | Benzoxazinoid pathway |
| | | R - SEQ ID NO:41 | | |
| 8 | *Bx5* | F - SEQ ID NO:42 | E=109.7%, R²=0.992, Slope=-3.109 | Benzoxazinoid pathway |
| | | R - SEQ ID NO:43 | | |
| 9 | *BX6* | F - SEQ ID NO:44 | E=107.6%, R²=0.961, Slope=-3.153 | Benzoxazinoid pathway |
| | | R - SEQ ID NO:45 | | |
| 10 | *BX7* | F - SEQ ID NO:46 | E=106.9%, R²=0.989, Slope=-3.167 | Benzoxazinoid pathway |
| | | R - SEQ ID NO:47 | | |
| 11 | *BX8* | F - SEQ ID NO:48 | E=114.0%, R²=0.987, Slope=-3.027 | Benzoxazinoid pathway |
| | | R - SEQ ID NO:49 | | |
| 12 | *BX9* | F - SEQ ID NO:50 | E=117.3%, R²=0.999, Slope=-2.967 | Benzoxazinoid pathway |
| | | R - SEQ ID NO:51 | | |
| 13 | *BX10* + *BX11* | F - SEQ ID NO:52 | E=109.5%, R²=0.999, Slope=-3.114 | Benzoxazinoid pathway |
| | | R - SEQ ID NO:53 | | |
| 14 | *BX12* | F - SEQ ID NO:54 | E=95.4%, R²=0.996, Slope=-3.437 | Benzoxazinoid pathway |
| | | R - SEQ ID NO:55 | | |
| 15 | *BX13* | F - SEQ ID NO:56 | E=99.3%, R²=0.961, Slope=-3.340 | Benzoxazinoid pathway |
| | | R - SEQ ID NO:57 | | |
| 16 | *IGL* (Indole Glycerol Phosphate Lyase) | F - SEQ ID NO:58 | E=111.8%, R²=0.996, Slope=-3.069 | Benzoxazinoid pathway |
| | | R - SEQ ID NO:59 | | |
| 17 | *GLU1* (GLU: beta glucosidase) | F - SEQ ID NO:60 | E=110.6%, R²=0.998, Slope=-3.092 | Benzoxazinoid pathway |
| | | R - SEQ ID NO:61 | | |
| 18 | *GLU2* | F - SEQ ID NO:62 | E=110.4%, R²=0.985, Slope=-3.095 | Benzoxazinoid pathway |
| | | R - SEQ ID NO:63 | | |
| 19 | *ZmWAK-RLK1* plasmid construction | F - SEQ ID NO:64 | | |
| | | R - SEQ ID NO:65 | | |

### Example 4: Mycelium development

The second leaves of 21-day seedling plants were harvested and cut into 2 × 2 cm² leaf segments, which were placed and incubated on the phytoagar plates. A spore suspension (4.5 × 10⁴ spores/ml) was painted using swabs on the leaf surface. The petri dishes carrying samples were sealed using PARAFILM and incubated 24 hours at room temperature until harvest. Trypan blue straining was conducted as previously described (Chung, Chia-Lin, et al. "Resistance loci affecting distinct stages of fungal pathogenesis: use of introgression lines for QTL mapping and characterization in the maize-Setosphaeria turcica pathosystem." BMC plant biology 10.1 (2010): 103). The infected segments at 1 dpi were incubated overnight in an acetic acid:ethanol (1:3, v/v) solution, and then in a mixed solution of acetic acid:ethanol:glycerol (1:5: 1, v/v/v) for 4 hours. The samples were stained overnight in 0.01% (w/v) trypan blue lactophenol solution, and then washed once using ddH₂O and stored in 60% glycerol ready for use. Specimens were placed on slides and examined under the ZEISS Axio Imager 2 microscope system (CARL ZEISS, Jena, Germany). The numbers of germinated spores, germ tubes, appressoria and successful penetrations (hyphae inside of cell or between cell walls) were counted. Three independent experiments were performed.

### Example 5: RNA extraction, RNA sequencing and data analysis

The second leaves of seedling plants were harvested with four biological replicates at 0, 9-hpi, 3-dpi and 10-dpi, which corresponded to before inoculation, the germination/penetration, biotrophic growth and necrotrophic growth, respectively (Jennings, P. R., and A. J. Ullstrup. "A HISTOLOGICAL STUDY OF 3 HELMINTHOSPORIUM LEAF BLIGHTS OF CORN." Phytopathology 47.12 (1957): 707-714; Hilu, H. M., and A. L. Hooker. "Host-pathogen relationship of Helminthosporium turcicum in resistant and susceptible corn seedlings." (1964): 570-5). Forty-eight samples (4 genotypes, 4 time points, 3 biological replicates) were subjected for total RNA extraction using SV Total RNA Isolation Kits (Promega, Dübendorf, Switzerland). 1 µl of total RNA was checked by Nanodrop 1000 Spectrophotometer (Thermo Fisher Scientific, Wilmington, USA) to estimate the RNA concentration. Meanwhile, 15 plants in each genotype were evaluated for the AUDPC value to control if the infection worked.

The quantity and quality in RNA sequencing were determined using Qubit^{®} 1.0 Fluorometer (Thermo Fisher Scientific, Wilmington, USA) and Bioanalyzer 2100 (Agilent, Waldbronn, Germany). The TruSeq Stranded mRNA Sample Prep Kit (Illumina, Inc., Hayward, USA) was used for library preparation. 1 µg of total RNA per sample was ribosome depleted and then subjected for synthesizing double-strand cDNA. Each cDNA sample was fragmented, end-repaired, polyadenylated and then ligated with TruSeq adaptor that contains the index for multiplexing. The cDNA fragments containing TruSeq adapters at the both ends were enriched with PCR reaction. The enriched libraries were quantified and qualified, and then normalized to 10 nM. The TruSeq SR Cluster Kit v4 cBot (Illumina, Inc., Hayward, USA) was used for cluster generation using 8 pM of pooled normalized libraries. Sequencing was performed on the Illumina HiSeq2500 at single end 125 bp using the TruSeq SBS Kit v4 (Illumina, Inc., Hayward, USA).

The maize reference genome Zea_mays.AGPv3.27 and the corresponding annotation were downloaded (http://www.maizegdb.org/). The RNA sequencing reads were mapped on the reference genome with STAR (Dobin, Alexander, et al. "STAR: ultrafast universal RNA-seq aligner." Bioinformatics 29.1 (2013): 15-21) allowing one mismatch per 100 bp and no multimapper with the following command: STAR - outFilterMultiMapNmax 1 - outFilterMismatchNoverLmax 0.01 - alignIntronMax 10000. Read counts were determined from the mapping files with featureCounts 1.4.6 (Liao, Yang, Gordon K. Smyth, and Wei Shi. "featureCounts: an efficient general purpose program for assigning sequence reads to genomic features." Bioinformatics 30.7 (2013): 923-930). Statistical analyses were done with the R package edgeR and genes were tested for differential expression with pairwise comparisons and tagwise estimation of dispersion. A gene was considered to be expressed when at least 10 reads were mapped on it and a gene was considered to be differentially expressed with log₂FC ≥ |2| and FDR < 0.01. First, pairwise comparisons were performed between *Htn1* and no *Htn1* plants for each genotype and each time points separately. The results were then compared between time points and then between the two genotypes. The Gene Ontology analysis for differentially expressed genes (DEGs) was conducted by using online software agriGO (Du, Zhou, et al. "agriGO: a GO analysis toolkit for the agricultural community." Nucleic acids research 38.suppl_2 (2010): W64-W70). The significant terms were colored if adjusted *p* ≤ 0.05.

### Example 6: RT-qPCR assay

1µg total RNA was subjected for first strand cDNA synthesis using the iScript Advanced cDNA kit (172-5038, Rio-Rad). 1:20 diluted cDNA was applied for quantifying expression using a Real-Time System C1000TM Thermal cycler (96 or 384 wells, Bio-Rad). The expression of targets was normalized by the reference genes *FPGS* and *Actin* as described (Hurni et al. 2015). The primers for expression analysis are shown in Table 1 above.

### Example 7: Benzoxazinoids (BXDs) extraction and measurement

60 - 100 mg leaves (without veins) of the seedling plants were harvested and freezing immediately in liquid nitrogen, grinded and added the extraction buffer (1 mg sample + 10 µl extraction buffer). The samples were mixed thoroughly and centrifuged at 13,000 rpm under 4°C. The supernatant was transferred into new tube and centrifuged once more under same condition, to remove the possible leaf particles. The supernatant was collected being ready for BXDs measurement.

Benzoxazinoid contents were analyzed by an Acquity UPLC equipment (Waters) coupled to a UV detector and coupled to a mass spectrometer (Waters) (Meihls, Lisa N., et al. "Natural variation in maize aphid resistance is associated with 2, 4-dihydroxy-7-methoxy-1, 4-benzoxazin-3-one glucoside methyltransferase activity." The Plant Cell Online 25.6 (2013): 2341-2355). An Acquity BEH C18 column (Waters) was used. The temperatures of the autosampler and column were 15°C and 40°C, respectively. The mobile phase consisted of 99% water, 1% acetonitrile, and 0.1% Formic acid (A) and acetonitrile and 0.1% Formic acid (B). Flow rate was set to 0.4 ml min⁻¹ with 3% A and 97% B followed by column reconditioning. The injection volume was 5 µl. The extracted trace at 275 nm was used for benzoxazinoids quantification. The following extracted ion chromatograms were used for quantification with a mass window of ±0.01 D: mass-to-charge ratio (m/z) for DIMBOA (retention time [RT] 5.62 min) and DIMBOA-Glc (RT 5.64 min), m/z for HDMBOA-Glc (RT 8.19 min), m/z for HMBOA-Glc (RT 5.34 min) and DIM₂BOA-Glc (RT 5.825 min). Benzoxazinoids absolute concentrations were determined by external calibration curves obtained from purified DIMBOA-Glc, DIMBOA and HDMBOA-Glc standards.

### Example 8: ZmWAK-RLK1 encodes a plasma membrane localized protein

To determine the subcellular localization of the ZmWAK-RLK1 protein, a fusion construct consisting of a full-length coding sequence fused to the sequence of an enhanced green fluorescence protein (eGFP) at the C terminus was generated (cf. SEQ ID NO:9 for the nucleic acid plasmid construct). The ZmWAK-RLK1 fusion protein localized to the plasma membrane before and after plasmolysis when transiently expressed in onion epidermal cells. Furthermore, infiltration into leaves of *Nicotiana benthamiana* confirmed the localization of ZmWAK-RLK1 to the plasma membrane two days after infiltration (cf. Fig. 12). These data, particularly confocal analysis of onion epidermal cells after transient expression of ZmWAK-RKL1-eGFP and the positive control PIP2A-mCherry that is known to localize to the plasma membrane demonstrate that ZmWAK-RLK1 is a plasma membrane protein.

### Example 9: ZmWAK-RLK1 reduced fungal penetrations

Spores of the hemibiotrophic fungus *E*. *turcicum* penetrate the maize epidermis mostly between 6-18 hours after inoculation (hpi) (Jennings and Ullstrup, 1957). To investigate if *ZmWAK-RLK1* changes the outcome of fungal penetration attempts, we investigated the infection process at one day post inoculation (dpi) using trypan blue staining (data not shown). The number of successful penetration events were evaluated in three EMS-induced *ZmWAK-RLK1* loss-of-function mutant lines (RLK1b, RLK1d and RLK1f) and their corresponding sister lines that were generated in the near isogenic line (NIL) RP3Htn1 (Hurni et al. 2015). No significant difference in the establishment of germ tubes and appressoria was observed in genotypes with/without *ZmWAK-RLK1* (data not shown). In contrast, the number of successful penetration events was significantly lower if *ZmWAK-RLK1* was functional compared to loss-of-function mutants as demonstrated in Fig. 2 A and B. This indicates that *ZmWAK-RLK1* leads to a reduction of pathogen penetrations into host tissues.

### Example 10: Transcriptome and metabolism analysis identified alterations to the BXDs biosynthesis pathway in the presence of ZmWAK-RLK1

To decipher the immune network specifically influenced by *ZmWAK-RLK1,* we performed a transcriptome analysis by RNA sequencing in two pairs of near isogenic lines, w22 and W22Htn1 as well as B37 and B37Htn1. NCLB development was significantly reduced in the presence of *ZmWAK-RLK1* in both NILs (Fig. 3 A-C). Leaf samples were collected at 0 and 9 hpi (penetration stage) as well as 3 (biotrophic growth) and 10 dpi (necrotrophic growth) (Jennings and Ullstrup, 1957). Forty-eight samples were sequenced and 1.159 billion reads were obtained (Table 2). More than 820 million reads were uniquely mapped with an average of 17.08 million reads per sample (70.7%) (Table 2). No obvious difference of percentage of mapped reads in genotypes with or without *Htn1* was observed. A total of 15,345 genes were expressed and they were used for further analysis. By conducting a multidimensional scaling (MDS) analysis using expression normalized by edgeR, the biological replicates for the same genotype-timepoints combination were mostly grouped together, suggesting the repeatability of replicates (data not shown). More differentially expressed gene (DEGs) were detected in B37Htn1/B37 compared to w22Htn1/w22 (Fig. 4). These genes differently expressed in both NILs in at least one of timepoints. To identify DEGs associated with *ZmWAK-RLK1* and to rule out genetic background effects, only genes that were differentially expressed in both NIL pairs were considered.

**Table 2: Statistics of RNA-seq reads sequenced and mapped**

| Sample number | Samples | Raw reads | Uniquely mapped reads^{a} | Percentage of uniquely mapped reads (%) | Percentage of multi-mapped reads (%) | Percentage of unmapped reads (%) |
|---|---|---|---|---|---|---|
| 1 | 0-w22-1 | 19,894,158 | 12,757,882 | 64.13 | 12.66 | 20.34 |
| 2 | 0-w22-2 | 25,718,662 | 18,612,570 | 72.37 | 6.63 | 20.18 |
| 3 | 0-w22-3 | 24,937,383 | 17,948,211 | 71.97 | 6.41 | 20.85 |
| 4 | 0-w22Htn1-1 | 27,452,042 | 20,264,307 | 73.82 | 5.96 | 19.70 |
| 5 | 0-w22Htn1-2 | 19,815,200 | 14,439,891 | 72. 87 | 6.07 | 20.44 |
| 6 | 0-w22Htn 1-3 | 24,173,527 | 16,215,534 | 67.08 | 9.55 | 21.40 |
| 7 | 0-B37-1 | 22,180,888 | 14,689,311 | 66.23 | 5.27 | 28.03 |
| 8 | 0-B37-2 | 24,474,895 | 18,273,286 | 74.66 | 5.96 | 18.75 |
| 9 | 0-B37-3 | 27,774,311 | 20,221,020 | 72.80 | 6.73 | 19.74 |
| 10 | 0-B37Htn1-1 | 26,728,646 | 19,277,404 | 72.12 | 6.16 | 21.04 |
| 11 | 0-B37Htn1-2 | 24,336,731 | 17,640,859 | 72.49 | 606 | 20.82 |
| 12 | 0-B37Htn1-3 | 22,625,055 | 16,499,617 | 72.93 | 6.15 | 20.30 |
| 13 | 9h-w22-1 | 25,343,283 | 17,460,901 | 68.90 | 7.81 | 22.49 |
| 14 | 9h-w22-2 | 26,384,078 | 18,489,206 | 70.08 | 7.16 | 22.03 |
| 15 | 9h-w22-3 | 21,846,924 | 13,680,343 | 62.62 | 5.97 | 30.81 |
| 16 | 9h-w22Htn1-1 | 34,074,874 | 23,121,230 | 67.85 | 6.27 | 25.40 |
| 17 | 9h-w22Htn1-2 | 24,276,403 | 17,299,847 | 71.26 | 702 | 21.07 |
| 18 | 9h-w22Htn1-3 | 30,200,422 | 20,381,456 | 67.49 | 7.23 | 24.62 |
| 19 | 9h-B37-1 | 17,962,777 | 12,878,288 | 71.69 | 7.38 | 20.10 |
| 20 | 9h-B37-2 | 23,815,810 | 16,892,476 | 70.93 | 7.12 | 21.22 |
| 21 | 9h-B37-3 | 24,188,988 | 17,604,433 | 72.78 | 7.15 | 19.38 |
| 22 | 9h-B37Htn1-1 | 25,195,971 | 17,304,100 | 68.68 | 7.80 | 22.65 |
| 23 | 9h-B37Htn1-2 | 23,902,398 | 16,173,755 | 67.67 | 7.27 | 24.40 |
| 24 | 9h-B37Htn1-3 | 24,731,481 | 17,279,056 | 69. 87 | 7.13 | 22.39 |
| 25 | 3d-w22-1 | 22,399,000 | 15,772,632 | 70.42 | 6.44 | 22.49 |
| 26 | 3d-w22-2 | 26,175,191 | 18,816,409 | 71.89 | 6.43 | 21.06 |
| 27 | 3d-w22-3 | 23,253,678 | 16,758,123 | 7207 | 6.45 | 20.82 |
| 28 | 3d-w22Htn1-1 | 23,878,639 | 17,185,076 | 71.97 | 5.92 | 21.58 |
| 29 | 3d-w22Htn1-2 | 20,568,384 | 14,338,420 | 69.71 | 709 | 22.35 |
| 30 | 3d-w22Htn1-3 | 34,008,596 | 24,127,141 | 70.94 | 5.81 | 22.76 |
| 31 | 3d-B37-1 | 23,690,913 | 14,726,042 | 62.16 | 4.70 | 32.71 |
| 32 | 3d-B37-2 | 27,646,350 | 19,934,380 | 72.10 | 5.89 | 21.42 |
| 33 | 3d-B37-3 | 21,114,803 | 16,208,484 | 76.76 | 5.69 | 16.98 |
| 34 | 3d-B37Htn1-1 | 21,758,134 | 15,409,264 | 70.82 | 5.68 | 2303 |
| 35 | 3d-B37Htn1-2 | 27,236,811 | 19,866,766 | 72.94 | 5.84 | 20.68 |
| 36 | 3d-B37Htn1-3 | 22,700,637 | 16,553,064 | 72.92 | 5.60 | 21.00 |
| 37 | 10d-w22-1 | 23,051,842 | 16,566,330 | 71.87 | 6.16 | 21.24 |
| 38 | 10d-w22-2 | 28,611,557 | 20,463,896 | 71.52 | 6.01 | 21.77 |
| 39 | 10d-w22-3 | 12,829,435 | 7,480,564 | 58.31 | 4.82 | 36.38 |
| 40 | 10d-w22Htn1-1 | 24,435,356 | 17,199,294 | 70.39 | 5.45 | 23.76 |
| 41 | 10d-w22Htn1-2 | 28,996,022 | 21,442,119 | 73.95 | 5.78 | 19.69 |
| 42 | 10d-w22Htn1-3 | 18,649,090 | 13,429,238 | 72.01 | 5.78 | 21.71 |
| 43 | 10d-B37-1 | 13,275,463 | 9,658,508 | 72.75 | 5.46 | 21.16 |
| 44 | 10d-B37-2 | 15,217,491 | 11,288,257 | 74.18 | 5.38 | 19.89 |
| 45 | 10d-B37-3 | 23,349,541 | 17,096,788 | 73.22 | 5.36 | 20.83 |
| 46 | 10d-B37Htn1-1 | 20,104,137 | 14,270,397 | 70.98 | 4.94 | 23.79 |
| 47 | 10d-B37Htn1-2 | 38,203,107 | 27,216,898 | 71.24 | 5.40 | 22.82 |
| 48 | 10d-B37Htn1-3 | 26,094,601 | 18,820,165 | 72.12 | 5.61 | 21.68 |
| Total | | 1,159,283,685 | 820,033,238 | 70.74 | NA^{b} | NA^{b} |
| Mean | | 24,151,743 | 17,084,026 | 70.74 | NA^{b} | NA^{b} |

| | | | | | | |
|---|---|---|---|---|---|---|
| "Parameters for mapping: less than 1% mismatch, 1 locus mapped, intron size is less than 10 kb; ^{b}NA = not analyzed. | | | | | | |

Two-hundred and fifteen common DEGs were identified across all time points (Fig. 13). 132 and 83 genes were induced and repressed in the *ZmWAK-RLK1*-containing NIL compared to the respective susceptible line (heat map and Venn diagram not shown). 108 DEGs were only found at timepoint 0 before inoculation, while 107 of DEGs were found only after infection. Twenty-nine DEGs were differently expressed at all time points including timepoint. An overrepresentation analysis using agriGO was performed to identify enriched Gene Ontology (GO) terms associated with *ZmWAK-RLK1.* The functional groups were enriched for terms of defense response (e.g. GO:0009814) and metabolic/biosynthetic process (e.g. GO:0006725) (data not shown).

To further analyze if the presence of *ZmWAK-RLK1* is associated with BXDs biosynthesis (Fig. 5 A), the content of major BXDs in second leaves of w22Htn1 and w22 before and after infection was quantified (Fig. 5 B to F). The content of four BXDs DIMBOA-Glc, DIMBOA, HMBOA-Glc and DIM₂BOA-Glc was significantly lower in w22Htn1 compared to w22 at all timepoints (data not shown), which indicated a constitutive reduction on BXDs content when *ZmWAK-RLK1* is present. Furthermore, via RT-qPCR the transcriptional levels of *ZmWAK-RLK1* and specifically the genes in the BXDs biosynthesis pathway before and after pathogen inoculation were determined. The expression of genes (A) *ZmWAK-RLK1,* (B) *Bx1,* (C) *Igl,* (D) *Bx2,* (E) *Bx3,* (F) *Bx4,* (G) *Bx5,* (H) *Bx6,* (I) *Bx7,* (J) *Bx8,* (K) *Bx9,* (L) *Bx10*/*11*, (M) *Bx12*, (N) *Ex13,* (O) *Glu1* and (P) *Glu2* at different timepoints before and after infection was measured and statistics were conducted separately in w22 and B37 genetic background using Tukey's HSD (P = 0.05) in four biological replicates.

The *ZmWAK-RLK1* expression in NILs showed no significant difference (Fig. 11 A). Overall, the transcriptional levels of BXD biosynthesis genes were genotype-specific (Fig. 11 A to P)). For instance, BX1 and its protein homolog IGL can convert indole-3-glycerol phosphate into indole, which is the first step of BXD metabolism (Frey et al. 2000). Their coding genes *Bx1* and *Igl* showed opposite contributions of gene expression in B37 and w22, but the combined expression of *Bx1* and *Igl* was consistently lower in genotypes with *ZmWAK-RLK1* (Fig. 11 B and C).

### Example 11: Mutations in ZmWAK-RLK1 is associated with the reduction of secondary metabolite DIM₂BOA-Glc

To further analyze the role of different BXD biosynthesis genes as well as the metabolites of this pathway in NCLB resistance, the ZmWAK-RLK1 mutants (RLK1b, d and f, SEQ ID NOs: 3 to 6 and G548R mutant of SEQ ID NO:2) and their sister lines were used (Hurni et al. 2015). The transcript levels of several BXD genes were quantified and the content of major BXD compounds in mutants which lost the resistance caused by ZmWAK-RLK1 (Fig. 6 A to D). In contrast to the NILs, there was no difference in the DIMBOA-Glc, DIMBOA and HMBOA, HDMBOA-Glc concentration. However, the content of DIM₂BOA-Glc was significantly lower when *ZmWAK-RLK1* was intact (Fig. 6 A, and further data not shown). The reduced DIM₂BOA-Glc correlated with a reduction in *Igl* transcript levels, while no obvious difference in the transcriptional levels of *ZmWAK-RLK1* and *Bx1* was detected in *ZmWAK-RLK1* (Fig. 6 B to D) *Bx6*, *Bx7* and *Bx13* are key genes of the BXDs pathway to produce DIM₂BOA-Glc, and these genes were slightly but not significantly upregulated in mutants (data not shown). This phenomenon can be explained by a feedback regulation. Therefore, ZmWAK-RLK1 clearly seems to be associated with the reduction of secondary metabolite DIM₂BOA-Glc, possibly by reducing the expression levels of *Bx1* and/or *Igl.*

### Example 12: Mutations in BXDs biosynthesis genes decreased NCLB susceptibility

To further analyze the role of BXDs in NCLB resistance/susceptibility, mutants in the three BXD biosynthesis genes *Bx1*, *Bx2* and *Bx6* were tested upon inoculation with *E. turcicum.* These mutants showed strong reduction in several BXDs compounds, including DIM₂BOA-Glc (Fig. 7). Interestingly, all three mutants showed a strong reduction of NCLB susceptibility at the seedling stage (Fig. 8 A and B). This confirmed a negative correlation of BXDs content and NCLB disease resistance. Furthermore, the *ZmWAK-RLK1* expression at 10 dpi was checked (Fig. 8 C). No significant difference was detected in the *bx* mutants if compared to the wild-type. Considering the reduction of *Bx1* and *Igl* co-expression in *ZmWAK-RLK1* genotypes (Fig. 6 A, Fig. 5 B and C, Fig. 9 B), the data suggest that the BXDs synthesis pathway likely acts downstream of *ZmWAK-RLK1.*

Therefore, *ZmWAK-RLK1* underlying quantitative NCLB disease resistance is based on a decrease of the biosynthesis of secondary metabolite BXDs, and DIM₂BOA-Glc served as a candidate susceptibility component for promoting fungal infection (Fig. 8 D).

### Example 13: Tissue-specific expression of WAK-RLK1

Further to the determination of the subcellular localization of a *ZmWAK-RLK1* protein (see Example 8 above), the tissue specific expression of RLK1 in different genotypes, and different time points was determined. The genes *FPGS* and *ACTIN* served as reference to normalize the expression. The results shown in Fig. 10 demonstrate that RLK1 is a ubiquitously and steadily expressed gene which further underpins the function of said kinase as master regulator in relevant plant metabolic pathways.

### Example 14: TILLING

To develop and screen a mutant population of plant material, TILLING was performed. A TILLING mutant population can be created, e.g., starting from KWS line RP3Htn1 according to Kato (2000, The maize handbook, pp. 212-219)). Pollen is harvested from field-grown RP3Htn1 plants and treated with 0.1% EMS solution for 45 min. Silks of individual plants are then pollinated and emerging ears bagged. From 436 pollinated M0 plants seeds were harvested, in one experiment. An additional propagation and selfing led to 10,084 individual M1 plants. Leaf material from these M1 plants was collected for DNA isolation. DNA of dried leaf samples (10 leaf discs bunches/sample) was isolated from 10,000 M1 individuals with the CTAB extraction method (Traitgenetics, Gatersleben, Germany). DNA is then aliquoted to 100µl with 20ng/pl. Primer development for mutant screening is performed. The amplification assay consisted of 20 ng/µl DNA, 5x GoTaq-Buffer, 25 µM dNTPs, 10 µM forward Primer, 10 µM reverse Primer, 5 Units/µl GoTaq. After denaturation for 300 s at 94°C the amplification cycles were performed with 35 cycles of 60 s at 94°C, 60 s at 60°C and 60 s at 72°C followed by a final elongation time for 600 s at 72°C. Next, Sanger-sequencing of PCR products is performed according to established protocols. Sequences are then assembled, for example with the help of the software Lasergene Seqman NGen (DNASTAR) and heterozygote SNPs called with the software default settings. Positive mutant plants were sequenced again with the Sanger-method in order to confirm the polymorphism.

## Claims

1. A method for producing a *Zea mays* plant having increased *Helminthosporium turcicum* resistance, wherein the *Helminthosporium turcicum* resistance is regulated by at least one wall-associated kinase, the method comprising:
(i) (a) providing at least one plant cell, tissue, organ, or whole plant having a genotype with at least one gene encoding a wall-associated kinase in the genome of said plant cell, tissue, organ, or whole plant; or
(i) (b) introducing at least one gene encoding a wall-associated kinase into the genome of at least one cell of at least one of a plant cell, tissue, organ, or whole plant, wherein the at least one gene encoding at least one wall-associated kinase is stably introduced into said genome, and wherein a stable introduction comprises Agrobacterium-mediated transformation, genome editing, or a combination thereof; and
(ii) (a) mutating the at least one gene encoding the wall-associated kinase in the at least one plant cell, tissue, organ, or whole plant via deletion, insertion or substitution of one or more nucleotides by at least one of a site-specific nuclease (SSN), oligonucleotide directed mutagenesis, chemical mutagenesis, or TILLING; and/or
(ii) (b) increasing the expression level of at least one wall-associated kinase and/or reducing the expression level of at least one molecule within the signaling pathway from the at least one wall-associated kinase to the synthesis of at least one benzoxazinoid or within the synthesis pathway of at least one benzoxazinoid in the at least one plant cell, tissue, organ, or whole plant by at least one of a site-specific nuclease (SSN), oligonucleotide directed mutagenesis, chemical mutagenesis, or TILLING; and
(iii) selecting a plant generated from the at least one plant cell, tissue, organ, or whole plant of (ii) (a) or (ii) (b), which has increased *Helminthosporium turcicum* resistance, based on the determination of a reduced synthesis of the benzoxazinoid DIM₂BOA being in the glucoside or aglucone form, or a benzoxazolinone, in response to *Helminthosporium turcicum* infection as compared to a corresponding control plant of the same genotype lacking the mutation of (ii) (a) or the modulation of (ii) (b), wherein the synthesis of the at least one benzoxazinoid is regulated by the at least one wall-associated kinase;
wherein the at least one wall-associated kinase is a WAK-RLK1 and
a) is encoded by a nucleic acid molecule comprising the nucleotide sequence of SEQ ID NO: 1 or 7,
b) is encoded by a nucleic acid molecule comprising the nucleotide sequence having at least 80% sequence identity to nucleotide sequence of SEQ ID NO: 1 or 7, preferably over the entire length of the sequence,
c) is encoded by a nucleic acid molecule hybridizing with a complementary sequence to a) or b) under stringent conditions, wherein stringent hybridization conditions are hybridization in 4×SSC at 65°C and subsequent multiple washes in 0.1 × SSC at 65°C for approximately 1 hour,
d) is encoded by a nucleic acid molecule comprising the nucleotide sequence coding for an amino acid sequence of SEQ ID NO: 2 or 8,
e) is encoded by a nucleic acid molecule comprising the nucleotide sequence coding for an amino acid sequence having at least 80% sequence identity to amino acid sequence of SEQ ID NO: 2 or 8, preferably over the entire length of the sequence,
f) comprises the amino acid sequence of SEQ ID NO: 2 or 8, or
g) comprises an amino acid sequence having at least 80% sequence identity to amino acid sequence of SEQ ID NO: 2 or 8, preferably over the entire length of the sequence,
provided that any sequence of a) to g), optionally after expression, still encodes at least one functional Htn1 according to SEQ ID NO: 2, Ht2 or Ht3 according to SEQ ID NO: 8, or an allelic variant of the wall-associated kinase which is located at or mapped on a locus in bin 5 or bin 6 on the long arm of chromosome 8;
wherein the at least one molecule within the signaling pathway from the at least one wall-associated kinase to the synthesis of at least one benzoxazinoid or within the synthesis pathway of at least one benzoxazinoid is selected from the group constiting of:
I) the genes *bx1* (SEQ ID NO: 10), *bx2* (SEQ ID NO: 12), *igl* (SEQ ID NO: *14), bx6* (SEQ ID NO: 16), *bx11* (SEQ ID NO: 18), *bxl4* (SEQ ID NO: 20), *opr2* (SEQ ID NO: 22), *lox3* (SEQ ID NO: 24) *or aocl* (SEQ ID NO: 26), or
II) a gene having identity of at least 80% to one of the genes of I), preferably over the full length of the gene, in particular the coding regions of the gene, or
III) the proteins BX1 (SEQ ID NO: 11), BX2 (SEQ ID NO: 13), IGL (SEQ ID NO: 15), BX6 (SEQ ID NO: 17), BX11 (SEQ ID NO: 19), BX14 (SEQ ID NO: 21), OPR2 (SEQ ID NO: 23), LOX3 (SEQ ID NO: 25) or AOC1 gene (SEQ ID NO: 27), or
IV) a protein having identity of at least 80% to one of the proteins of III), preferably over the full length of the protein, and
wherein the benzoxazinoid whose synthesis is regulated by the at least one wall-associated kinase is DIM₂BOA, the aforementioned benzoxazinoid being in the glucoside or aglucone form, or a benzoxazolinone, or any combination with DIMBOA, HMBOA, HM₂BOA, HDMBOA, HDM₂BOA, HBOA, DHBOA, DIBOA or TRIBOA;
wherein the method is no essentially biological process for the production of the *Zea mays* plant.

2. The method according to claim 1, wherein the at least one molecule within the signalling pathway from the at least one wall-associated kinase to the synthesis of at least one benzoxazinoid or within the synthesis pathway of at least one benzoxazinoid is selected from the group constiting of:
I) the genes *bx1* (SEQ ID NO: 10), *bx2* (SEQ ID NO: 12), *igl* (SEQ ID NO: *14), bx6* (SEQ ID NO: 16), *bx11* (SEQ ID NO: 18), *bx14* (SEQ ID NO: 20), *opr2* (SEQ ID NO: 22), *lox3* (SEQ ID NO: 24) *or aocl* (SEQ ID NO: 26), or
II) a gene having identity of at least 90% to one of the genes of I), preferably over the full length of the gene, in particular the coding regions of the gene, or
III) the proteins BX1 (SEQ ID NO: 11), BX2 (SEQ ID NO: 13), IGL (SEQ ID NO: 15), BX6 (SEQ ID NO: 17), BX11 (SEQ ID NO: 19), BX14 (SEQ ID NO: 21), OPR2 (SEQ ID NO: 23), LOX3 (SEQ ID NO: 25) or AOC1 gene (SEQ ID NO: 27), or
IV) a protein having identity of at least 90% to one of the proteins of III), preferably over the full length of the protein.

## Patentansprüche

1. Verfahren zum Herstellen einer *Zea mays*-Pflanze mit verstärkter *Helminthosporium turcicum*-Resistenz, wobei die *Helminthosporium turcicum*-Resistenz durch mindestens eine wandassoziierte Kinase reguliert wird, wobei das Verfahren Folgendes umfasst:
(i) (a) Bereitstellen mindestens einer Pflanzenzelle, mindestens eines Gewebes, mindestens eines Organs oder mindestens einer ganzen Pflanze mit einem Genotyp mit mindestens einem Gen, das eine wandassoziierte Kinase codiert, im Genom der Pflanzenzelle, des Gewebes, des Organs oder der ganzen Pflanze; oder
(i) (b) Einführen mindestens eines Gens, das eine wandassoziierte Kinase codiert, in das Genom mindestens einer Zelle von mindestens einem aus einer Pflanzenzelle, einem Gewebe, einem Organ oder einer ganzen Pflanze, wobei das mindestens eine Gen, das eine wandassoziierte Kinase codiert stabil in das besagte Genom eingeführt ist, und wobei die stabile Einführung eine Agrobakterium-basierte Transformation, Genom Editierung, oder eine Kombination davon, umfasst; und
(ii) (a) Mutieren des mindestens einen Gens, das die wandassoziierte Kinase codiert, in der mindestens einen Pflanzenzelle, dem mindestens einen Gewebe, dem mindestens einen Organ oder der mindestens einen ganzen Pflanze durch Deletion, Insertion oder Substitution von einem oder mehreren Nukleotiden durch mindestens eines aus einer positionsspezifischen Nuklease (SSN), Oligonukleotid-gerichteter Mutagenese, chemischer Mutagenese oder TILLING; und/oder
(ii) (b) Erhöhen des Expressionsniveaus mindestens einer wandassoziierten Kinase und/oder Reduzieren des Expressionsniveaus mindestens eines Moleküls innerhalb des Signalwegs von der mindestens einen wandassoziierten Kinase zur Synthese mindestens eines Benzoxazinoids oder innerhalb des Synthesewegs von mindestens einem Benzoxazinoid in der mindestens einen Pflanzenzelle, dem mindestens einen Gewebe, dem mindestens einen Organ oder der mindestens einen ganzen Pflanze durch mindestens eines aus einer positionsspezifischen Nuklease (SSN), Oligonukleotid-gerichteter Mutagenese, chemischer Mutagenese oder TILLING; und
(iii) Auswählen einer Pflanze, die aus der mindestens einen Pflanzenzelle, dem mindestens einen Gewebe, dem mindestens einen Organ oder der mindestens einen ganzen Pflanze von (ii) (a) oder (ii) (b) erzeugt wurde, die eine erhöhte *Helminthosporium turcicum*-Resistenz aufweist, basierend auf der Bestimmung einer reduzierten Synthese des Benzoxazinoids DIMzBOA in der Glucosid- oder Agluconform oder eines Benzoxazolinons als Reaktion auf eine *Helminthosporium turcicum*-Infektion im Vergleich zu einer entsprechenden Kontrollpflanze des gleichen Genotyps, der die Mutation von (ii) (a) oder die Modulation von (ii) (b) fehlt, wobei die Synthese des mindestens einen Benzoxazinoids durch die mindestens eine wandassoziierte Kinase reguliert wird;
wobei die mindestens eine wandassoziierte Kinase eine WAK-RLK1 ist und
a) von einem Nukleinsäuremolekül codiert wird, dass die Nukleotidsequenz von SEQ ID NR.: 1 oder 7 umfasst,
b) von einem Nukleinsäuremolekül codiert wird, dass die Nukleotidsequenz umfasst, die eine Sequenzidentität von mindestens 80 % mit der Nukleotidsequenz von SEQ ID NR.: 1 oder 7 aufweist, vorzugsweise über die gesamte Länge der Sequenz,
c) von einem Nukleinsäuremolekül codiert wird, das unter stringenten Bedingungen mit einer zu a) oder b) komplementären Sequenz hybridisiert, wobei es sich bei stringenten Hybridisierungsbedingungen um eine Hybridisierung in 4×SSC bei 65 °C und anschließende mehrfache Waschschritte in 0,1×SSC bei 65 °C für etwa 1 Stunde handelt,
d) von einem Nukleinsäuremolekül codiert wird, dass die Nukleotidsequenz umfasst, die eine Aminosäure von SEQ ID NR.: 2 oder 8 codiert,
b) von einem Nukleinsäuremolekül codiert wird, dass die Nukleotidsequenz umfasst, die eine Aminosäuresequenz codiert, die eine Sequenzidentität von mindestens 80 % mit der Aminosäuresequenz von SEQ ID NR.: 2 oder 8 aufweist, vorzugsweise über die gesamte Länge der Sequenz,
f) die Aminosäuresequenz von SEQ ID NR.: 2 oder 8 umfasst, oder
g) eine Aminosäuresequenz umfasst, die eine Sequenzidentität von mindestens 80 % mit der Aminosäuresequenz von SEQ ID NR.: 2 oder 8 aufweist, vorzugsweise über die gesamte Länge der Sequenz,
vorausgesetzt, dass eine beliebige Sequenz von a) bis g), gegebenenfalls nach Expression, immer noch mindestens eine funktionelle Htn 1 gemäß SEQ ID NR.: 2, Ht2 oder Ht3 gemäß SEQ ID NR.: 8, oder eine allelische Variante der wandassoziierten Kinase codiert, die sich an einem Lokus in Bin 5 oder Bin 6 auf dem langen Arm von Chromosom 8 befindet oder darauf kartiert ist;
wobei das mindestens eine Molekül innerhalb des Signalwegs von der mindestens einen wandassoziierten Kinase zur Synthese mindestens eines Benzoxazinoids oder innerhalb des Synthesewegs mindestens eines Benzoxazinoids ausgewählt ist aus der Gruppe bestehend aus:
I) den Genen *bx1* (SEQ ID NR.: 10), *bx2* (SEQ ID NR.: 12), *igl* (SEQ ID NR.: *14), bx6* (SEQ ID NR.: 16), *bx11* (SEQ ID NR.: 18), *bxl4* (SEQ ID NR.: 20), *opr2* (SEQ ID NR.: 22), *lox3* (SEQ ID NR.: 24) oder *aoc1* (SEQ ID NR.: 26), oder
II) einem Gen mit Identität von mindestens 80 % zu einem der Gene von I), vorzugsweise über die gesamte Länge des Gens, insbesondere der codierenden Regionen des Gens, oder
III) den Proteinen BX1 (SEQ ID NR.: 11), BX2 (SEQ ID NR.: 13), IGL (SEQ ID NR.: 15), BX6 (SEQ ID NR.: 17), BX11 (SEQ ID NR.: 19), BX14 (SEQ ID NR.: 21), OPR2 (SEQ ID NR.: 23), LOX3 (SEQ ID NR.: 25) oder dem AOC1-Gen (SEQ ID NR.: 27), oder
IV) einem Protein mit Identität von mindestens 80 % zu einem der Proteine von III), vorzugsweise über die gesamte Länge des Proteins, und
wobei es sich bei dem Benzoxazinoid, dessen Synthese, durch die mindestens eine wandassoziierte Kinase reguliert wird, um DIM₂BOA, wobei das genannte Benzoxazinoid in der Glucosid- oder Agluconform vorliegt, oder um ein Benzoxazolinon oder eine beliebige Kombination mit DIMBOA, HMBOA, HM₂BOA, HDMBOA, HDM₂BOA, HBOA, DHBOA, DIBOA oder TRIBOA handelt;
wobei das Verfahren kein im Wesentlichen biologisches Verfahren zur Herstellung der *Zea mays*-Pflanze ist.

2. Verfahren nach Anspruch 1, wobei das mindestens eine Molekül innerhalb des Signalwegs von der mindestens einen wandassoziierten Kinase zur Synthese mindestens eines Benzoxazinoids oder innerhalb des Synthesewegs mindestens eines Benzoxazinoids ausgewählt ist aus der Gruppe bestehend aus:
I) den Genen *bx1* (SEQ ID NR.: 10), *bx2* (SEQ ID NR.: 12), *igl* (SEQ ID NR.: 14), *bx6* (SEQ ID NR.: 16), *bx11* (SEQ ID NR.: 18), *bx14* (SEQ ID NR.: 20), *opr2* (SEQ ID NR.: 22), *lox3* (SEQ ID NR.: 24) oder *aoc1* (SEQ ID NR.: 26), oder
II) einem Gen mit Identität von mindestens 90 % zu einem der Gene von I), vorzugsweise über die gesamte Länge des Gens, insbesondere der codierenden Regionen des Gens, oder
III) den Proteinen BX1 (SEQ ID NR.: 11), BX2 (SEQ ID NR.: 13), IGL (SEQ ID NR.: 15), BX6 (SEQ ID NR.: 17), BX11 (SEQ ID NR.: 19), BX14 (SEQ ID NR.: 21), OPR2 (SEQ ID NR.: 23), LOX3 (SEQ ID NR.: 25) oder dem AOC1-Gen (SEQ ID NR.: 27), oder
IV) einem Protein mit Identität von mindestens 90 % zu einem der Proteine von III), vorzugsweise über die gesamte Länge des Proteins.

## Revendications

1. Procédé de production d'une plante *Zea mays* présentant une résistance accrue à *Helminthosporium turcicum,* dans lequel la résistance à *Helminthosporium turcicum* est régulée par au moins une WAK (wall-associated kinase), le procédé comprenant :
(i) (a) la fourniture d'au moins un(e) cellule, tissu, organe de plante, ou plante entière présentant un génotype avec au moins un gène codant pour une WAK (wall-associated kinase) dans le génome de ladite/dudit cellule, tissu, organe de plante, ou plante entière ; ou
(i) (b) l'introduction d'au moins un gène codant pour une WAK (wall-associated kinase) dans le génome d'au moins un(e) cellule, tissu, organe de plante ou plante entière, dans lequel au moins un gène codant pour au moins une kinase associée à la paroi est introduit de manière stable dans ledit génome, et dans lequel une introduction stable comprend une transformation médiée par Agrobacterium, l'édition du génome ou une combinaison de ceux-ci; et
(ii) (a) la mutation d'au moins un gène codant pour la WAK (wall-associated kinase) dans au moins un(e) cellule, tissu, organe de plante, ou plante entière par délétion, insertion ou substitution d'un ou plusieurs nucléotides par au moins une nucléase site-spécifique (SSN), une mutagenèse dirigée par un oligonucléotide, une mutagenèse chimique, ou un TILLING ; et/ou
(ii) (b) l'augmentation du niveau d'expression d'au moins une WAK (wall-associated kinase) et/ou la réduction du niveau d'expression d'au moins une molécule à l'intérieur de la voie de signalisation d'au moins une WAK (wall-associated kinase) à la synthèse d'au moins un benzoxazinoïde ou à l'intérieur de la voie de synthèse d'au moins un benzoxazinoïde dans au moins un(e) cellule, tissu, organe de plante ou plante entière par au moins une nucléase site spécifique (SSN), une mutagenèse dirigée par un oligonucléotide, une mutagenèse chimique ou un TILLING ; et
(iii) la sélection d'une plante générée à partir d'au moins un(e) cellule, organe, tissu de plante ou plante entière de (ii) (a) ou (ii) (b), qui présente une résistance accrue à *Helminthosporium turcicum,* sur la base de la détermination d'une synthèse réduite du benzoxazinoïde DIM₂BOA étant sous la forme glucoside ou aglucone, ou d'une benzoxazolinone, en réponse à une infection par *Helminthosporium turcicum* comparé à une plante témoin correspondante du même génotype ne présentant pas la mutation de (ii) (a) ou la modulation de (ii) (b), dans lequel la synthèse d'au moins un benzoxazinoïde est régulée par au moins une WAK (wall-associated kinase) ;
dans lequel au moins une WAK (wall-associated kinase) est une WAK-RLK1 et
a) est codée par une molécule d'acide nucléique comprenant la séquence nucléotidique de SEQ ID NO : 1 ou 7,
b) est codée par une molécule d'acide nucléique comprenant la séquence nucléotidique présentant une identité de séquence d'au moins 80 % avec la séquence nucléotidique de SEQ ID NO : 1 ou 7, de préférence sur la longueur totale de la séquence,
c) est codée par une molécule d'acide nucléique s'hybridant avec une séquence complémentaire de a) ou b) dans des conditions stringentes, dans lequel les conditions d'hybridation stringentes sont une hybridation dans du SSC 4× à 65°C et de multiples lavages subséquents dans du SSC 0,1× à 65°C pendant approximativement 1 heure,
d) est codée par une molécule d'acide nucléique comprenant la séquence nucléotidique codant pour une séquence d'acides aminés de SEQ ID NO : 2 ou 8,
e) est codée par une molécule d'acide nucléique comprenant la séquence nucléotidique codant pour une séquence d'acides aminés présentant une identité de séquence d'au moins 80 % avec la séquence d'acides aminés de SEQ ID NO : 2 ou 8, de préférence sur la longueur totale de la séquence,
f) comprend la séquence d'acides aminés de SEQ ID NO: 2 ou 8, ou
g) comprend une séquence d'acides aminés présentant une identité de séquence d'au moins 80 % avec la séquence d'acides aminés de SEQ ID NO : 2 ou 8, de préférence sur la longueur totale de la séquence,
à condition que toute séquence de a) à g), facultativement après l'expression, code toujours pour au moins un Htn1 selon SEQ ID NO : 2, un Ht2 ou un Ht3 selon SEQ ID NO : 8 fonctionnel, ou un variant allélique de la WAK (wall-associated kinase) qui est situé ou cartographié sur un locus dans le bin 5 ou le bin 6 sur le bras long du chromosome 8 ;
dans lequel au moins une molécule à l'intérieur de la voie de signalisation d'au moins une WAK (wall-associated kinase) à la synthèse d'au moins un benzoxazinoïde ou à l'intérieur de la voie de synthèse d'au moins un benzoxazinoïde est sélectionnée dans le groupe constitué par :
I) les gènes *bx1* (SEQ ID NO : 10), *bx2* (SEQ ID NO : 12), *igl* (SEQ ID NO : 14), *bx6* (SEQ ID NO : 16), *bx11* (SEQ ID NO: 18), *bxl4* (SEQ ID NO : 20), *opr2* (SEQ ID NO : 22), *lox3* (SEQ ID NO : 24) ou *aoc1* (SEQ ID NO : 26), ou
II) un gène présentant une identité d'au moins 80 % avec l'un des gènes de 1), de préférence sur la longueur totale du gène, en particulier les régions codantes du gène, ou
III) les protéines BX1 (SEQ ID NO : 11), BX2 (SEQ ID NO : 13), IGL (SEQ ID NO : 15), BX6 (SEQ ID NO : 17), BX11 (SEQ ID NO : 19), BX14 (SEQ ID NO : 21), OPR2 (SEQ ID NO : 23), LOX3 (SEQ ID NO : 25) ou gène AOC1 (SEQ ID NO : 27), ou
IV) une protéine présentant une identité d'au moins 80 % avec l'une des protéines de III), de préférence sur la longueur totale de la protéine, et
dans lequel le benzoxazinoïde dont la synthèse est régulée par Pau moins une WAK (wall-associated kinase) est DIM₂BOA, le benzoxazinoïde susmentionné étant la forme glucoside ou aglucone, ou une benzoxazolinone, ou toute combinaison avec DIMBOA, HMBOA, HM₂BOA, HDMBOA, HDM₂BOA, HBOA, DHBOA, DIBOA ou TRIBOA;
dans lequel la méthode n'est pas un procédé essentiellement biologique pour la production de la plante Zea mays

2. Procédé selon la revendication 1, dans lequel au moins une molécule à l'intérieur de la voie de signalisation d'au moins une WAK (wall-associated kinase) à la synthèse d'au moins un benzoxazinoïde ou à l'intérieur de la voie de synthèse d'au moins un benzoxazinoïde est sélectionnée dans le groupe constitué par :
I) les gènes *bx1* (SEQ ID NO : 10), *bx2* (SEQ ID NO : 12), *igl* (SEQ ID NO : 14), *bx6* (SEQ ID NO : 16), *bx11* (SEQ ID NO : 18), *bx14* (SEQ ID NO : 20), *opr2* (SEQ ID NO : 22), *lox3* (SEQ ID NO : 24) ou *aocl* (SEQ ID NO : 26), ou
II) un gène présentant une identité d'au moins 90 % avec l'un des gènes de 1), de préférence sur la longueur totale du gène, en particulier les régions codantes du gène, ou
III) les protéines BX1 (SEQ ID NO : 11), BX2 (SEQ ID NO : 13), IGL (SEQ ID NO : 15), BX6 (SEQ ID NO : 17), BX11 (SEQ ID NO : 19), BX14 (SEQ ID NO : 21), OPR2 (SEQ ID NO : 23), LOX3 (SEQ ID NO : 25) ou gène AOC1 (SEQ ID NO : 27), ou
IV) une protéine présentant une identité d'au moins 90 % avec l'une des protéines de III), de préférence sur la longueur totale de la protéine.
